# EUROPEAN PATENT SPECIFICATION

(11) **EP 2 682 746 B1**
(45) Date of publication and mention of the grant of the patent: **24.05.2017**
(21) Application number: 13176355.9
(22) Date of filing: 01.02.2008
(51) Int. Cl.: G01N 33/49, G01N 27/00, H01J 49/26

(54) **Methods for the diagnosis of ovarian cancer health states and risk of ovarian cancer health states**
Verfahren zur Diagnose des Gesundheitszustands von Eierstockkrebs und der Gefahr von Gesundheitszuständen von Eierstockkrebs
Procédés pour le diagnostic d'états de santé associés au cancer de l'ovaire et du risque d'états de santé associés au cancer des ovaires

(30) Priority: 01.02.2007 US 887693 P
(43) Date of publication of application: 08.01.2014
(62) Divisional of application: 08714593.4
(73) Proprietor: Phenomenome Discoveries Inc., Saskatoon, Saskatchewan S7N 4L8 (CA)
(72) Inventor: Ritchie, Shawn, Saskatoon Saskatchewan S7V 1G6 (CA); Bingham, Erin, Saskatoon Saskatchewan S7W 1B5 (CA)
(74) Representative: D Young & Co LLP

(56) References cited:
- WO-A2-2007/030928
- ZHANG H ET AL: "Biomarker discovery for ovarian cancer using SELDI-TOF-MS", GYNECOLOGIC ONCOLOGY, ACADEMIC PRESS, LONDON, GB, vol. 102, no. 1, 1 July 2006 (2006-07-01), pages 61-66, XP024921539, ISSN: 0090-8258, DOI: DOI:10.1016/J.YGYNO.2005.11.029 [retrieved on 2006-07-01]
- BERGEN H R ET AL: "DISCOVERY OF OVARIAN CANCER BIOMARKERS IN SERUM USING NANOLC ELECTROSPRAY IONIZATION TOF AND FT-ICR MASS SPECTROMETRY", DISEASE MARKERS, WILEY, CHICHESTER, GB, vol. 19, no. 4/05, 1 January 2003 (2003-01-01), pages 239-249, XP008039466, ISSN: 0278-0240
- CONRADS T P ET AL: "High-resolution serum proteomic features for ovarian cancer detection", ENDOCRINE - RELATED CANCER, BIOSCIENTIFICA LTD, GB, vol. 11, no. 2, 1 June 2004 (2004-06-01), pages 163-178, XP002452710, ISSN: 1351-0088, DOI: 10.1677/ERC.0.0110163
- KOZAK K R ET AL: "CHARACTERIZATION OF SERUM BIOMAKERS FOR DETECTION OF EARLY STAGE OVARIAN CANCER", PROTEOMICS, WILEY - VCH VERLAG, WEINHEIM, DE, vol. 5, no. 17, 1 November 2005 (2005-11-01), pages 4589-4596, XP008056606, ISSN: 1615-9853, DOI: 10.1002/PMIC.200500093
- DATABASE BIOSIS [Online] BIOSCIENCES INFORMATION SERVICE, PHILADELPHIA, PA, US; April 2005 (2005-04), DOS REIS FRANCISCO J CANDIDO ET AL: "Sequential serum proteomic analysis for the idenification of proteins of interest in ovarian cancer.", XP055088033, Database accession no. PREV200700268494 & PROCEEDINGS OF THE AMERICAN ASSOCIATION FOR CANCER RESEARCH ANNUAL MEETING, vol. 46, April 2005 (2005-04), page 91, 96TH ANNUAL MEETING OF THE AMERICAN-ASSOCIATION-FOR-CANCER-RESEARCH; ANAHEIM, CA, USA; APRIL 16 -20, 2005 ISSN: 0197-016X
- C. Denkert ET AL: "Mass Spectrometry-Based Metabolic Profiling Reveals Different Metabolite Patterns in Invasive Ovarian Carcinomas and Ovarian Borderline Tumors", Cancer research, vol. 66, no. 22, 15 November 2006 (2006-11-15), pages 10795-10804, XP055176842, ISSN: 0008-5472, DOI: 10.1158/0008-5472.CAN-06-0755

## Description

### FIELD OF INVENTION

The present invention relates to methods for diagnosing ovarian cancer, or the risk of developing ovarian cancer.

### BACKGROUND OF THE INVENTION

Ovarian cancer is the fifth leading cause of cancer death among women (1). It has been estimated that over 22,000 new cases of ovarian cancer will be diagnosed this year, with 16,210 deaths predicted in the United States alone (2). Ovarian cancer is typically not identified until the patient has reached stage III or IV, which is associated with a poor prognosis; the five-year survival rate is estimated at around 25- 30% (3). The current screening procedures for ovarian cancer involve the combination of bimanual pelvic examination, transvaginal ultrasonography, and serum screening for elevated cancer antigen-125 (CA125), a protein cancer antigen (2). The efficacy of CA125 screening for ovarian cancer is currently of unknown benefit, as there is a lack of evidence that the screen reduces mortality rates, and it is under scrutiny due to the risks associated with false positive results (1, 4). According to the American Cancer Society, CA125 measurement and transvaginal ultrasonography are not reliable screening or diagnostic tests for ovarian cancer, and that the only current method available to make a definite diagnosis is by surgery (http://www.cancer.org).

CA125 is a high molecular weight mucin that has been found to be elevated in most ovarian cancer cells as compared to normal cells (2). A CA125 test result that is higher than 30-35U/ml is typically accepted as being at an elevated level (2). There have been difficulties in establishing the accuracy, sensitivity, and specificity of the CA125 screen for ovarian cancer due to the different thresholds used to define elevated CA125, varying sizes of patient groups tested, and broad ranges in the age and ethnicity of patients (1). According to the Johns Hopkins University pathology website, the CA125 test only returns a true positive result for ovarian cancer in roughly 50% of stage I patients and about 80% in stage II, III and IV patients (http://pathology2.jhu.edu). Endometriosis, benign ovarian cysts, pelvic inflammatory disease, and even the first trimester of a pregnancy have all been reported to increase the serum levels of CA125 (4). The National Institute of Health's website states that CA125 is not an effective general screening test for ovarian cancer. They report that only about three out of 100 healthy women with elevated CA125 levels are actually found to have ovarian cancer, and about 20% of ovarian cancer diagnosed patients actually have elevated CA125 levels (http://www.nlm.nih.gov/medlineplus/ency/article/007217.htm).

Zhang et al., Gynecologic Oncology, 2006, 102(1): 61-66 describes a proteomics-based method for identifying protein markers of ovarian cancer.

Bergen et al., Disease Markers, 2003, 19:239-249 describes another proteomics-based method for identifying protein markers of ovarian cancer. This reference describes the challenge of elucidating candidate biomarkers from abundant serum proteins, and incorporates the use of cut-off filters to facilitate "the detection and analysis of low abundance proteins/peptides" (see page 247, second column, lines 2-3).

Conrads et al., Endocrine-Related Cancer, Bioscientifica Ltd, GB, 2004, 11:163-178*)* describes a proteomics study comparing low resolution and high resolution mass spectrometry techniques.

Kozak et al., Proteomics 2005, 5:44589-4596 describes a study using micro-LC-MS/MS to identify four protein markers of ovarian cancer, i.e., transthyretin, beta-hemoglobin, apolipoprotein AI, and transferrin, from a group of previously reported SELDI-TOF-MS peaks.

Dos Reis et al., Proc. Amer. Assoc. Cancer Res., vol 46, April 2005 (2005-04), p. 91 describes a study using proteomic analysis to identify serum proteins that diminish in concentration during treatment for ovarian cancer. LC-MS/MS analysis identified four peptide fragments corresponding to apolipoprotein D, Sp-alpha, alpha-enolase, transketolase, and M2-PK.

WO2007030928 discloses the use of a metabolite with the formula C28H46O4 as a marker for ovarian cancer.

Denkert et al, Cancer Research 2006; 66: (22), November 15, 2006 relates to the use of mass spectrometry-based metabolic profiling in ovarian carcinomas.

It is clear that there is a need for improving ovarian cancer detection. A test that is able to detect risk for, or the presence of, ovarian cancer or that can predict aggressive disease with high specificity and sensitivity would be very beneficial and would impact ovarian cancer morbidity.

### SUMMARY OF THE INVENTION

The present invention provides a method for diagnosing ovarian cancer or the risk of developing ovarian cancer in a test subject of unknown ovarian cancer status, comprising the steps of:
analyzing a blood sample from a test subject to obtain quantifying data on molecules selected from metabolites identified by the exact masses 474.3736, 478.4022, 484.3764, 490.3658, 492.3815, 494.3971, 496.4128, 502.4022, 504.4179, 512.4077, 518.3971, 520.4128, 522.8284, 530.43351, 532.44916, 540.4389, 550.4597, 558.4648, 574.4597, 576.4754, 578.4910, 596.5016, and 598.5172 where a ± 5 ppm difference would indicate the same metabolite;
wherein the molecular formulas of the metabolites with these masses are C₃₀H₅₀O₄, C₃₀H₅₄O₄, C₂₈H₅₂O₆, C₃₀H₅₀O₅, C₃₀H₅₂O₅, C₃₀H₅₄O₅, C₃₀H₅₆O₅, C₃₂H₅₄O₄, C₃₂H₅₆O₄, C₃₀H₅₆O₆, C₃₂H₅₄O₅, C₃₂H₅₆O₅, C₃₂H₆₀O₅, C₃₄H₅₈O₄, C₃₄H₆₀O₄, C₃₂H₆₀O₆, C₃₄H₆₂O₅, C₃₆H₆₂O₄, C₃₆H₆₂O₅, C₃₆H₆₄O₅, C₃₆H₆₆O₅, C₃₆H₆₈O₆, and C₃₆H₇₀O₆, respectively;
wherein the metabolites have LC-MS/MS fragment patterns analyzed under negative ionization as follows:
   the metabolite having the molecular formula C₃₀H₅₀O₄ has a LC-MS/MS fragment pattern comprising daughter ions with m/z of: 473, 455, 429, 411, 223, 113;
   the metabolite having the molecular formula C₃₀H₅₄O₄ has a LC-MS/MS fragment pattern comprising daughter ions with m/z of: 477, 460, 433, 415, 281;
   the metabolite having the molecular formula C₂₈H₅₂O₆ has a LC-MS/MS fragment pattern comprising daughter ions with m/z of: 483, 465, 447, 439, 421, 315, 313, 297, 279, 241, 201, 171, 101;
   the metabolite having the molecular formula C₃₀H₅₀O₅ has a LC-MS/MS fragment pattern comprising daughter ions with m/z of: 489, 471, 445, 427, 373, 345, 319, 267, 249, 223;
   the metabolite having the molecular formula C₃₀H₅₂O₅ has a LC-MS/MS fragment pattern comprising daughter ions with m/z of: 491, 473, 445, 427, 319, 249, 241, 223, 213;
   the metabolite having the molecular formula C₃₀H₅₄O₅ has a LC-MS/MS fragment pattern comprising daughter ions with m/z of: 493, 475, 449, 431, 415, 307, 297, 279, 267, 241, 235, 223, 215, 197, 167, 151, 141, 113;
   the metabolite having the molecular formula C₃₀H₅₆O₅ has a LC-MS/MS fragment pattern comprising daughter ions with m/z of: 495, 477, 451, 433, 297, 279, 241, 223, 215, 197, 179, 169;
   the metabolite having the molecular formula C₃₂H₅₄O₄ has a LC-MS/MS fragment pattern comprising daughter ions with m/z of: 501, 483, 465, 457, 439, 279;
   the metabolite having the molecular formula C₃₂H₅₆O₄ has a LC-MS/MS fragment pattern comprising daughter ions with m/z of: 503, 485, 467, 459, 441, 279, 263, 223, 169;
   the metabolite having the molecular formula C₃₀H₅₆O₆ has a LC-MS/MS fragment pattern comprising daughter ions with m/z of: 511, 493, 467, 315, 297, 279, 259, 251, 151;
   the metabolite having the molecular formula C₃₂H₅₄O₅ has a LC-MS/MS fragment pattern comprising daughter ions with m/z of: 517, 499, 481, 473, 455, 445, 437, 389, 279,223;
   the metabolite having the molecular formula C₃₂H₅₆O₅ has a LC-MS/MS fragment pattern comprising daughter ions with m/z of: 519, 501, 483, 475, 459, 457, 447, 297, 279, 241, 223, 195, 115;
   the metabolite having the molecular formula C₃₂H₆₀O₅ has a LC-MS/MS fragment pattern comprising daughter ions with m/z of: 521, 503, 485, 477, 459, 441, 297, 279, 269, 241, 115;
   the metabolite having the molecular formula C₃₄H₅₈O₄ has a LC-MS/MS fragment pattern comprising daughter ions with m/z of: 529, 511, 485, 467, 251, 205;
   the metabolite having the molecular formula C₃₄H₆₀O₄ has a LC-MS/MS fragment pattern comprising daughter ions with m/z of: 531, 513, 495, 485, 469, 251, 181;
   the metabolite having the molecular formula C₃₂H₆₀O₆ has a LC-MS/MS fragment pattern comprising daughter ions with m/z of: 539, 521, 495, 477, 315, 313, 297, 279, 259,243,241,225,223,179,171;
   the metabolite having the molecular formula C₃₄H₆₂O₅ has a LC-MS/MS fragment pattern comprising daughter ions with m/z of: 549, 531, 513, 505, 487, 469, 297, 279, 269, 253, 125;
   the metabolite having the molecular formula C₃₆H₆₂O₄ has a LC-MS/MS fragment pattern comprising daughter ions with m/z of: 557, 539, 513, 495, 279, 155;
   the metabolite having the molecular formula C₃₆H₆₂O₅ has a LC-MS/MS fragment pattern comprising daughter ions with m/z of: 573, 555, 537, 529, 511, 493, 401, 295, 279,223;
   the metabolite having the molecular formula C₃₆H₆₄O₅ has a LC-MS/MS fragment pattern comprising daughter ions with m/z of: 575, 557, 539, 531, 513, 495, 403, 297, 279, 251, 183;
   the metabolite having the molecular formula C₃₆H₆₆O₅ has a LC-MS/MS fragment pattern comprising daughter ions with m/z of: 577, 559, 541, 533, 515, 497, 373, 297, 281,279;
   the metabolite having the molecular formula C₃₆H₆₈O₆ has a LC-MS/MS fragment pattern comprising daughter ions with m/z of: 595, 577, 559, 551, 515, 315, 297, 281, 279, 171, 155, 141, 127;
   the metabolite having the molecular formula C₃₆H₇₀O₆ has a LC-MS/MS fragment pattern comprising daughter ions with m/z of: 597, 579, 561, 517, 315, 297, 279; respectively
   wherein the molecules are analyzed by a liquid chromatography - mass spectrometry (LC-MS) method or direct injection triple-quadrupole mass spectrometry method;
   comparing the quantifying data obtained on molecules in said test subject with quantifying data obtained from said molecules from a plurality of ovarian cancer-negative humans; wherein the absence or significant reduction of one or more than one of said metabolite indicates the presence of ovarian cancer or the risk of developing ovarian cancer.

The methods measure the intensities of specific small molecules, also referred to as metabolites, in the sample from the patient, and compare these intensities to the intensities observed in a population of healthy individuals. The sample obtained from the human is a blood sample.

The present invention may significantly improve the ability to detect ovarian cancer or the risk of developing ovarian cancer, and may therefore save lives. The statistical performance of a test based on these samples suggests that the test will outperform the CA125 test, the only other serum-based diagnostic test for ovarian cancer. Alternatively, a combination of the test described herein and the CA125 test may improve the overall diagnostic performance of each test.

In a further embodiment of the present invention there is provided a method for diagnosing a patient for the presence of an ovarian cancer, or the risk of developing ovarian cancer, comprising the steps of: screening a sample from said patient for the presence or absence of one or more metabolic markers selected from the group consisting of metabolites with an accurate mass of, or substantially equivalent to masses to 474.3736, 478.4022, 484.3764, 490.3658, 492.3815, 494.3971, 496.4128, 502.4022, 504.4179, 512.4077, 518.3971, 520.4128, 522.8284, 530.43351, 532.44916, 540.4389, 550.4597, 558.4648, 574.4597, 576.4754, 578.4910, 596.5016, and 598.5172, where a +/- 5 ppm difference would indicate the same metabolite; wherein the absence or significant reduction of one or more of said metabolic markers indicates the presence of an ovarian cancer, or the risk of developing ovarian cancer. In this embodiment of the invention the molecular formulas of the metabolites with these masses are C30H50O4, C30H5404, C28H52O6, C30H5005, C30H52O5, C30H5405, C30H5605, C32H5404, C32H56O4, C30H56O6, C32H5405, C32H5605, C32H6005, C34H5804, C34H60O4, C32H6006, C34H6205, C36H62O4, C36H6205, C36H6405, C36H66O5, C36H68O6, and C36H7006, respectively and the proposed structures are as shown below: 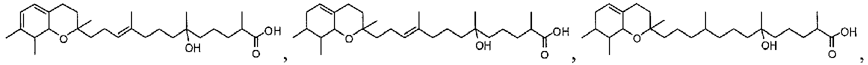 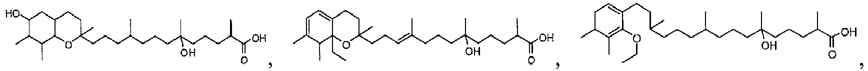 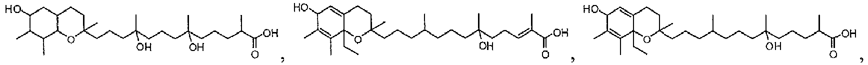 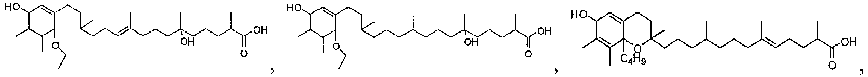 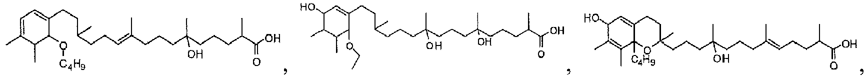 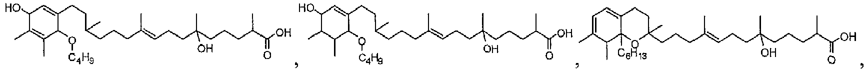 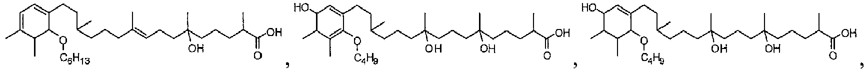 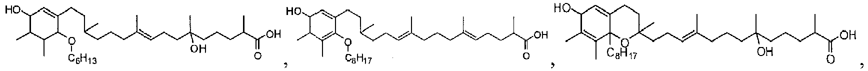 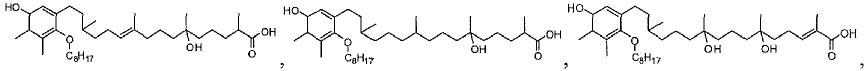 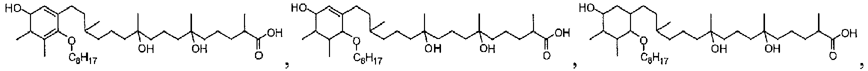 respectively.

In a further embodiment of the present invention there is provided a method for diagnosing the presence or absence of ovarian cancer in a test subject of unknown ovarian cancer status, comprising: analyzing a blood sample from a test subject to obtain quantifying data on molecules selected from the group comprised of molecules identified by the neutral accurate masses 474.3736, 478.4022, 484.3764, 490.3658, 492.3815, 494.3971, 496.4128, 502.4022, 504.4179, 512.4077, 518.3971, 520.4128, 522.8284, 530.43351, 532.44916, 540.4389, 550.4597, 558.4648, 574.4597, 576.4754, 578.4910, 596.5016, and 598.5172, where a +/- 5 ppm difference would indicate the same metabolite, or molecules having masses substantially equal to these molecules or fragments of derivatives thereof; comparing the quantifying data obtained on said molecules in said test subject with quantifying data obtained from said molecules from a plurality of ovarian cancer-positive humans or quantifying data obtained from a plurality of ovarian cancer-negative humans; and wherein said comparison can be used to determine the probability that the test subject is ovarian cancer-positive or -negative. In this embodiment of the invention the molecular formulas of the metabolites with these masses are C30H5004, C30H54O4, C28H52O6, C30H50O5, C30H52O5, C30H54O5, C30H56O5, C32H54O4, C32H56O4, C30H56O6, C32H54O5, C32H56O5, C32H60O5, C34H58O4, C34H60O4, C32H60O6, C34H62O5, C36H62O4, C36H62O5, C36H64O5, C36H66O5, C36H68O6, and C36H70O6, respectively and the proposed structures are as shown below: 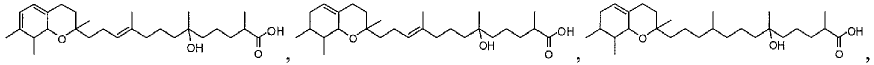 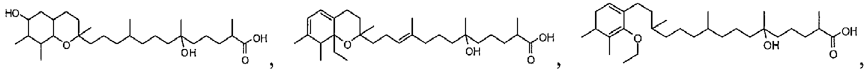 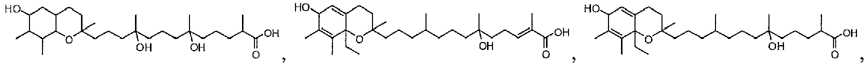 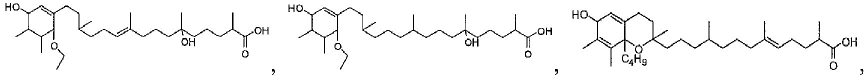 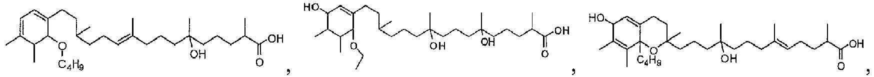 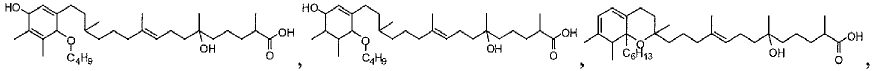 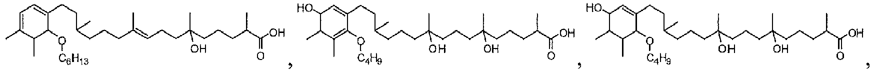 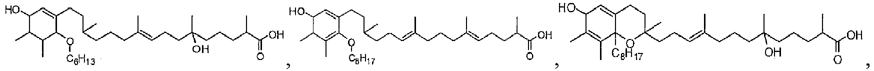 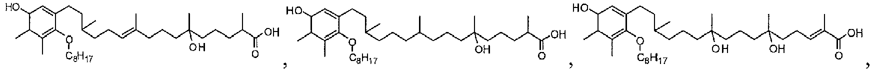 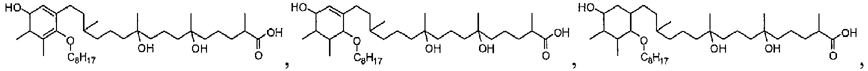 respectively.

The identification of ovarian cancer biomarkers with improved diagnostic accuracy in human serum, therefore, would be extremely beneficial, as the test would be non-invasive and could possibly be used to monitor individual susceptibility to disease prior to, or in combination with, conventional methods. A serum test is minimally invasive and would be accepted across the general population. The present invention relates to a method of diagnosing ovarian cancer, or the risk of developing ovarian cancer, by measuring the levels of specific small molecules present in human serum and comparing them to "normal" reference level s. In yet a further embodiment of the present invention, any one or combination of the metabolites identified in the present invention can be used to indicate the presence of ovarian cancer. A diagnostic assay based on small molecules, or metabolites, in serum fulfills the above criteria for an ideal screening test, as development of assays capable of detecting specific metabolites is relatively simple and cost effective per assay. Translation of the method into a clinical assay compatible with current clinical chemistry laboratory hardware would be commercially acceptable and effective, and would result in a rapid deployment worldwide. Furthermore, the requirement for highly trained personnel to perform and interpret the test would be eliminated.

The selected metabolites, identified according to the present invention, were further characterized by molecular formulae and structure. This additional information for the metabolites is shown in Table 35.

### BRIEF DESCRIPTION OF THE DRAWINGS

These and other features of the invention will become more apparent from the following description in which reference is made to the appended drawings wherein:

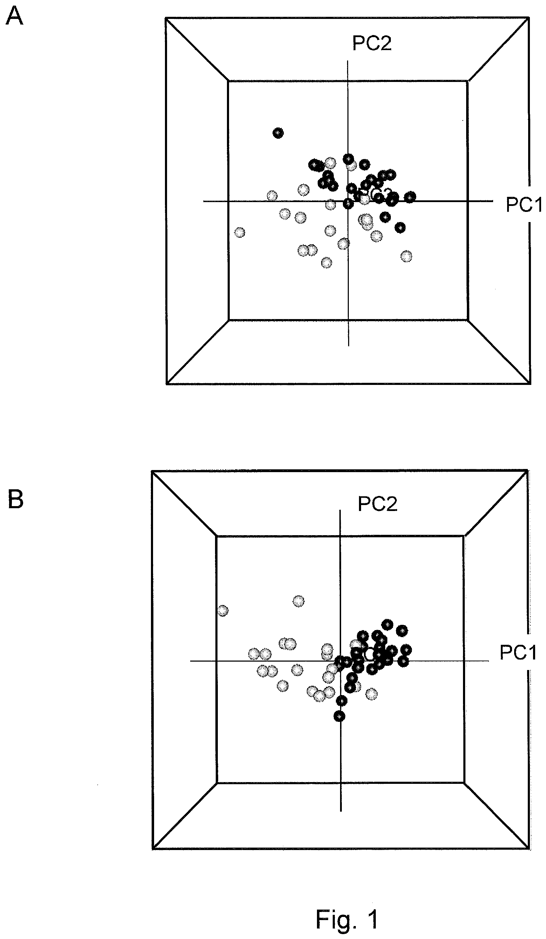

FIGURE 1 shows a principal component analysis (PCA) plot of ovarian cancer and normal metabolite profiles of serum samples. FIGURE 1A uses the complete metabolomic dataset (1,422 masses), while FIGURE 1B uses 424 metabolites, with p<0.05. Each point represents an individual patient sample. Grey points represent ovarian cancer patient samples, and black points represent normal controls. With PCA, samples that cluster near to each other must have similar properties based on the data. Therefore, it is evident from this plot that the ovarian cancer patient population shares common metabolic features, and which are distinct from the control population.

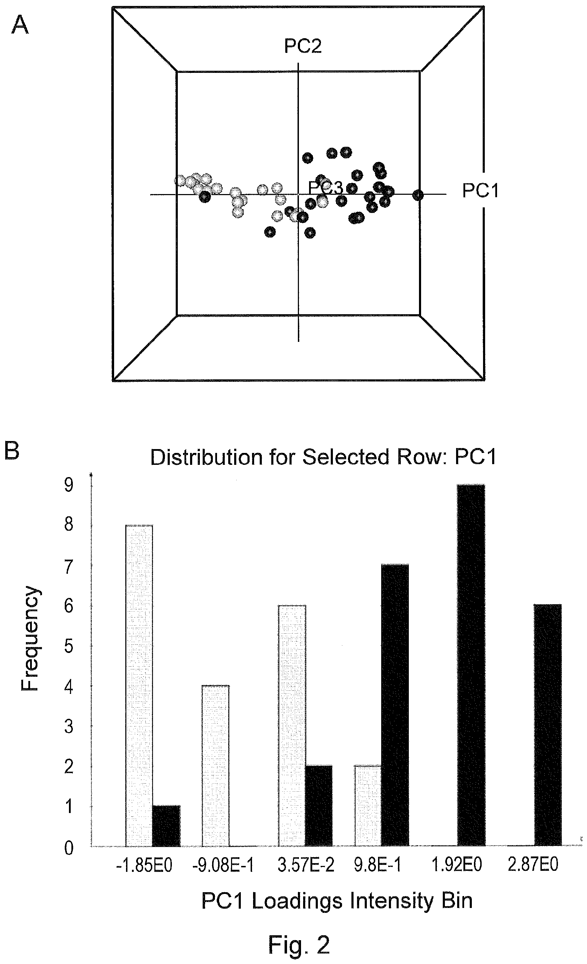

FIGURE 2A shows a PCA plot resulting from 37 metabolites that were selected from the table of 424 based upon the following criteria: p<0.0001, ¹³C peaks excluded, and only metabolites detected in analysis mode 1204 (organic, negative APCI). Grey points, ovarian cancer samples; black points, normal controls.
FIGURE 2B shows the distribution of patient samples binned according to the PC1 loadings score (the position of the point along the x-axis) from FIGURE 2A. This shows that, using the origin of the PCA plot as a cutoff point, two of the 20 ovarian cancer patients (grey) group with the control bins (90% sensitivity), while three of the 25 normal subjects (black) group with the ovarian cancer patients (88% specificity).

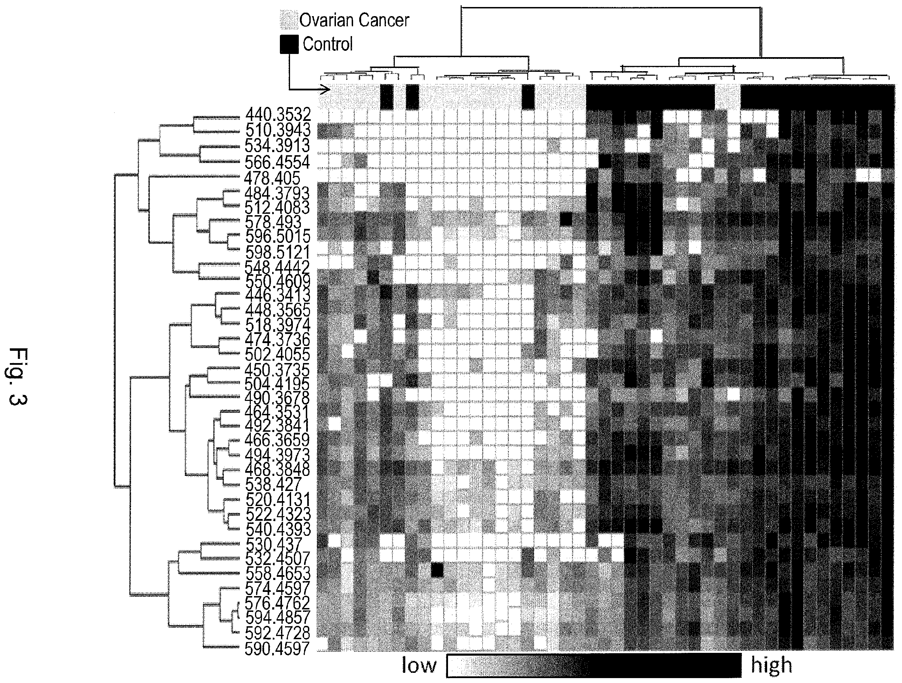

FIGURE 3 shows a hierarchically clustered metabolite array of the 37 selected metabolites. The samples have been clustered using a Euclidean squared distance metric, while the 37 metabolites have been clustered using a Pearson correlation metric. White cells indicate metabolites with absent intensities, while increasingly darker cells correspond to larger metabolite intensities, respectively. These results mirror the PCA results shown in FIGURE 2 (A and B), which indicate that two ovarian cancer samples cluster with the control group, and three controls cluster with the ovarian cancer group. The plot, however, indicates that the entire cluster of molecules is *deficient* from the serum of the ovarian cancer patients relative to the controls. The detected masses are shown along the left side of the figure, while de-identified patient ID numbers are shown along the top of the figure (grey headers, ovarian cancer; black headers, controls). Cells with darker shades of grey to black represent metabolite signals with higher intensities than white or lightly shaded cells.

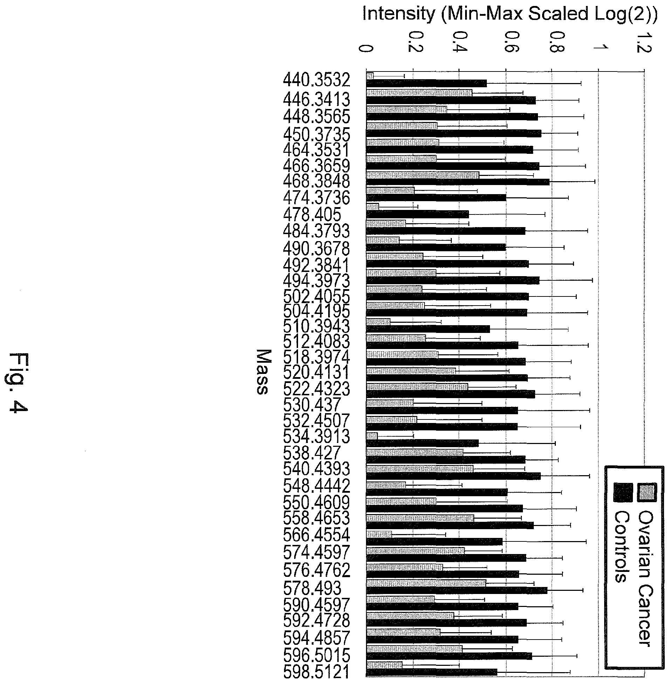

FIGURE 4 shows a bar graph of the relative intensities of the 37 selected metabolites. The intensity values (± 1 s.d.) were derived by rescaling the log(2) transformed intensities of individual metabolites between zero and one. The graph shows that all 37 molecules in the ovarian cancer cohort (grey) are significantly lower in intensity relative to the control cohort (black).

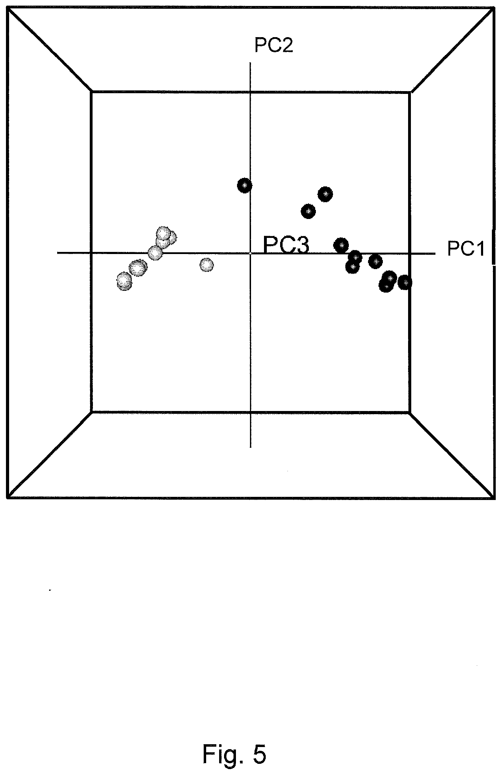

FIGURE 5 shows a PCA plot of 20 samples (10 ovarian cancer, 10 controls) that was generated using intensities of 29 of the 37 metabolites rediscovered using full-scan HPLC-coupled time-of-flight (TOF) mass spectrometry of the same extract analyzed previously with the FTMS. The ovarian cancer samples (grey) are shown to cluster perfectly apart from the controls (black), verifying that the markers are indeed present in the extracts and are specific for the presence of ovarian cancer.

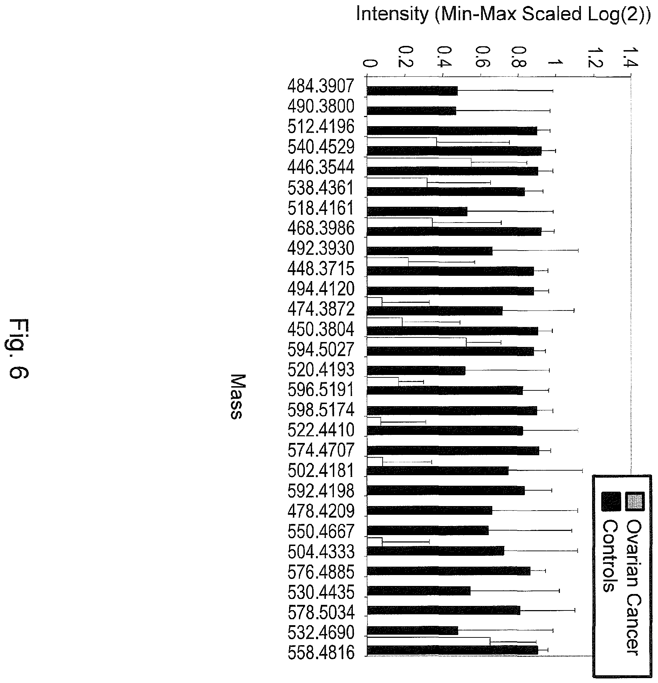

FIGURE 6 shows a graph of 29 of the 37-metabolite panel, identified in a non-targeted analysis on the TOF mass spectrometer (± 1 s.d.). The results verify those observed with the FTMS data, that is, these molecules are significantly lower in intensity in ovarian cancer patients (grey) compared to controls (black).

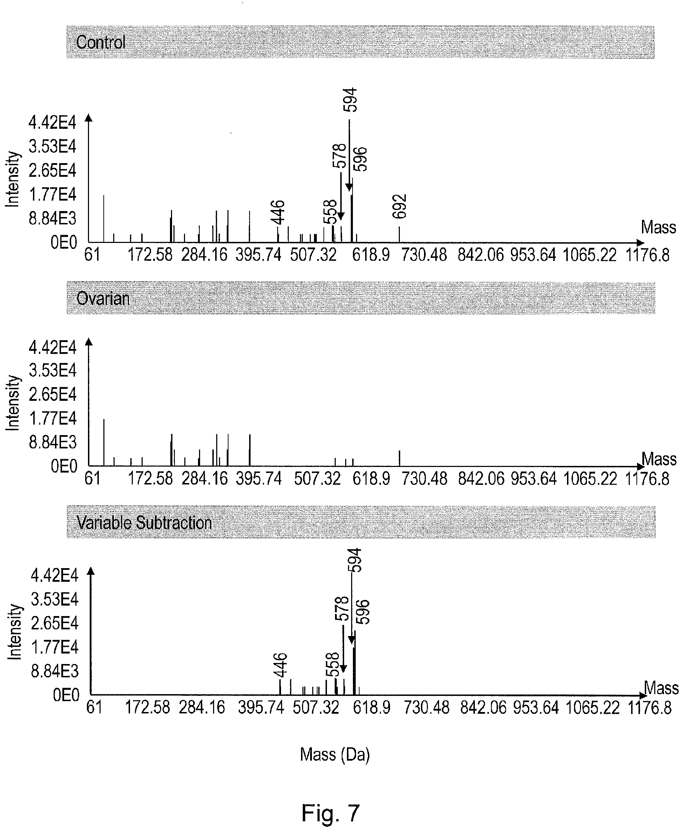

FIGURE 7 shows the extracted mass spectra for the retention time window between 15 and 20 minutes from the HPLC-TOF analysis. This shows the masses detected within this elution time of the HPLC column. The peaks represent an average of the 10 controls (top panel) and 10 ovarian cancers (middle panel). The bottom panel shows the net difference between the top and middle spectra. This clearly shows that peaks in the mass range of approximately 450 to 620 are deficient from the ovarian cancer samples (middle panel) relative to the controls (top panel).

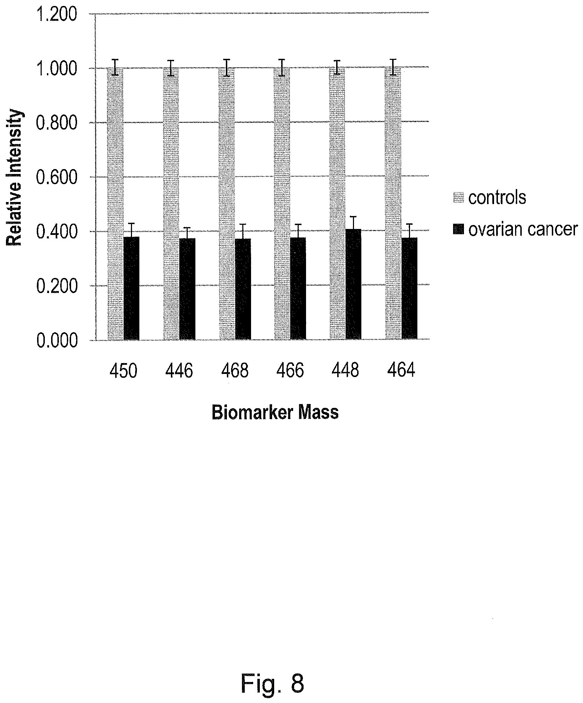

FIGURE 8 shows the relative intensities of six of the C28 ovarian markers using the targeted HTS triple-quadrupole method (relative intensity +/- SEM). Controls = 289 subjects, ovarian = 20 subjects.

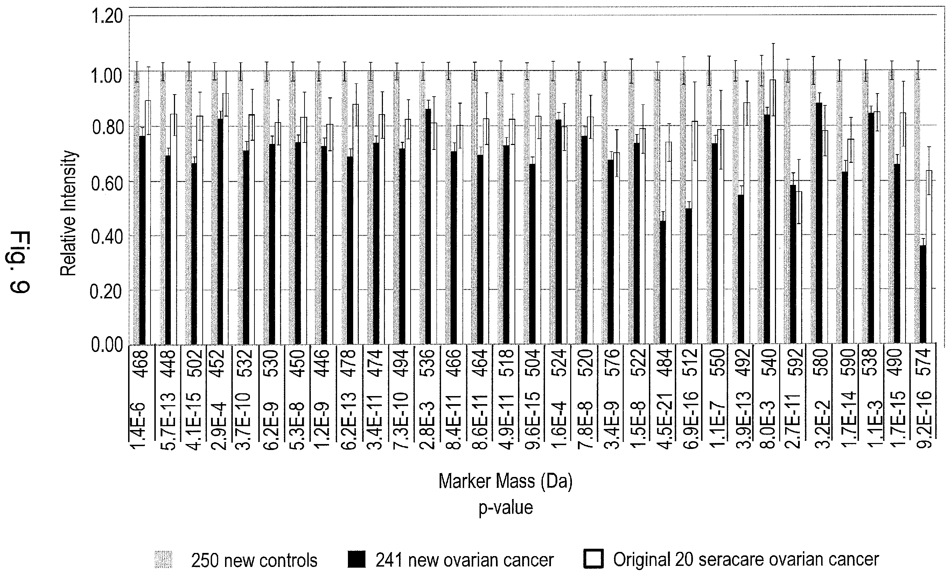

FIGURE 9 shows the relative intensities of 31 ovarian markers using the targeted HTS triple-quadrupole method. Controls = 289 subjects, ovarian = 241 new cases (black bars) and the 20 original Seracare cases (white bars). The panel was derived from a combination of molecules in Table 1, 2 and 3.

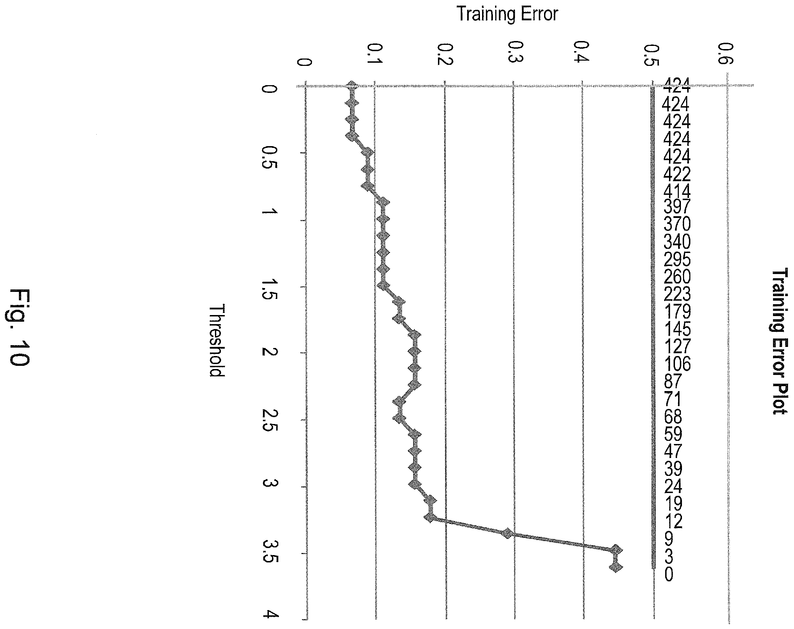

FIGURE 10 shows a training error plot for a shrunken centroid supervised classification algorithm using all masses listed in Table 1. The plot shows that the lowest training error (representing the highest diagnostic accuracy) is achieved with the maximum number of metabolites (listed across the top of the plot), that is, all masses in Table 1 (424 total).

### DETAILED DESCRIPTION OF A PREFERRED EMBODIMENT

The present invention relates to the diagnosis of ovarian cancer (OC), or the risk of developing OC,. The present invention describes the relationship between endogenous small molecules and OC. Specifically, the present invention relates to the diagnosis of OC, or the risk of developing OC, through the measurement of vitamin E isoforms and related metabolites. More specifically, the present invention relates to the relationship between vitamin E-related metabolites in human serum and the implications thereof in OC.

The present invention discloses for the first time clear and unambiguous biochemical changes specifically associated with OC. These findings also imply that the measurement of these biomarkers may provide a universal means of measuring the effectiveness of OC therapies. This would dramatically decrease the cost of performing clinical trials as a simple biochemical test can be used to assess the viability of new therapeutics. Furthermore, one would not have to wait until the tumor progresses or until the patient dies to determine whether the therapy provided any benefit. The use of such a test would enable researchers to determine in months, rather than years, the effectiveness of dose, formulation, and chemical structure modifications of OC therapies.

The present invention relates to a method of diagnosing OC by measuring the levels of specific small molecules present in human serum and comparing them to "normal" reference levels. In one embodiment of the present application there is described a novel method for the early detection and diagnosis of OC and the monitoring the effects of OC therapy.

One method of the present invention uses accurate masses in an FTMS based method. The accurate masses that can be used according to this invention include the claimed masses shown in Table 1, or a subset thereof.

A further method involves the use of a high-throughput screening (HTS) assay developed from a subset of claimed metabolites selected from Table 1 for the diagnosis of one or more diseases or particular health-states. The utility of the claimed method is demonstrated and validated through the development of a HTS assay capable of diagnosing an OC-positive health-state.

The impact of such an assay on OC would be tremendous, as literally everyone could be screened longitudinally throughout their lifetime to assess risk and detect ovarian cancer early. Given that the performance characteristics of the test are representative for the general OC population, this test alone may be superior to any other currently available OC screening method, as it may have the potential to detect disease progression prior to that detectable by conventional methods. The early detection of OC is critical to positive treatment outcome.

The term "vitamin E" collectively refers to eight naturally occurring isoforms, four tocopherols (alpha, beta, gamma, and delta) and four tocotrienols (alpha, beta, gamma, and delta). The predominant form found in western diets is gamma-tocopherol whereas the predominant form found in human serum/plasma is alpha-tocopherol. Tocotrienols are also present in the diet, but are more concentrated in cereal grains and certain vegetable oils such as palm and rice bran oil. Interestingly, it is suggested that tocotrienols may be more potent than tocopherols in preventing cardiovascular disease and cancer (5). This may be attributable to the increased distribution of tocotrienols within lipid membranes, a greater ability to interact with radicals, and the ability to be quickly recycled more quickly than tocopherol counterparts (6). It has been demonstrated that in rat liver microsomes, the efficacy of alpha-tocotrienol to protect against iron-mediated lipid peroxidation was 40 times higher that that of alpha-tocopherol (6). However, measurements in human plasma indicate that trienols are either not detected or present only in minute concentrations (7), due possibly to the higher lipophilicity resulting in preferential bilary excretion (8).

A considerable amount of research related to the discrepancy between the distribution of alpha and gamma tocopherol has been performed on these isoforms. It has been known and reported as early as 1974 that gamma- and alpha- tocopherol have similar intestinal absorption but significantly different plasma concentrations (9). In the Bieri and Evarts study (9), rats were depleted of vitamin E for 10 days and then fed a diet containing an alpha:gamma ratio of 0.5 for 14 days. At day 14, the plasma alpha:gamma ratio was observed to be 5.5. The authors attributed this to a significantly higher turnover of gamma-tocopherol, however, the cause of this increased turnover was unknown. Plasma concentrations of the tocopherols are believed to be tightly regulated by the hepatic tocopherol binding protein. This protein has been shown to preferentially bind to alpha-tocopherol (10). Large increases in alpha-tocopherol consumption result in only small increases in plasma concentrations (11). Similar observations hold true for tocotrienols, where high dose supplementation has been shown to result in maximal plasma concentrations of approximately only 1 to 3 micromolar (12). More recently, Birringer et al (8) showed that although upwards of 50% of ingested gamma-tocopherol is metabolized by human hepatoma HepG2 cells by omega-oxidation to various alcohols and carboxylic acids, less than 3% of alpha-tocopherol is metabolized by this pathway. This system appears to be responsible for the increased turnover of gamma-tocopherol. In this paper, they showed that the creation of the omega COOH from gamma-tocopherol occured at a rate of >50X than the creation of the analogous omega COOH from alpha-tocopherol. Birringer also showed that the trienols are metabolized via a similar, but more complex omega carboxylation pathway requiring auxiliary enzymes (8).

It is likely that the existence of these two structurally selective processes has biological significance. Birringer et al (8) propose that the purpose of the gamma-tocopherol-specific P450 omega hydroxylase is the preferential elimination of gamma-tocopherol/trienol as 2,7,8-trimethyl-2-(beta-carboxy-3'-carboxyethyl)-6-hydroxychroman (gamma-CEHC). We argue, however, that if the biological purpose is simply to eliminate gamma-tocopherol/trienol, it would be far simpler and more energy efficient via selective hydroxylation and glucuronidation. The net biological effect of these two processes, which has not been commented on in the vitamin E literature, is that the two primary dietary vitamin E isoforms (alpha and gamma), upon entering the liver during first-pass metabolism, are shunted into two separate metabolic systems. System 1 quickly moves the most biologically active antioxidant isoform (alpha-tocopherol) into the blood stream to supply the tissues of the body with adequate levels of this essential vitamin. System 2 quickly converts gamma-tocopherol into the omega COOH. In the present invention it is disclosed that significant concentrations of multiple isoforms of gamma-tocopherol/tocotrienol omega COOH are present in normal human serum at all times. We were able to estimate that the concentration of each of these molecules in human serum is in the low micromolar range by measuring cholic acid, an organically soluble carboxylic acid-containing internal standard used in the triple-quadrupole method. This is within the previously reported plasma concentration range of 0.5 to 2 micromolar for γ-tocopherol (approximately 20 times lower than that of alpha-tocopherol) (13) The cumulative total, therefore, of all said novel γ-tocoenoic acids in serum is not trivial, and likely exceeds that of γ-tocopherol itself. None of the other shorter chain length gamma-tocopherol/trienol metabolites described by Birringer et al (8) were detected in the serum. Also, the alpha and gamma tocotrienols were also not detected in the serum of patients used in the studies reported in this work, suggesting that the primary purpose of the gamma-tocopherol/trineol-specific P450 omega hydroxylase is the formation of the omega COOH and not gamma-CEHC. Not to be bound by the correctness of the theory, it is therefore suggested that the various gamma-tocopherol/tocotrienol omega COOH metabolites disclosed in the present application are novel bioactive agents and that they perform specific and necessary biological functions for the maintenance of normal health and for the prevention of disease.

Of relevance is also the fact that it has been shown that mammals are able to convert trienols to tocopherols *in vivo* (14, 15). Since several of the novel vitamin E-like metabolites disclosed herein contain a semi-saturated phytyl side chain, the possibility of a tocotrienol precursor cannot be excluded.

Just as trienols have been reported to have biological activities separate from the tocopherols (16), gamma-tocopherol has been reported to have biological functions separate and distinct from alpha-tocopherol. For example, key differences between alpha tocopherol and alpha tocotrienol include the ability of alpha tocotrienol to specifically prevent neurodegeneration by regulating specific mediators of cell death (17), the ability of trienols to lower cholesterol (18), the ability to reduce oxidative protein damage and extend life span of *C. elegans* (19), and the ability to suppress the growth of breast cancer cells (20, 21). Key differences between the gamma and alpha forms of tocopherol include the ability of gamma to decrease proinflammatory eicosanoids in inflammation damage in rats (22) and inhibition of cyclooxygenase (COX-2) activity (23). In Jiang et al (23) it was reported that it took 8-24 hours for gamma-tocopherol to be effective and that arachadonic acid competitively inhibits the suppression activity of gamma-tocopherol. It is hypothesized that the omega COOH metabolites of gamma-tocopherol may be the primary bioactive species responsible for its anti-inflammation activity. The conversion of arachadonic acid into eicosanoids is a critical step in inflammation. It is more conceivable that omega COOH forms of gamma-tocopherol, due to their structural similarities to arachadonic acid, are more potent competitive inhibitors of this formation than native gamma-tocopherol.

Not wishing to be bound by any particular theory, in the present invention it is hypothesized that the novel metabolites disclosed herein are indicators of vitamin E activity and that the decrease of such metabolites is indicative of one of the following situations:
a. A hyper-oxidative or metabolic state that is consuming vitamin E and related metabolites at a rate in excess of that being supplied by the diet;
b. A dietary deficiency or impaired absorption of vitamin E and related metabolites;
c. A dietary deficiency or impaired absorption/epithelial transport of vitamin E-related metabolites.
d. An enzymatic deficiency in cytochrome p450 enzymes, including but not limited to CYP4F2, responsible for omega carboxylation of gamma-tocopherol. Such deficiency may comprise a genetic alteration such as single nucleotide polymorphism (SNP), translocation or epigenetic modification such as methylation. Alternatively the deficiency may result from protein post-translational modification, or lack of activation through required ancillary factors, or through transcriptional silencing mediated by promoter mutations or improper transcriptional complex assembly formation.

In all of the aforementioned related epidemiological studies concerning vitamin E, there is little known about the correlation between gamma tocopherol and OC. At the time of this application, a PubMed search for "Ovarian Cancer" and "Gamma Tocopherol" returned only one publication reporting no change in plasma gamma tocopherol levels between OC patients and controls (24). More recent findings have eluded to a potential inverse association between alpha-tocopherol supplementation and ovarian cancer risk (25). Basic research has shown that alpha tocopherol can inhibit telomerase activity in ovarian cancer cells *in vitro,* suggesting a potential role in the control of ovarian cancer cell growth. No *in vitro* effects of gamma tocopherol on ovarian cancer cells has been reported.

Based on the discoveries disclosed in this application, it is contemplated that although dietary deficiencies or deficiencies in specific vitamin E metabolizing enzymes may increase the risk of OC incidence, it is also contemplated that the presence of OC may result in the decrease of vitamin E isoforms and related metabolites. These decreased levels are not likely to be the result of a simple dietary deficiency, as such a strong association would have been previously revealed in epidemiological studies, such as in the study performed by Helzlsouer et al (24).

Based on the discoveries disclosed in this application, it is also contemplated that the decreased levels of vitamin E-like metabolites are not the result of a simple dietary deficiency, but rather impairment in the colonic epithelial uptake of vitamin E and related molecules. This therefore represents a rate-limiting step for the sufficient provision of anti-oxidant capacity to epithelial cells under an oxidative stress load. In this model, the dietary effects of increased iron consumption through red meats, high saturated fat, and decreased fiber (resulting in a decreased iron chelation effect (26)) results in the previously mentioned Fenton-induced free radical propagation, of which sufficient scavenging is dependent upon adequate epithelial levels of vitamin E. Increases in epithelial free radical load, combined with a vitamin E-related transport deficiency, would therefore be reflected by a decrease in vitamin E-like metabolites as anti-oxidants, as well as decreases in the reduced carboxylated isoforms resulting from hepatic uptake and P450-mediated metabolism. It has recently been shown that the uptake of Vitamin E into CaCo-2 colonic epithelial cells is a saturable process, heavily dependent upon a protein-mediated event (27). Because protein transporters are in essence enzymes, and follow typical Michaelis-Menton kinetics, the rate at which vitamin E can be taken up into colonic epithelial cells would reach a maximal velocity (Vmax), which may not be capable of providing a sufficient anti-oxidant protective effect for the development of OC. At some point in time, therefore, increasing rates of oxidative stress above the rate at which vitamin E can be transported from the diet will deplete the endogenous pool.

### Discovery and identification of differentially expressed metabolites in ovarian cancer-positive versus normal healthy controls

*Clinical Samples.* In order to determine whether there are biochemical markers of a given health-state in a particular population, a group of patients representative of the health-state (i.e. a particular disease) and a group of "normal" counterparts are required. Biological samples taken from the patients in a particular health-state category can then be compared to equivalent samples taken from the normal population with the objective of identifying differences between the two groups, by extracting and analyzing the samples using various analytical platforms including, but not limited to, FTMS and LC-MS. The biological samples originate from blood (serum/plasma).

For the ovarian cancer diagnostic assay described herein, serum samples were obtained from representative populations of healthy ovarian cancer-negative individuals and professionally diagnosed ovarian cancer-positive patients. Throughout this application, the term "serum" will be used, but it will be obvious to those skilled in the art that plasma or whole blood or a sub-fraction of whole blood may also be used in the method. The biochemical markers of ovarian cancer described in the invention were derived from the analysis of 20 serum samples from ovarian cancer positive patients and 25 serum samples from healthy controls. In subsequent validation tests, 539 control samples (not diagnosed with ovarian cancer; 289 subjects using the C28 HTS panel, and another 250 using the 31 molecule HTS panel) and 241 ovarian cancer samples were assessed. All samples were single time-point collections, while 289 ovarian cancer samples were taken either immediately prior to or immediately following surgical resection of a tumor (prior to chemotherapy or radiation therapy). The 250 ovarian subset (shown in Figure 8) was collected following treatment (chemo, surgery or radiation).

*Non-Targeted Metabolomic Strategies.* Multiple non-targeted metabolomics strategies have been described in the scientific literature including NMR (28), GC-MS (29-31), LC-MS , and FTMS strategies (28, 32-34). The metabolic profiling strategy employed for the discovery of differentially expressed metabolites in this application was the non-targeted FTMS strategy invented by Phenomenome Discoveries Inc. (30, 34-37). Non-targeted analysis involves the measurement of as many molecules in a sample as possible, without any prior knowledge or selection of components prior to the analysis. Therefore, the potential for non-targeted analysis to discover novel metabolite biomarkers is high versus targeted methods, which detect a predefined list of molecules. The present invention uses a non-targeted method to identify metabolite components that differ between ovarian cancer-positive and healthy individuals, followed by the development of a high-throughput targeted assay for a subset of the metabolites identified from the non-targeted analysis. However, it would be obvious to anyone skilled in the art that other metabolite profiling strategies could potentially be used to discover some or all of the differentially regulated metabolites disclosed in this application, and that the metabolites described herein, however discovered or measured, represent unique chemical entities that are independent of the analytical technology that may be used to detect and measure them.

*Sample Processing.* When a blood sample is drawn from a patient there are several ways in which the sample can be processed. The range of processing can be as little as none (i.e. frozen whole blood) or as complex as the isolation of a particular cell type. The most common and routine procedures involve the preparation of either serum or plasma from whole blood. All blood sample processing methods, including spotting of blood samples onto solid-phase supports, such as filter paper or other immobile materials, are also contemplated by the invention.

*Sample Extraction.* The processed blood sample described above is then further processed to make it compatible with the analytical technique to be employed in the detection and measurement of the biochemicals contained within the processed blood sample (in our case, a serum sample). The types of processing can range from as little as no further processing to as complex as differential extraction and chemical derivatization. Extraction methods may include, but are not limited to, sonication, soxhlet extraction, microwave assisted extraction (MAE), supercritical fluid extraction (SFE), accelerated solvent extraction (ASE), pressurized liquid extraction (PLE), pressurized hot water extraction (PHWE), and/or surfactant assisted extraction (PHWE) in common solvents such as methanol, ethanol, mixtures of alcohols and water, or organic solvents such as ethyl acetate or hexane. The preferred method of extracting metabolites for FTMS non-targeted analysis is to perform a liquid/liquid extraction whereby non-polar metabolites dissolve in an organic solvent and polar metabolites dissolve in an aqueous solvent. The metabolites contained within the serum samples used in this application were separated into polar and non-polar extracts through sonication and vigorous mixing (vortex mixing).

*Mass spectrometry analysis of extracts.* Extracts of biological samples are amenable to analysis on essentially any mass spectrometry platform, either by direct injection or following chromatographic separation. Typical mass spectrometers are comprised of a source, which ionizes molecules within the sample, and a detector for detecting the ionized particles. Examples of common sources include electron impact, electrospray ionization (ESI), atmospheric pressure chemical ionization (APCI), matrix assisted laser desorption ionization (MALDI), surface enhanced laser desorption ionization (SELDI), and derivations thereof. Common ion detectors can include quadrupole-based systems, time-of-flight (TOF), magnetic sector, ion cyclotron, and derivations thereof.

The present invention will be further illustrated in the following examples.

### Example 1: Identification of Differentially Expressed Metabolites

The invention described herein involved the analysis of serum extracts from 45 individuals (20 with ovarian cancer, 25 healthy controls) by direct injection into a FTMS and ionization by either ESI or APCI in both positive and negative modes. The advantage of FTMS over other MS-based platforms is the high resolving capability that allows for the separation of metabolites differing by only hundredths of a Dalton, many which would be missed by lower resolution instruments. Sample extracts were diluted either three or six-fold in ethanol:0.1% (v/v) ammonium hydroxide (50:50, v/v) for negative ionization modes, or in methanol:0.1% (v/v) formic acid (50:50, v/v) for positive ionization modes. For APCI, sample extracts were directly injected without diluting. All analyses were performed on a Bruker Daltonics APEX III FTMS equipped with a 7.0 T actively shielded superconducting magnet (Bruker Daltonics, Billerica, MA). Samples were directly injected using electrospray ionization (ESI) and atmospheric pressure chemical ionization (APCI) at a flow rate of 600 µL per hour. Ion transfer/detection parameters were optimized using a standard mix of serine, tetra-alanine, reserpine, Hewlett-Packard tuning mix, and the adrenocorticotrophic hormone fragment 4-10. In addition, the instrument conditions were tuned to optimize ion intensity and broad-band accumulation over the mass range of 100-1000 amu according to the instrument manufacturer's recommendations. A mixture of the abovementioned standards was used to internally calibrate each sample spectrum for mass accuracy over the acquisition range of 100-1000 amu.

In total six separate analyses comprising combinations of extracts and ionization modes were obtained for each sample:

### Aqueous Extract

1. Positive ESI (analysis mode 1101)
2. Negative ESI (analysis mode 1102)

### Organic Extract

3. Positive ESI (analysis mode 1201)
4. Negative ESI (analysis mode 1202)
5. Positive APCI (analysis mode 1203)
6. Negative APCI (analysis mode 1204)

*Mass Spectrometry Data Processing.* Using a linear least-squares regression line, mass axis values were calibrated such that each internal standard mass peak had a mass error of < 1 ppm compared with its theoretical mass. Using XMASS software from Bruker Daltonics Inc., data file sizes of 1 megaword were acquired and zero-filled to 2 megawords. A sinm data transformation was performed prior to Fourier transform and magnitude calculations. The mass spectra from each analysis were integrated, creating a peak list that contained the accurate mass and absolute intensity of each peak. Compounds in the range of 100-2000 m/z were analyzed. In order to compare and summarize data across different ionization modes and polarities, all detected mass peaks were converted to their corresponding neutral masses assuming hydrogen adduct formation. A self-generated two-dimensional (mass vs. sample intensity) array was then created using *DISCOVA*metrics™ software (Phenomenome Discoveries Inc., Saskatoon, SK, Canada). The data from multiple files were integrated and this combined file was then processed to determine all of the unique masses. The average of each unique mass was determined, representing the y-axis. A column was created for each file that was originally selected to be analyzed, representing the x-axis. The intensity for each mass found in each of the files selected was then filled into its representative x,y coordinate. Coordinates that did not contain an intensity value were left blank. Once in the array, the data were further processed, visualized and interpreted, and putative chemical identities were assigned. Each of the spectra were then peak picked to obtain the mass and intensity of all metabolites detected. These data from all of the modes were then merged to create one data file per sample. The data from all 45 samples were then merged and aligned to create a two-dimensional metabolite array in which each sample is represented by a column and each unique metabolite is represented by a single row. In the cell corresponding to a given metabolite sample combination, the intensity of the metabolite in that sample is displayed. When the data is represented in this format, metabolites showing differences between groups of samples (i.e., normal and cancer) can be determined.

*Advanced Data Interpretation.* A student's T-test was used to select for metabolites that differ between the normal and the ovarian cancer-positive samples (p<0.05). Four hundred and twenty four metabolites met this criterion (shown in Table 1). These are all features that differ statistically between the two populations and therefore have potential diagnostic utility. The features are described by their accurate mass and analysis mode (1204, organic extract and negative APCI), which together are sufficient to provide the putative molecular formulas and chemical characteristics (such as polarity and putative functional groups) of each metabolite. Table 1 also shows the average biomarker intensities and standard deviations of the intensities in the normal and ovarian samples. A log(2) ratio of the metabolite intensities (normal/ovarian) is shown in the far right column. By definition, since each of the metabolites in Table 1 shows a statistically significant difference (p<0.05) between the ovarian and control populations, each mass alone could be individually used to determine whether the health state of a person is "normal" or "ovarian" in nature. For example, this diagnosis could be performed by determining optimal cut-off points for each of the masses in Table 1, and by comparing the relative intensity of the biomarker in an unknown sample to the levels of the marker in the normal and ovarian population, a likelihood ratio for either being ovarian-positive or normal calculated for the unknown sample. This approach could be used individually for any or all of the masses listed in Table 1. Alternatively, this approach could be used on each mass, and then a combined average likelihood score based upon all the masses used.

Similar approaches to the above example would include any methods that use each or all of the masses to generate an averaged or standardized value representing all measure biomarker intensities for ovarian cancer. For example, the intensity of each mass would be measured, and then either used directly or following a normalization method (such as mean normalization, log normalization, Z-score transformation, min-max scaling, etc) to generate a summed or averaged score. Such sums or averages will differ significantly between the ovarian and normal populations, allowing cut-off scores to be used to predict the likelihood of ovarian cancer or normality in future unclassified samples. The cutoff scores themselves, whether for individual masses or for averages or standardized averages of all the masses in Table 1, can be selected using standard operator-receiver characteristic calculations.

A third example in which all masses listed in Table 1 could be used to provide a diagnostic output would be through the use of either a multivariate supervised or unsupervised classification or clustering algorithms. Similar to those listed below for optimal feature set selection, multivariate classification methods such as principal component analysis (PCA) and hierarchical clustering (HCA) (both unsupervised, ie, the algorithm does not know which samples belong to which disease variable), and supervised methods such as supervised PCA, partial least squared discriminant analysis (PLSDA), logistic regression, artificial neural networks (ANNs), support vector machine (SVMs), Bayesian methods and others (see 38 for review), perform optimally with more features. This is shown in the example in Figure 10 in which a supervised shrunken centroid approach was used to generate a plot of how many of the masses in Table 1 were required for optimal diagnostic classification. The figure shows that the lowest misclassification rate is achieved with all 424 masses (listed across the top of the figure), and that by increasing the threshold of the algorithm, the use of fewer metabolites results in a higher misclassification rate. Therefore, all 424 masses used collectively together results in the highest degree of diagnostic accuracy.

However, the incorporation and development of 424 signals into a commercially useful assay is impractical, and therefore supervised methods such as those listed above are often employed to determine the fewest number of features required to maintain an acceptable level of diagnostic accuracy. In this application, no supervised training classifiers were used to narrow the list further; rather, the list was reduced to 37 (see Table 2) based on univariate analysis, ¹³C filtering, and mode selection. Any other subset from the 424 masses listed in Table 1 can be used according to the present invention to develop a assay for detecting ovarian cancer. A subset of 30 metabolite markers is listed in Table 35. Furthermore, a subset of 29 metabolite markers is listed in Table 3. Alternatively, several supervised methods also exist, of which any one could have been used to identify an alternative subset of masses, including artificial neural networks (ANNs), support vector machines (SVMs), partial least squares discriminant analysis (PLSDA), sub-linear association methods, Bayesian inference methods, supervised principal component analysis, shrunken centroids, or others (see (38) for review).

### Example 2: Discovery of metabolites associated with ovarian cancer using a FTMS non-targeted metabolomic approach.

The identification of metabolites that can distinguish ovarian cancer patient serum from healthy control serum began with the generation of comprehensive metabolomic profiles of 20 ovarian cancer patients and 25 controls, as described in Example 1. The full dataset comprised 1,244 sample-specific masses, of which 424 showed p-values of less than 0.05 when the data was log(2) transformed and a student's t-test between the ovarian cancer samples and controls performed (Table 1). Each of these masses is statistically significant in discriminating between the ovarian cancer and control cohorts, and therefore has potential diagnostic utility. In addition any subset of the 424-metabolite markers has potential diagnostic utility. Table 1 shows these masses ordered according to the p-value (with the lowest p-values at the beginning of the table).

A statistical analysis technique called principal component analysis (PCA) was used to examine the variance within a multivariate dataset. This method is referred to as "unsupervised", meaning that the method is unaware of which samples belong to which cohorts. The output of a PCA analysis is a two or three-dimensional plot that projects a single point for each sample on the plot according to its variance. The more closely together that points cluster, the lower the variance is between the samples, or the more similar the samples are to each other based on the data. In Figure 1, PCA was first performed on the complete set of 1,244 masses, and the points colored according to disease state. Even with no filtering of masses according to significance or p-value, the PCA plot indicates that there is a strong metabolic signature present that is capable of discriminating the ovarian cancer samples from the controls. To identify the maximum number of masses with statistically significant differences in intensity between the ovarian cancer and control samples, a student's t-test was performed, resulting in 424 metabolites with p-values less than 0.05. The PCA plot in Figure 1B was generated using these 424 metabolites, which shows more tightly clustered groups, particularly for the control cohort (black). This further shows that the 424 masses not only retain, but improve upon the ability to discriminate between the two groups.

However, the incorporation of all 424 masses with p<0.05 into a routine clinical screening method is not practical. As described above, any number of statistical methods, including both supervised and non-supervised methods, could be used to extract subsets of these 424 masses as optimal diagnostic markers, and various methods would yield slightly different results. A subset of 37 metabolites (see Table 2) was selected from the list of 424 as one potential panel of ovarian cancer screening markers. The 37 metabolites were selected by filtering the data for masses with p-values less than 0.0001, removing all ¹³C isotopes, and excluding metabolites *not* detected in mode 1204. The list of 37 metabolites are shown in Table 2, and include masses 440.3532, 446.3413, 448.3565, 450.3735, 464.3531, 466.3659, 468.3848, 474.3736, 478.405, 484.3793, 490.3678, 492.3841, 494.3973, 502.4055, 504.4195, 510.3943, 512.4083, 518.3974, 520.4131, 522.4323, 530.437, 532.4507, 534.3913, 538.427, 540.4393, 548.4442, 550.4609, 558.4653, 566.4554, 574.4597, 576.4762, 578.493, 590.4597, 592.4728, 594.4857, 596.5015, 598.5121, where a +/- 5 ppm difference would indicate the same metabolite. A PCA plot based solely on these masses, is shown in Figure 2A, which indicates a high degree of separation between the ovarian cancer and the control samples along the PC1 axis. Since the PC1 axis of this dataset is capturing 80% of the overall variance, the PC1 position of every sample could be used as a diagnostic score for each patient. A distribution of the PC1 scores of every sample for each cohort is shown in Figure 2B, which shows the number of ovarian cancer samples and controls that have PC1 scores falling within six binned ranges. If the origin of the PCA plot in Figure 2A is used as a cutoff point, one can see that two of the ovarian cancer patients cluster with the control side of the distribution, while three controls cluster with the ovarian cancer side. This suggests an approximate sensitivity of 90% and specificity of 88%.

The PCA plot does not adequately allow one to visualize the actual intensities of the metabolites responsible for the separation of the clusters. A second statistical method was therefore used, called hierarchical clustering (HCA), to arrange the patient samples into groups based on a Euclidean distance measurements using the said 37 metabolites, which themselves were clustered using a Pearson correlation distance measurement. The resulting metabolite array is shown in Figure 3, and clearly reiterates the results observed with the PCA analysis, that is, the ovarian cancer and control cohorts are clearly discernable, with two ovarian cancer patients clustering within the control cohort, and three controls clustering within the ovarian cancer cohort. The array itself is comprised of cells representing the log(2) intensity from the FTMS, where white indicates metabolites with zero intensity, and increasing shades of grey indicate metabolites with increasing intensity values, respectively. It is clear that the 37 metabolites are all absent or relatively lower in intensity in the ovarian cancer cohort relative to the controls. The graph in Figure 4 further illustrates this point by plotting the average log(2) intensity (subsequently scaled between zero and one), of the 37 metabolites (± 1 s.d.).

### Example 3: Independent method confirmation of discovered metabolites

The metabolites and their associations with the clinical variables described in Example 1 are further confirmed using an independent mass spectrometry system. Representative sample extracts from each variable group are re-analyzed by LC-MS using an HP 1050 high-performance liquid chromatography (HPLC), or equivalent, interfaced to an ABI Q-Star (Applied Biosystems Inc., Foster City, CA), or equivalent, mass spectrometer to obtain mass and intensity information for the purpose of identifying metabolites that differ in intensity between the clinical variables under investigation. This is also a non-targeted approach, which provides retention time indices (time it takes for metabolites to elute off the HPLC column), and allows for tandem MS structural investigation. In this case, to verify that the sample extracts from the ovarian cancer patients and the controls did indeed have differential abundances of said markers, selected extracts from each cohort were analyzed independently using said approach. Of the 37 said metabolites described previously, 29 were detected across a set of 10 ovarian cancer and 10 control samples. A PCA plot based on these 29 masses is shown in Figure 5. The results suggested that the 29 metabolites (see Table 3), as detected on the TOF MS and include masses 446.3544, 448.3715, 450.3804, 468.3986, 474.3872, 476.4885, 478.4209, 484.3907, 490.3800, 492.3930, 494.4120, 502.4181, 504.4333, 512.4196, 518.4161, 520.4193, 522.4410, 530.4435, 532.4690, 538.4361, 540.4529, 550.4667, 558.4816, 574.4707, 578.5034, 592.4198, 594.5027, 596.5191, 598.5174, where a+/- 5 ppm difference would indicate the same metabolite, were clearly differentially expressed, as evidenced by complete separation of the 10 ovarian cancer samples from the 10 controls. A bar graph of the 29 metabolites is shown in Figure 6, which reaffirms a clear deficiency or reduction of these molecules in the ovarian cancer cohort relative to the controls.

The retention times of the 29 metabolites shown in Figure 6 ranged between approximately 15 to 18 minutes under the chromatographic conditions. To further illustrate the specificity of molecules eluting within this time window for ovarian cancer, averaged extracted mass spectra between 15 and 20 minutes for the controls, the ovarian cancers, and the net difference between the two cohorts were generated as shown in Figure 7. By comparing the top panel (controls) to the middle panel (ovarian cancer), it is evident that the peaks are at equal heights in both samples until approximately mass 400 is reached, at which point peaks are clearly detectable in the control group (upper panel), but not in the ovarian cancer subjects (middle panel). The bottom panel illustrates the net difference, which includes the 29 masses that overlap with the 3 7 identified in the FTMS data.

### Example 4: MSMS Fragmentation and structural investigation of selected ovarian cancer metabolite markers

The following example describes the tandem mass spectrometry analysis of a subset of the ovarian markers. The general principle is based upon the selection and fragmentation of each of the parent ions into a pattern of daughter ions. The fragmentation occurs within the mass spectrometer through a process called collision-induced dissociation, wherein an inert gas (such as argon) is allowed to collide with the parent ion resulting in its fragmentation into smaller components. The charge will then travel with one of the corresponding fragments. The pattern of resulting fragment or "daughter ions" represents a specific "fingerprint" for each molecule. Differently structured molecules (including those with the same formulas) will produce different fragmentation patterns, and therefore represents a very specific way of identifying the molecule. By assigning accurate masses and formulas to the fragment ions, structural insights about the molecules can be determined.

In this example, MSMS analysis was carried out on a subset of 31 ovarian markers (from Tables 2 and 3). The resulting fragment ions for each of the selected parent ions are listed in Tables 4 through 34. The parent ion is listed at the top of each table (as its neutral mass), and the subsequent fragments shown as negatively charged ions [M-H]. The intensity (in counts and percent) is shown in the middle and right columns, respectively. The specific retention time (from the high performance liquid chromatography) is shown at the top of the middle column. The ovarian markers all had retention times under the chromatographic conditions used (see methods below) between 16 and 18 minutes.

Proposed structures based upon interpretation of the fragmentation patterns are summarized in Table 35. Subsequent Tables 36 through 65 list the fragment masses and proposed structures of each fragment for each parent molecule. The masses in the table are given as the nominal detected mass [M-H] and the proposed molecular formula is given for each fragment. In addition, the right-hand column indicates the predicted neutral fragment losses.

Interpretation of the MSMS data revealed that the metabolite markers are structurally related to the gamma-tocopherol form of vitamin E, in that they comprise a chroman ring-like moiety and phytyl side-chain. However, these molecules possess several important differences from gamma tocopherol:
a). omega-carboxylated phytyl sidechains (carboxylation at the terminal carbon position of the phytyl chain).
b). semi-saturated and open chroman ring-like systems
c). increased carbon number due to potential hydrocarbon chain addition to the ring system.
Based on the similarity to gamma-tocopherol and the presence of the omega-carboxyl moieties, the class of novel metabolites was named "gamma-tocoenoic acids."

HPLC analysis were carried out with a high performance liquid chromatograph equipped with quaternary pump, automatic injector, degasser, and a Hypersil ODS column (5 µm particle size silica, 4.6 i.d x 200 mm) and semi-prep column (5 µm particle size silica, 9.1 i.d x 200 mm), with an inline filter. Mobile phase: linear gradient H₂O-MeOH to 100% MeOH in a 52 min period at a flow rate 1.0 ml/min.

Eluate from the HPLC was analyzed using an ABI QSTAR® XL mass spectrometer fitted with an atmospheric pressure chemical ionization (APCI) source in negative mode. The scan type in full scan mode was time-of-flight (TOF) with an accumulation time of 1.0000 seconds, mass range between 50 and 1500 Da, and duration time of 55 min. Source parameters were as follows: Ion source gas 1 (GS1) 80; Ion source gas 2 (GS2) 10; Curtain gas (CUR) 30; Nebulizer Current (NC) -3.0; Temperature 400°C; Declustering Potential (DP) -60; Focusing Potential (FP) -265; Declustering Potential 2 (DP2) -15. In MS/MS mode, scan type was product ion, accumulation time was 1.0000 seconds, scan range between 50 and 650 Da and duration time 55 min. For MSMS analysis, all source parameters are the same as above, with collision energy (CE) of -35 V and collision gas (CAD, nitrogen) of 5 psi.

### Example 5: Targeted triple-quadrupole assay for selected ovarian markers

The following example describes the development of a high-throughput screening (HTS) assay based upon triple-quadrupole mass spectrometry for a subset of the ovarian markers. The preliminary method was initially established to determine the ratio of six of the ovarian 28-carbon containing metabolites to an internal standard molecule added during the extraction procedure. This is similar to the HTS method reported in applicant's co-pending CRC/Ovarian PCT application published on March 22, 2007 (WO 2007/030928). The ability of this method to differentiate between ovarian cancer patients and subjects without ovarian cancer is shown in Figure 8, where the 20 ovarian cancer subjects used to make the initial discovery are compared to 289 disease-free subjects. The six C28 carbon molecules (neutral masses 450 (C28H5004), 446 (C28H46O4), 468 (C28H52O5), 448 (C28H48O4), 464 (C28H48O5) and 466 (C28H50O5) were validated to be significantly lower in the serum of the ovarian patients versus the controls. The p-values for each of the molecules are shown in Table 66.

Based upon completion of MSMS analysis of the remaining molecules, a new HTS triple-quadrupole method was developed to analyze a larger subset of the ovarian markers. This expanded triple-quadrupole method measures a comprehensive panel of the gamma Tocoenoic acids, and includes the metabolites listed in Table 67. The method measures the daughter fragment ion of each parent, as well an internal standard molecule (see methods below). The biomarker peak areas are then normalized by dividing by the internal standard peak areas.

The method was then used to validate the reduction of gamma tocoenoic acids in a subsequent independent population of controls and ovarian cancer positive subjects. The graph in Figure 9 shows the average difference in signal intensity for each of the gamma tocoenoic acids in ovarian cancer patients relative to controls. The cohorts comprised 250 controls (i.e. not diagnosed with ovarian cancer at the time samples were taken, grey bars), and 241 ovarian cancer subjects (black bars). The averages of the original 20 ovarian cancer discovery samples (white bars) are also shown for this method. The results confirm that serum from ovarian cancer patients has low levels of gamma-tocoenoic acids relative to disease-free controls. The p-values for each metabolite (250 controls versus 241 ovarian cancers) are shown for each marker in Table 67 as well as in Figure 9.

Serum samples are extracted as described for non-targeted FTMS analysis. The ethyl acetate organic fraction is used for the analysis of each sample. 15uL of internal standard is added (1ng/mL of (24-¹³C)-Cholic Acid in methanol) to each sample aliquot of 120uL ethyl acetate fraction for a total volume of 135uL. The autosampler injects 100uL of the sample by flow-inj ection analysis into the 4000QTRAP. The carrier solvent is 90%methanol: 10%ethyl acetate, with a flow rate of 3 60uL/min into the APCI source.

The MS/MS HTS method was developed on a quadrupole linear ion trap ABI 4000QTrap mass spectrometer equipped with a TurboV™ source with an APCI probe. The source gas parameters were as follows: CUR: 10.0, CAD: 6, NC: -3.0, TEM: 400, GS1: 15, interface heater on. "Compound" settings were as follows: entrance potential (EP): -10, and collision cell exit potential (CXP): -20.0. The method is based on the multiple reaction monitoring (MRM) of one parent ion transition for each metabolite and a single transition for the internal standard. Each of the transitions is monitored for 250 ms for a total cycle time of 2.3 seconds. The total acquisition time per sample is approximately 1 min. The method is similar to that described in the PCT case referred to above (WO 2007/030928), but was expanded to include a larger subset of the molecules as shown in Table 67.

The present invention has been described with regard to one or more embodiments. However, it will be apparent to persons skilled in the art that a number of variations and modifications can be made without departing from the scope of the invention as defined in the claims.

### REFERENCES

1. Screening for ovarian cancer: recommendation statement. Ann Fam Med 2004;2: 260-2.
2. Chu CS, Rubin SC. Screening for ovarian cancer in the general population. Best Pract Res Clin Obstet Gynaecol 2005.
3. Hanna L, Adams M. Prevention of ovarian cancer. Best Pract Res Clin Obstet Gynaecol 2005.
4. Rosenthal A, Jacobs I. Familial ovarian cancer screening. Best Pract Res Clin Obstet Gynaecol 2005.
5. Theriault A, Chao JT, Wang Q, Gapor A, Adeli K. Tocotrienol: a review of its therapeutic potential. Clin Biochem 1999;32: 309-19.
6. Serbinova E, Kagan V, Han D, Packer L. Free radical recycling and intramembrane mobility in the antioxidant properties of alpha-tocopherol and alpha-tocotrienol. Free Radic Biol Med 1991;10: 263-75.
7. Lee BL, New AL, Ong CN. Simultaneous determination of tocotrienols, tocopherols, retinol, and major carotenoids in human plasma. Clin Chem 2003;49: 2056-66.
8. Birringer M, Pfluger P, Kluth D, Landes N, Brigelius-Flohe R. Identities and differences in the metabolism of tocotrienols and tocopherols in HepG2 cells. J Nutr 2002;132: 3113-8.
9. Bieri JG, Evarts RP. Gamma tocopherol: metabolism, biological activity and significance in human vitamin E nutrition. Am J Clin Nutr 1974;27: 980-6.
10. Traber MG. Determinants of plasma vitamin E concentrations. Free Radic Biol Med 1994;16: 229-39.
11. Princen HM, van Duyvenvoorde W, Buytenhek R, et al. Supplementation with low doses of vitamin E protects LDL from lipid peroxidation in men and women. Arterioscler Thromb Vasc Biol 1995;15: 325-33.
12. Schaffer S, Muller WE, Eckert GP. Tocotrienols: constitutional effects in aging and disease. J Nutr 2005;135: 151-4.
13. Winklhofer-Roob BM, van't Hof MA, Shmerling DH. Reference values for plasma concentrations of vitamin E and A and carotenoids in a Swiss population from infancy to adulthood, adjusted for seasonal influences. Clin Chem 1997;43: 146-53.
14. Qureshi AA, Qureshi N, Wright JJ, et al. Lowering of serum cholesterol in hypercholesterolemic humans by tocotrienols (palmvitee). Am J Clin Nutr 1991;53: 1021S-6S.
15. Qureshi AA, Peterson DM, Hasler-Rapacz JO, Rapacz J. Novel tocotrienols of rice bran suppress cholesterogenesis in hereditary hypercholesterolemic swine. J Nutr 2001;131: 223-30.
16. Sen CK, Khanna S, Roy S. Tocotrienols: Vitamin E beyond tocopherols. Life Sci 2006;78: 2088-98.
17. Khanna S, Roy S, Ryu H, et al. Molecular basis of vitamin E action: tocotrienol modulates 12-lipoxygenase, a key mediator of glutamate-induced neurodegeneration. J Biol Chem 2003;278: 43508-15.
18. Qureshi AA, Sami SA, Salser WA, Khan FA. Synergistic effect of tocotrienol-rich fraction (TRF(25)) of rice bran and lovastatin on lipid parameters in hypercholesterolemic humans. J Nutr Biochem 2001;12: 318-29.
19. Adachi H, Ishii N. Effects of tocotrienols on life span and protein carbonylation in Caenorhabditis elegans. J Gerontol A Biol Sci Med Sci 2000;55: B280-5.
20. Nesaretnam K, Guthrie N, Chambers AF, Carroll KK. Effect of tocotrienols on the growth of a human breast cancer cell line in culture. Lipids 1995;30: 1139-43.
21. McIntyre BS, Briski KP, Tirmenstein MA, Fariss MW, Gapor A, Sylvester PW. Antiproliferative and apoptotic effects of tocopherols and tocotrienols on normal mouse mammary epithelial cells. Lipids 2000;35: 171-80.
22. Jiang Q, Ames BN. Gamma-tocopherol, but not alpha-tocopherol, decreases proinflammatory eicosanoids and inflammation damage in rats. Faseb J 2003;17: 816-22.
23. Jiang Q, Elson-Schwab I, Courtemanche C, Ames BN. gamma-tocopherol and its major metabolite, in contrast to alpha-tocopherol, inhibit cyclooxygenase activity in macrophages and epithelial cells. Proc Natl Acad Sci U S A 2000;97: 11494-9.
24. Helzlsouer KJ, Alberg AJ, Norkus EP, Morris JS, Hoffman SC, Comstock GW. Prospective study of serum micronutrients and ovarian cancer. Journal of the National Cancer Institute 1996;88: 32-7.
25. Pan SY, Ugnat AM, Mao Y, Wen SW, Johnson KC. A case-control study of diet and the risk of ovarian cancer. Cancer Epidemiol Biomarkers Prev 2004;13: 1521-7.
26. Babbs CF. Free radicals and the etiology of colon cancer. Free Radic Biol Med 1990;8: 191-200.
27. Reboul E, Klein A, Bietrix F, et al. Scavenger receptor class B type I (SR-BI) is involved in vitamin E transport across the enterocyte. J Biol Chem 2006;281: 4739-45.
28. Reo NV. NMR-based metabolomics. Drug Chem Toxicol 2002;25: 375-82.
29. Fiehn O, Kopka J, Dormann P, Altmann T, Trethewey RN, Willmitzer L. Metabolite profiling for plant functional genomics. Nat Biotechnol 2000;18: 1157-61.
30. Hirai MY, Yano M, Goodenowe DB, et al. Integration of transcriptomics and metabolomics for understanding of global responses to nutritional stresses in Arabidopsis thaliana. Proc Natl Acad Sci U S A 2004; 101: 10205-10.
31. Roessner U, Luedemann A, Brust D, et al. Metabolic profiling allows comprehensive phenotyping of genetically or environmentally modified plant systems. Plant Cell 2001;13: 11-29.
32. Castrillo JI, Hayes A, Mohammed S, Gaskell SJ, Oliver SG. An optimized protocol for metabolome analysis in yeast using direct infusion electrospray mass spectrometry. Phytochemistry 2003;62: 929-37.
33. Fiehn O. Metabolomics--the link between genotypes and phenotypes. Plant Mol Biol 2002;48: 155-71.
34. Aharoni A, Ric de Vos CH, Verhoeven HA, et al. Nontargeted metabolome analysis by use of Fourier Transform Ion Cyclotron Mass Spectrometry. Omics 2002;6: 217-34.
35. Hirai MY, Klein M, Fujikawa Y, et al. Elucidation of gene-to-gene and metabolite-to-gene networks in arabidopsis by integration of metabolomics and transcriptomics. J Biol Chem 2005;280: 25590-5.
36. Murch SJ, Rupasinghe HP, Goodenowe D, Saxena PK. A metabolomic analysis of medicinal diversity in Huang-qin (Scutellaria baicalensis Georgi) genotypes: discovery of novel compounds. Plant Cell Rep 2004;23: 419-25.
37. Tohge T, Nishiyama Y, Hirai MY, et al. Functional genomics by integrated analysis of metabolome and transcriptome of Arabidopsis plants over-expressing an MYB transcription factor. Plant J 2005;42: 218-35.
38. Wu B, Abbott T, Fishman D, et al. Comparison of statistical methods for classification of ovarian cancer using mass spectrometry data. Bioinformatics 2003; 19: 1636-43.
39. Sontag TJ, Parker RS. Cytochrome P450 omega-hydroxylase pathway of tocopherol catabolism. Novel mechanism of regulation of vitamin E status. J Biol Chem 2002;277: 25290-6.
40. Blakeborough MH, Owen RW, Bilton RF. Free radical generating mechanisms in the colon: their role in the induction and promotion of colorectal cancer? Free Radic Res Commun 1989;6: 359-67.
41. Graf E, Eaton JW. Dietary suppression of colonic cancer. Fiber or phytate? Cancer 1985;56: 717-8.
42. Campbell S, Stone W, Whaley S, Krishnan K. Development of gamma (gamma)-tocopherol as a colorectal cancer chemopreventive agent. Crit Rev Oncol Hematol 2003;47: 249-59.
43. Rubbo H, Radi R, Trujillo M, et al. Nitric oxide regulation of superoxide and peroxynitrite-dependent lipid peroxidation. Formation of novel nitrogen-containing oxidized lipid derivatives. J Biol Chem 1994;269: 26066-75.
44. Radi R, Beckman JS, Bush KM, Freeman BA. Peroxynitrite-induced membrane lipid peroxidation: the cytotoxic potential of superoxide and nitric oxide. Arch Biochem Biophys 1991;288: 481-7.
45. Cooney RV, Franke AA, Harwood PJ, Hatch-Pigott V, Custer LJ, Mordan LJ. Gamma-tocopherol detoxification of nitrogen dioxide: superiority to alpha-tocopherol. Proc Natl Acad Sci USA 1993;90: 1771-5.
46. Stone WL, Papas AM, LeClair IO, Qui M, Ponder T. The influence of dietary iron and tocopherols on oxidative stress and ras-p21 levels in the colon. Cancer Detect Prev 2002;26: 78-84.

**Table 1: List of 424 masses generated from FTMS analysis of serum from ovarian cancer patients and controls (p<0.05, student's t-test between ovarian cancer positive and control cohort).**

| | | | **Normal** | **Normal** | **Ovarian** | **Ovarian** | |
|---|---|---|---|---|---|---|---|
| **Detected Mass** | **Analysis Mode** | **P-Value** | **AVG** | **SD** | **AVG** | **SD** | **log(2) ratio : N/O** |
| 492.3841 | 1204 | 2.82E-08 | 2.28 | 0.63 | 0.79 | 0.84 | 2.87 |
| 590.4597 | 1204 | 4.23E-08 | 2.51 | 0.57 | 1.13 | 0.83 | 2.23 |
| 447.3436 | 1204 | 4.52E-08 | 1.17 | 0.79 | 0.00 | 0.00 | NA |
| 450.3735 | 1204 | 8.20E-08 | 2.28 | 0.48 | 0.92 | 0.91 | 2.47 |
| 502.4055 | 1204 | 9.62E-08 | 2.11 | 0.62 | 0.72 | 0.84 | 2.92 |
| 484.3793 | 1204 | 1.09E-07 | 1.77 | 0.70 | 0.44 | 0.71 | 4.03 |
| 577.4801 | 1204 | 1.10E-07 | 2.68 | 0.64 | 1.16 | 0.96 | 2.31 |
| 490.3678 | 1204 | 1.36E-07 | 1.67 | 0.71 | 0.40 | 0.63 | 4.21 |
| 548.4442 | 1204 | 2.36E-07 | 1.74 | 0.67 | 0.48 | 0.70 | 3.65 |
| 466.3659 | 1204 | 4.01E-07 | 2.48 | 0.67 | 1.00 | 0.99 | 2.47 |
| 494.3973 | 1204 | 4.59E-07 | 2.43 | 0.75 | 0.98 | 0.90 | 2.49 |
| 576.4762 | 1204 | 7.50E-07 | 4.03 | 0.73 | 2.76 | 0.73 | 1.46 |
| 592.4728 | 1204 | 7.99E-07 | 3.78 | 0.86 | 2.06 | 1.14 | 1.83 |
| 464.3531 | 1204 | 8.09E-07 | 2.33 | 0.63 | 1.02 | 0.90 | 2.30 |
| 467.3716 | 1204 | 1.37E-06 | 0.97 | 0.72 | 0.05 | 0.20 | 21.42 |
| 448.3565 | 1204 | 1.46E-06 | 2.30 | 0.62 | 1.08 | 0.85 | 2.14 |
| 574.4597 | 1204 | 1.58E-06 | 3.68 | 0.84 | 2.26 | 0.87 | 1.63 |
| 594.4857 | 1204 | 1.65E-06 | 4.95 | 0.90 | 3.34 | 1.04 | 1.48 |
| 595.4889 | 1204 | 1.84E-06 | 3.64 | 0.85 | 1.85 | 1.32 | 1.97 |
| 594.4878 | 1202 | 1.92E-06 | 3.15 | 0.94 | 1.47 | 1.10 | 2.14 |
| 518.3974 | 1204 | 2.04E-06 | 2.52 | 0.73 | 1.15 | 0.95 | 2.20 |
| 574.4638 | 1202 | 2.17E-06 | 1.65 | 0.88 | 0.41 | 0.56 | 4.00 |
| 504.4195 | 1204 | 2.42E-06 | 1.87 | 0.70 | 0.67 | 0.77 | 2.79 |
| 534.3913 | 1204 | 2.52E-06 | 1.05 | 0.72 | 0.11 | 0.34 | 9.85 |
| 576.4768 | 1202 | 2.76E-06 | 2.07 | 0.78 | 0.88 | 0.67 | 2.36 |
| 519.3329 | 1101 | 4.35E-06 | 2.57 | 0.57 | 1.37 | 0.95 | 1.88 |
| 532.4507 | 1204 | 4.62E-06 | 1.45 | 0.61 | 0.48 | 0.62 | 2.99 |
| 538.4270 | 1204 | 6.45E-06 | 3.63 | 0.76 | 2.22 | 1.09 | 1.64 |
| 566.4554 | 1204 | 7.29E-06 | 1.44 | 0.89 | 0.27 | 0.57 | 5.34 |
| 440.3532 | 1204 | 7.63E-06 | 0.92 | 0.73 | 0.05 | 0.24 | 17.30 |
| 520.4131 | 1204 | 8.72E-06 | 2.72 | 0.71 | 1.51 | 0.90 | 1.81 |
| 596.5015 | 1204 | 1.14E-05 | 5.56 | 1.05 | 3.91 | 1.18 | 1.42 |
| 597.5070 | 1202 | 1.20E-05 | 2.33 | 1.07 | 0.85 | 0.90 | 2.75 |
| 530.4370 | 1204 | 1.38E-05 | 1.65 | 0.79 | 0.52 | 0.75 | 3.21 |
| 541.3148 | 1101 | 1.46E-05 | 2.53 | 0.59 | 1.35 | 1.02 | 1.88 |
| 510.3943 | 1204 | 1.47E-05 | 1.12 | 0.71 | 0.22 | 0.46 | 5.06 |
| 474.3736 | 1204 | 1.58E-05 | 1.53 | 0.69 | 0.53 | 0.69 | 2.91 |
| 575.4631 | 1204 | 1.58E-05 | 2.32 | 0.96 | 0.97 | 0.87 | 2.38 |
| 578.4930 | 1204 | 1.66E-05 | 3.82 | 0.77 | 2.53 | 1.02 | 1.51 |
| 512.4083 | 1204 | 1.74E-05 | 2.34 | 1.08 | 0.91 | 0.85 | 2.57 |
| 597.5068 | 1204 | 1.76E-05 | 4.16 | 1.01 | 2.46 | 1.35 | 1.69 |
| 522.4323 | 1204 | 1.88E-05 | 2.84 | 0.76 | 1.71 | 0.81 | 1.66 |
| 478.4050 | 1204 | 1.93E-05 | 0.88 | 0.65 | 0.11 | 0.34 | 8.31 |
| 596.5056 | 1202 | 2.19E-05 | 3.58 | 1.14 | 1.93 | 1.16 | 1.85 |
| 593.4743 | 1204 | 2.28E-05 | 2.26 | 1.13 | 0.77 | 0.94 | 2.94 |
| 468.3848 | 1204 | 2.45E-05 | 3.14 | 0.78 | 1.94 | 0.93 | 1.62 |
| 598.5121 | 1204 | 2.53E-05 | 2.01 | 1.13 | 0.55 | 0.88 | 3.64 |
| 558.4653 | 1204 | 2.79E-05 | 4.36 | 0.61 | 3.40 | 0.78 | 1.29 |
| 550.4609 | 1204 | 3.35E-05 | 2.10 | 0.73 | 0.94 | 0.95 | 2.22 |
| 559.4687 | 1204 | 3.35E-05 | 2.94 | 0.60 | 1.86 | 0.96 | 1.58 |
| 578.4909 | 1202 | 3.86E-05 | 1.66 | 0.88 | 0.59 | 0.63 | 2.83 |
| 783.5780 | 1101 | 4.45E-05 | 3.92 | 0.46 | 3.11 | 0.73 | 1.26 |
| 850.7030 | 1203 | 4.45E-05 | 3.38 | 0.60 | 2.17 | 1.15 | 1.56 |
| 540.4393 | 1204 | 4.81E-05 | 3.41 | 0.96 | 2.08 | 1.01 | 1.64 |
| 446.3413 | 1204 | 4.92E-05 | 3.08 | 0.80 | 1.93 | 0.93 | 1.60 |
| 482.3605 | 1204 | 0.0001 | 0.81 | 0.70 | 0.08 | 0.37 | 9.71 |
| 521.4195 | 1204 | 0.0001 | 1.20 | 0.82 | 0.30 | 0.54 | 4.05 |
| 524.4454 | 1204 | 0.0001 | 1.06 | 0.81 | 0.18 | 0.47 | 5.79 |
| 540.4407 | 1202 | 0.0001 | 1.56 | 0.83 | 0.58 | 0.62 | 2.71 |
| 541.4420 | 1204 | 0.0001 | 1.96 | 0.80 | 0.89 | 0.84 | 2.20 |
| 579.4967 | 1204 | 0.0001 | 2.53 | 0.86 | 1.34 | 1.03 | 1.90 |
| 580.5101 | 1204 | 0.0001 | 2.41 | 0.78 | 1.31 | 0.95 | 1.84 |
| 610.4853 | 1204 | 0.0001 | 2.18 | 0.73 | 1.07 | 1.01 | 2.03 |
| 616.4670 | 1201 | 0.0001 | 1.50 | 0.91 | 0.42 | 0.70 | 3.59 |
| 749.5365 | 1202 | 0.0001 | 3.85 | 0.45 | 2.99 | 0.88 | 1.29 |
| 750.5403 | 1202 | 0.0001 | 2.82 | 0.44 | 1.89 | 0.98 | 1.49 |
| 784.5813 | 1101 | 0.0001 | 2.83 | 0.45 | 2.08 | 0.68 | 1.36 |
| 785.5295 | 1204 | 0.0001 | 3.02 | 0.36 | 2.46 | 0.49 | 1.23 |
| 814.5918 | 1202 | 0.0001 | 2.54 | 0.39 | 2.05 | 0.38 | 1.24 |
| 829.5856 | 1102 | 0.0001 | 4.40 | 0.50 | 3.61 | 0.74 | 1.22 |
| 830.5885 | 1102 | 0.0001 | 3.29 | 0.51 | 2.54 | 0.67 | 1.29 |
| 830.6539 | 1102 | 0.0001 | 2.48 | 0.35 | 1.86 | 0.60 | 1.33 |
| 851.7107 | 1203 | 0.0001 | 3.03 | 0.57 | 1.79 | 1.28 | 1.69 |
| 244.0560 | 1101 | 0.0002 | 1.52 | 1.13 | 2.76 | 0.82 | 0.55 |
| 306.2570 | 1204 | 0.0002 | 3.11 | 0.39 | 2.64 | 0.40 | 1.18 |
| 508.3783 | 1204 | 0.0002 | 0.97 | 0.78 | 0.18 | 0.43 | 5.55 |
| 513.4117 | 1204 | 0.0002 | 0.87 | 0.84 | 0.07 | 0.29 | 13.31 |
| 521.3479 | 1101 | 0.0002 | 2.32 | 0.38 | 1.50 | 0.90 | 1.55 |
| 536.4105 | 1204 | 0.0002 | 2.57 | 0.68 | 1.65 | 0.83 | 1.56 |
| 565.3393 | 1102 | 0.0002 | 4.16 | 0.48 | 3.36 | 0.83 | 1.24 |
| 570.4653 | 1203 | 0.0002 | 2.21 | 0.39 | 1.48 | 0.81 | 1.50 |
| 618.4836 | 1201 | 0.0002 | 1.50 | 1.04 | 0.42 | 0.69 | 3.59 |
| 757.5016 | 1204 | 0.0002 | 3.95 | 0.42 | 3.32 | 0.63 | 1.19 |
| 784.5235 | 1204 | 0.0002 | 3.74 | 0.35 | 3.21 | 0.51 | 1.16 |
| 852.7242 | 1204 | 0.0002 | 3.64 | 0.62 | 2.86 | 0.65 | 1.27 |
| 317.9626 | 1101 | 0.0003 | 0.85 | 1.21 | 2.20 | 1.03 | 0.39 |
| 523.3640 | 1101 | 0.0003 | 2.51 | 0.44 | 1.73 | 0.88 | 1.45 |
| 546.4305 | 1204 | 0.0003 | 0.80 | 0.80 | 0.07 | 0.30 | 12.16 |
| 555.3101 | 1102 | 0.0003 | 1.93 | 0.48 | 1.15 | 0.84 | 1.68 |
| 577.4792 | 1202 | 0.0003 | 0.73 | 0.68 | 0.09 | 0.27 | 8.52 |
| 726.5454 | 1204 | 0.0003 | 2.78 | 0.37 | 1.95 | 0.98 | 1.43 |
| 568.4732 | 1204 | 0.0004 | 2.00 | 1.01 | 0.88 | 0.95 | 2.27 |
| 824.6890 | 1203 | 0.0004 | 2.33 | 0.77 | 1.24 | 1.13 | 1.88 |
| 469.3872 | 1204 | 0.0005 | 1.04 | 0.73 | 0.29 | 0.59 | 3.62 |
| 534.4644 | 1204 | 0.0005 | 1.32 | 0.79 | 0.50 | 0.65 | 2.65 |
| 723.5198 | 1202 | 0.0005 | 3.06 | 0.64 | 2.05 | 1.13 | 1.49 |
| 886.5582 | 1102 | 0.0005 | 3.50 | 0.32 | 2.95 | 0.65 | 1.19 |
| 897.5730 | 1102 | 0.0005 | 2.26 | 0.49 | 1.58 | 0.72 | 1.43 |
| 226.0687 | 1102 | 0.0006 | 1.93 | 0.86 | 2.79 | 0.65 | 0.69 |
| 531.3123 | 1102 | 0.0006 | 2.38 | 0.30 | 1.81 | 0.70 | 1.32 |
| 558.4666 | 1202 | 0.0006 | 2.35 | 0.82 | 1.41 | 0.89 | 1.67 |
| 566.3433 | 1102 | 0.0006 | 2.43 | 0.49 | 1.77 | 0.71 | 1.38 |
| 569.4783 | 1204 | 0.0006 | 0.94 | 0.88 | 0.14 | 0.43 | 6.67 |
| 595.4938 | 1202 | 0.0006 | 1.56 | 1.14 | 0.49 | 0.67 | 3.20 |
| 876.7223 | 1203 | 0.0006 | 4.38 | 0.59 | 3.61 | 0.81 | 1.21 |
| 518.3182 | 1101 | 0.0007 | 2.39 | 0.32 | 1.63 | 0.98 | 1.46 |
| 537.4151 | 1204 | 0.0007 | 1.15 | 0.85 | 0.33 | 0.60 | 3.47 |
| 545.3460 | 1101 | 0.0007 | 2.45 | 0.48 | 1.59 | 1.04 | 1.54 |
| 552.3825 | 1201 | 0.0007 | 0.00 | 0.00 | 0.70 | 0.97 | 0.00 |
| 557.4533 | 1204 | 0.0007 | 1.47 | 0.64 | 0.70 | 0.78 | 2.10 |
| 572.4472 | 1204 | 0.0007 | 1.59 | 0.80 | 0.73 | 0.77 | 2.18 |
| 581.5130 | 1204 | 0.0007 | 0.96 | 0.80 | 0.20 | 0.50 | 4.69 |
| 699.5206 | 1204 | 0.0007 | 2.58 | 0.74 | 1.54 | 1.16 | 1.68 |
| 750.5434 | 1204 | 0.0007 | 3.83 | 0.57 | 2.86 | 1.16 | 1.34 |
| 787.5446 | 1204 | 0.0007 | 3.16 | 0.33 | 2.73 | 0.45 | 1.16 |
| 826.7051 | 1203 | 0.0007 | 4.43 | 0.61 | 3.65 | 0.83 | 1.21 |
| 596.4792 | 1203 | 0.0008 | 3.36 | 0.42 | 2.77 | 0.66 | 1.21 |
| 675.6358 | 1203 | 0.0008 | 3.37 | 0.37 | 2.80 | 0.67 | 1.20 |
| 727.5564 | 1204 | 0.0008 | 3.65 | 0.50 | 2.81 | 1.02 | 1.30 |
| 770.5108 | 1204 | 0.0008 | 3.19 | 0.41 | 2.53 | 0.79 | 1.26 |
| 506.3212 | 1202 | 0.0009 | 2.55 | 0.29 | 2.20 | 0.36 | 1.16 |
| 728.5620 | 1204 | 0.0009 | 2.99 | 0.36 | 2.35 | 0.80 | 1.27 |
| 813.5889 | 1202 | 0.0009 | 3.51 | 0.45 | 3.05 | 0.40 | 1.15 |
| 647.5740 | 1203 | 0.001 | 2.72 | 0.58 | 1.86 | 1.03 | 1.46 |
| 725.5376 | 1204 | 0.001 | 3.21 | 0.84 | 2.11 | 1.24 | 1.52 |
| 327.0325 | 1204 | 0.0011 | 2.59 | 0.31 | 2.01 | 0.76 | 1.29 |
| 496.3360 | 1101 | 0.0011 | 2.65 | 0.34 | 1.99 | 0.86 | 1.33 |
| 591.3542 | 1202 | 0.0011 | 4.23 | 0.45 | 3.74 | 0.48 | 1.13 |
| 648.5865 | 1203 | 0.0011 | 5.73 | 0.44 | 5.00 | 0.92 | 1.14 |
| 676.6394 | 1203 | 0.0011 | 2.24 | 0.36 | 1.50 | 0.99 | 1.49 |
| 805.5606 | 1101 | 0.0011 | 3.98 | 0.45 | 3.38 | 0.71 | 1.18 |
| 827.7086 | 1203 | 0.0011 | 3.70 | 0.60 | 2.85 | 1.01 | 1.30 |
| 887.5625 | 1102 | 0.0011 | 2.58 | 0.37 | 2.01 | 0.72 | 1.29 |
| 1016.9298 | 1203 | 0.0011 | 4.91 | 0.63 | 3.75 | 1.52 | 1.31 |
| 517.3148 | 1101 | 0.0012 | 4.35 | 0.36 | 3.61 | 0.98 | 1.20 |
| 551.4658 | 1204 | 0.0012 | 0.75 | 0.71 | 0.13 | 0.40 | 5.81 |
| 724.5245 | 1204 | 0.0012 | 3.42 | 0.69 | 2.44 | 1.19 | 1.40 |
| 755.4866 | 1204 | 0.0012 | 3.51 | 0.38 | 2.98 | 0.65 | 1.18 |
| 830.5894 | 1202 | 0.0012 | 4.90 | 0.49 | 4.36 | 0.55 | 1.12 |
| 854.5886 | 1102 | 0.0012 | 2.02 | 0.46 | 1.36 | 0.80 | 1.48 |
| 567.3548 | 1102 | 0.0013 | 3.40 | 0.41 | 2.81 | 0.73 | 1.21 |
| 853.5853 | 1102 | 0.0013 | 2.99 | 0.48 | 2.41 | 0.67 | 1.24 |
| 593.4734 | 1202 | 0.0014 | 0.50 | 0.65 | 0.00 | 0.00 | NA |
| 723.5193 | 1204 | 0.0014 | 4.46 | 0.77 | 3.33 | 1.42 | 1.34 |
| 1017.9341 | 1203 | 0.0014 | 4.56 | 0.65 | 3.46 | 1.43 | 1.32 |
| 649.5898 | 1203 | 0.0015 | 4.69 | 0.48 | 3.99 | 0.88 | 1.18 |
| 560.4799 | 1203 | 0.0016 | 2.71 | 0.37 | 2.14 | 0.73 | 1.26 |
| 751.5529 | 1202 | 0.0016 | 3.98 | 0.52 | 3.23 | 0.95 | 1.23 |
| 481.3171 | 1102 | 0.0017 | 1.78 | 0.36 | 1.28 | 0.63 | 1.39 |
| 556.4504 | 1204 | 0.0017 | 2.83 | 0.42 | 2.35 | 0.54 | 1.20 |
| 646.5709 | 1203 | 0.0017 | 3.54 | 0.60 | 2.80 | 0.87 | 1.26 |
| 749.5402 | 1204 | 0.0017 | 4.98 | 0.64 | 3.92 | 1.41 | 1.27 |
| 794.5128 | 1204 | 0.0017 | 2.48 | 0.32 | 1.77 | 1.00 | 1.40 |
| 821.5717 | 1102 | 0.0017 | 3.01 | 0.44 | 2.49 | 0.60 | 1.21 |
| 829.5859 | 1202 | 0.0017 | 6.00 | 0.50 | 5.48 | 0.54 | 1.09 |
| 840.6067 | 1202 | 0.0017 | 2.94 | 0.33 | 2.61 | 0.31 | 1.12 |
| 496.4165 | 1204 | 0.0018 | 2.10 | 0.90 | 1.21 | 0.88 | 1.74 |
| 729.5726 | 1204 | 0.0018 | 2.36 | 0.38 | 1.74 | 0.84 | 1.36 |
| 807.5762 | 1101 | 0.0018 | 4.21 | 0.41 | 3.68 | 0.66 | 1.15 |
| 819.5553 | 1102 | 0.0018 | 2.19 | 0.64 | 1.45 | 0.84 | 1.51 |
| 626.5286 | 1203 | 0.0019 | 3.78 | 0.36 | 3.43 | 0.35 | 1.10 |
| 857.6171 | 1102 | 0.0019 | 2.51 | 0.80 | 1.57 | 1.11 | 1.60 |
| 808.5794 | 1101 | 0.002 | 3.22 | 0.40 | 2.69 | 0.68 | 1.20 |
| 852.7196 | 1203 | 0.002 | 5.94 | 0.62 | 5.28 | 0.72 | 1.13 |
| 505.3227 | 1202 | 0.0021 | 4.06 | 0.30 | 3.72 | 0.38 | 1.09 |
| 566.3433 | 1202 | 0.0021 | 5.29 | 0.31 | 4.95 | 0.37 | 1.07 |
| 592.3570 | 1202 | 0.0021 | 2.46 | 0.44 | 1.99 | 0.53 | 1.24 |
| 541.3422 | 1102 | 0.0023 | 4.44 | 0.36 | 3.85 | 0.83 | 1.15 |
| 542.3452 | 1102 | 0.0023 | 2.64 | 0.35 | 2.07 | 0.79 | 1.28 |
| 779.5438 | 1101 | 0.0023 | 5.08 | 0.46 | 4.51 | 0.74 | 1.13 |
| 785.5936 | 1101 | 0.0023 | 4.21 | 0.41 | 3.74 | 0.56 | 1.13 |
| 786.5403 | 1204 | 0.0023 | 4.16 | 0.34 | 3.78 | 0.44 | 1.10 |
| 758.5654 | 1101 | 0.0024 | 4.35 | 0.44 | 3.83 | 0.63 | 1.14 |
| 1018.9433 | 1203 | 0.0024 | 4.22 | 0.70 | 2.91 | 1.88 | 1.45 |
| 495.3328 | 1101 | 0.0025 | 4.19 | 0.37 | 3.51 | 0.98 | 1.20 |
| 735.6555 | 1204 | 0.0025 | 4.05 | 0.42 | 3.45 | 0.80 | 1.17 |
| 752.5564 | 1202 | 0.0025 | 2.90 | 0.51 | 2.17 | 0.97 | 1.33 |
| 382.1091 | 1101 | 0.0026 | 0.22 | 0.55 | 0.85 | 0.79 | 0.25 |
| 569.3687 | 1102 | 0.0027 | 3.11 | 0.41 | 2.48 | 0.89 | 1.26 |
| 757.5618 | 1101 | 0.0027 | 5.38 | 0.44 | 4.87 | 0.64 | 1.11 |
| 837.5885 | 1202 | 0.0027 | 2.70 | 0.38 | 2.33 | 0.40 | 1.16 |
| 879.7420 | 1203 | 0.0027 | 5.51 | 0.59 | 4.89 | 0.70 | 1.13 |
| 300.2099 | 1204 | 0.0028 | 1.80 | 0.33 | 1.27 | 0.75 | 1.42 |
| 794.5423 | 1102 | 0.0029 | 2.56 | 0.33 | 2.05 | 0.72 | 1.25 |
| 806.5644 | 1101 | 0.0029 | 3.00 | 0.47 | 2.47 | 0.65 | 1.21 |
| 877.7269 | 1203 | 0.0029 | 3.56 | 0.64 | 2.79 | 0.99 | 1.28 |
| 522.4640 | 1203 | 0.0031 | 4.68 | 0.96 | 3.73 | 1.07 | 1.25 |
| 589.3401 | 1102 | 0.0031 | 2.72 | 0.42 | 2.18 | 0.72 | 1.25 |
| 320.2358 | 1204 | 0.0032 | 1.83 | 0.55 | 1.22 | 0.76 | 1.50 |
| 339.9964 | 1101 | 0.0032 | 1.92 | 0.94 | 2.87 | 1.11 | 0.67 |
| 559.4699 | 1202 | 0.0032 | 1.18 | 0.82 | 0.47 | 0.67 | 2.49 |
| 878.7381 | 1203 | 0.0032 | 6.24 | 0.60 | 5.65 | 0.68 | 1.11 |
| 749.5354 | 1201 | 0.0033 | 2.10 | 0.62 | 1.38 | 0.94 | 1.53 |
| 783.5139 | 1204 | 0.0033 | 3.72 | 0.31 | 3.33 | 0.52 | 1.12 |
| 243.0719 | 1101 | 0.0034 | 4.50 | 0.79 | 5.24 | 0.81 | 0.86 |
| 803.5437 | 1101 | 0.0035 | 3.78 | 0.45 | 3.17 | 0.84 | 1.19 |
| 812.5768 | 1202 | 0.0035 | 2.23 | 0.47 | 1.69 | 0.69 | 1.32 |
| 1019.9501 | 1203 | 0.0035 | 3.37 | 0.70 | 2.31 | 1.54 | 1.46 |
| 829.5596 | 1101 | 0.0036 | 2.09 | 0.47 | 1.49 | 0.83 | 1.40 |
| 831.5997 | 1102 | 0.0036 | 5.11 | 0.51 | 4.55 | 0.70 | 1.12 |
| 523.4677 | 1203 | 0.0037 | 3.27 | 0.93 | 2.29 | 1.22 | 1.43 |
| 780.5473 | 1101 | 0.0038 | 3.99 | 0.47 | 3.44 | 0.73 | 1.16 |
| 853.7250 | 1203 | 0.0038 | 5.25 | 0.62 | 4.65 | 0.70 | 1.13 |
| 899.5874 | 1102 | 0.0038 | 2.92 | 0.51 | 2.38 | 0.67 | 1.23 |
| 205.8867 | 1101 | 0.0041 | 2.79 | 0.28 | 3.04 | 0.28 | 0.92 |
| 519.3320 | 1201 | 0.0041 | 2.64 | 0.73 | 1.97 | 0.73 | 1.34 |
| 825.5544 | 1202 | 0.0041 | 3.04 | 0.86 | 2.26 | 0.85 | 1.34 |
| 562.5001 | 1204 | 0.0042 | 2.82 | 0.51 | 2.23 | 0.79 | 1.26 |
| 194.0804 | 1203 | 0.0044 | 0.72 | 0.80 | 0.13 | 0.39 | 5.63 |
| 273.8740 | 1101 | 0.0044 | 2.73 | 0.29 | 3.01 | 0.33 | 0.91 |
| 752.5579 | 1204 | 0.0044 | 4.10 | 0.67 | 3.19 | 1.32 | 1.29 |
| 570.3726 | 1202 | 0.0046 | 3.16 | 0.23 | 2.94 | 0.27 | 1.08 |
| 783.5783 | 1201 | 0.0046 | 6.25 | 0.37 | 5.89 | 0.42 | 1.06 |
| 283.9028 | 1101 | 0.0047 | 3.11 | 0.33 | 3.39 | 0.30 | 0.92 |
| 552.4048 | 1204 | 0.0047 | 0.73 | 0.70 | 0.19 | 0.47 | 3.91 |
| 763.5158 | 1202 | 0.0048 | 1.79 | 0.77 | 2.51 | 0.85 | 0.71 |
| 781.5612 | 1101 | 0.0049 | 4.88 | 0.41 | 4.41 | 0.65 | 1.11 |
| 779.5831 | 1204 | 0.005 | 2.60 | 0.50 | 1.94 | 0.96 | 1.34 |
| 817.5377 | 1102 | 0.0052 | 2.40 | 0.39 | 1.92 | 0.70 | 1.25 |
| 259.9415 | 1101 | 0.0053 | 2.95 | 0.47 | 2.30 | 0.97 | 1.28 |
| 612.5005 | 1204 | 0.0053 | 1.82 | 0.69 | 1.13 | 0.90 | 1.62 |
| 763.5144 | 1201 | 0.0053 | 1.44 | 0.66 | 2.13 | 0.92 | 0.67 |
| 770.5701 | 1204 | 0.0053 | 2.92 | 0.39 | 2.34 | 0.89 | 1.25 |
| 863.6872 | 1204 | 0.0053 | 5.33 | 0.40 | 4.90 | 0.58 | 1.09 |
| 509.3493 | 1202 | 0.0054 | 2.58 | 0.26 | 2.31 | 0.35 | 1.11 |
| 782.5087 | 1204 | 0.0055 | 4.09 | 0.36 | 3.73 | 0.48 | 1.10 |
| 552.4788 | 1204 | 0.0056 | 1.76 | 0.85 | 1.00 | 0.91 | 1.77 |
| 832.6027 | 1102 | 0.0057 | 3.97 | 0.51 | 3.44 | 0.71 | 1.15 |
| 782.5649 | 1101 | 0.0058 | 3.80 | 0.42 | 3.33 | 0.67 | 1.14 |
| 822.5750 | 1102 | 0.0058 | 2.00 | 0.44 | 1.55 | 0.60 | 1.29 |
| 828.5734 | 1102 | 0.0058 | 3.71 | 0.37 | 3.19 | 0.78 | 1.16 |
| 923.5882 | 1102 | 0.0058 | 1.94 | 0.42 | 1.44 | 0.73 | 1.35 |
| 793.5386 | 1102 | 0.0059 | 3.63 | 0.39 | 3.20 | 0.61 | 1.14 |
| 501.3214 | 1201 | 0.0061 | 2.49 | 0.43 | 2.13 | 0.39 | 1.17 |
| 777.5679 | 1204 | 0.0062 | 2.94 | 0.51 | 2.28 | 0.99 | 1.29 |
| 368.1653 | 1102 | 0.0064 | 0.97 | 1.17 | 0.16 | 0.50 | 6.00 |
| 809.5938 | 1101 | 0.0064 | 3.48 | 0.37 | 3.08 | 0.55 | 1.13 |
| 751.5548 | 1204 | 0.0065 | 5.22 | 0.72 | 4.38 | 1.25 | 1.19 |
| 804.5470 | 1101 | 0.0065 | 2.79 | 0.43 | 2.30 | 0.71 | 1.21 |
| 569.3691 | 1202 | 0.0066 | 5.05 | 0.23 | 4.82 | 0.30 | 1.05 |
| 568.3574 | 1102 | 0.0068 | 1.52 | 0.48 | 1.07 | 0.58 | 1.42 |
| 827.5698 | 1102 | 0.0068 | 4.74 | 0.39 | 4.21 | 0.82 | 1.13 |
| 786.5967 | 1101 | 0.007 | 3.12 | 0.38 | 2.73 | 0.54 | 1.14 |
| 753.5669 | 1204 | 0.0073 | 2.92 | 0.55 | 2.24 | 1.06 | 1.31 |
| 759.5159 | 1204 | 0.0073 | 5.19 | 0.34 | 4.84 | 0.49 | 1.07 |
| 855.6012 | 1102 | 0.0074 | 4.13 | 0.41 | 3.63 | 0.76 | 1.14 |
| 858.7902 | 1101 | 0.0074 | 0.06 | 0.20 | 0.32 | 0.41 | 0.18 |
| 756.4904 | 1204 | 0.0075 | 2.65 | 0.35 | 2.20 | 0.72 | 1.21 |
| 580.5345 | 1203 | 0.0077 | 2.21 | 0.71 | 1.51 | 0.97 | 1.46 |
| 784.5808 | 1201 | 0.0077 | 5.30 | 0.38 | 4.96 | 0.45 | 1.07 |
| 853.5864 | 1202 | 0.0078 | 4.92 | 0.53 | 4.44 | 0.63 | 1.11 |
| 560.4828 | 1204 | 0.0079 | 3.80 | 0.52 | 3.21 | 0.88 | 1.18 |
| 573.4855 | 1203 | 0.0079 | 4.39 | 0.35 | 4.06 | 0.46 | 1.08 |
| 587.3229 | 1202 | 0.0079 | 2.10 | 0.91 | 1.41 | 0.72 | 1.50 |
| 560.4816 | 1202 | 0.0081 | 2.02 | 0.55 | 1.38 | 0.96 | 1.46 |
| 952.7568 | 1203 | 0.0081 | 0.91 | 1.05 | 0.20 | 0.50 | 4.46 |
| 801.5551 | 1202 | 0.0082 | 2.59 | 0.56 | 2.11 | 0.59 | 1.23 |
| 741.5306 | 1204 | 0.0083 | 2.93 | 0.52 | 2.47 | 0.59 | 1.18 |
| 773.5339 | 1204 | 0.0083 | 3.58 | 0.28 | 3.07 | 0.87 | 1.17 |
| 854.5903 | 1202 | 0.0084 | 3.98 | 0.54 | 3.50 | 0.63 | 1.14 |
| 847.5955 | 1202 | 0.0085 | 2.55 | 0.48 | 2.13 | 0.54 | 1.20 |
| 736.6583 | 1204 | 0.0087 | 2.92 | 0.45 | 2.45 | 0.69 | 1.19 |
| 529.3167 | 1202 | 0.0088 | 3.21 | 0.32 | 2.88 | 0.48 | 1.11 |
| 810.5401 | 1204 | 0.0091 | 3.49 | 0.34 | 3.17 | 0.45 | 1.10 |
| 628.5425 | 1203 | 0.0092 | 3.22 | 0.45 | 2.86 | 0.40 | 1.12 |
| 518.4345 | 1203 | 0.0093 | 1.33 | 1.08 | 0.48 | 1.00 | 2.79 |
| 769.5644 | 1204 | 0.0093 | 4.01 | 0.39 | 3.62 | 0.57 | 1.11 |
| 990.8090 | 1204 | 0.0094 | 0.00 | 0.00 | 0.68 | 1.25 | 0.00 |
| 269.9704 | 1101 | 0.0095 | 3.86 | 0.62 | 3.27 | 0.85 | 1.18 |
| 804.7219 | 1203 | 0.0095 | 2.47 | 1.05 | 1.54 | 1.23 | 1.60 |
| 216.0401 | 1102 | 0.0097 | 3.01 | 0.84 | 3.64 | 0.69 | 0.83 |
| 300.2084 | 1202 | 0.0097 | 0.27 | 0.65 | 0.98 | 1.07 | 0.28 |
| 411.3186 | 1202 | 0.0097 | 2.88 | 0.29 | 2.49 | 0.64 | 1.16 |
| 746.5561 | 1102 | 0.0097 | 2.01 | 0.30 | 1.63 | 0.62 | 1.23 |
| 632.5753 | 1203 | 0.0098 | 1.46 | 0.85 | 0.77 | 0.85 | 1.90 |
| 895.5578 | 1102 | 0.0099 | 2.60 | 0.38 | 2.19 | 0.64 | 1.19 |
| 688.5294 | 1204 | 0.01 | 2.88 | 0.42 | 2.11 | 1.34 | 1.36 |
| 382.2902 | 1204 | 0.0101 | 0.04 | 0.18 | 0.38 | 0.61 | 0.09 |
| 758.5088 | 1204 | 0.0102 | 4.91 | 0.36 | 4.59 | 0.45 | 1.07 |
| 776.6068 | 1202 | 0.0102 | 1.71 | 0.63 | 2.16 | 0.44 | 0.79 |
| 609.3242 | 1102 | 0.0103 | 2.03 | 0.35 | 1.64 | 0.61 | 1.24 |
| 392.2940 | 1204 | 0.0107 | 1.78 | 0.95 | 0.85 | 1.40 | 2.10 |
| 747.5204 | 1202 | 0.0108 | 2.53 | 0.55 | 1.95 | 0.90 | 1.30 |
| 218.0372 | 1102 | 0.0113 | 1.34 | 0.77 | 1.96 | 0.79 | 0.68 |
| 811.5733 | 1202 | 0.0113 | 3.14 | 0.52 | 2.74 | 0.46 | 1.14 |
| 826.5577 | 1202 | 0.0113 | 2.01 | 0.88 | 1.36 | 0.74 | 1.48 |
| 265.8423 | 1101 | 0.0115 | 2.57 | 0.64 | 2.98 | 0.32 | 0.86 |
| 675.6374 | 1204 | 0.0115 | 3.87 | 0.48 | 3.45 | 0.59 | 1.12 |
| 570.4914 | 1204 | 0.0116 | 0.66 | 0.79 | 0.15 | 0.38 | 4.35 |
| 202.0454 | 1101 | 0.0118 | 2.55 | 1.09 | 3.38 | 1.00 | 0.76 |
| 856.6046 | 1102 | 0.0119 | 3.13 | 0.41 | 2.64 | 0.82 | 1.19 |
| 276.2096 | 1204 | 0.012 | 2.74 | 0.46 | 2.34 | 0.56 | 1.17 |
| 328.2629 | 1204 | 0.0121 | 1.73 | 0.25 | 1.94 | 0.30 | 0.89 |
| 702.5675 | 1101 | 0.0121 | 2.84 | 0.29 | 2.48 | 0.61 | 1.15 |
| 803.5684 | 1102 | 0.0122 | 5.99 | 0.46 | 5.54 | 0.70 | 1.08 |
| 804.5716 | 1102 | 0.0122 | 4.70 | 0.43 | 4.27 | 0.67 | 1.10 |
| 624.5134 | 1203 | 0.0127 | 4.04 | 0.39 | 3.72 | 0.44 | 1.09 |
| 721.6387 | 1204 | 0.0129 | 5.24 | 0.49 | 4.79 | 0.67 | 1.09 |
| 247.9576 | 1202 | 0.0132 | 0.00 | 0.00 | 0.94 | 1.82 | 0.00 |
| 440.3898 | 1204 | 0.0138 | 0.31 | 0.55 | 0.00 | 0.00 | NA |
| 926.7366 | 1203 | 0.014 | 2.14 | 0.97 | 1.38 | 0.99 | 1.55 |
| 839.6034 | 1202 | 0.0141 | 3.87 | 0.36 | 3.60 | 0.34 | 1.07 |
| 764.5187 | 1204 | 0.0143 | 1.87 | 1.08 | 2.65 | 0.94 | 0.71 |
| 722.6422 | 1204 | 0.0149 | 4.15 | 0.51 | 3.70 | 0.68 | 1.12 |
| 900.5895 | 1102 | 0.0149 | 1.93 | 0.46 | 1.49 | 0.70 | 1.29 |
| 590.3429 | 1202 | 0.015 | 4.26 | 0.37 | 3.95 | 0.43 | 1.08 |
| 724.5498 | 1101 | 0.0151 | 2.42 | 0.29 | 2.01 | 0.73 | 1.20 |
| 769.4958 | 1204 | 0.0151 | 2.99 | 0.39 | 2.47 | 0.92 | 1.21 |
| 857.6185 | 1202 | 0.0155 | 4.05 | 0.58 | 3.57 | 0.69 | 1.13 |
| 777.5299 | 1201 | 0.0156 | 2.02 | 0.62 | 1.61 | 0.44 | 1.26 |
| 333.8296 | 1101 | 0.0158 | 2.74 | 0.30 | 2.99 | 0.38 | 0.92 |
| 755.5476 | 1201 | 0.0158 | 2.81 | 0.46 | 2.47 | 0.42 | 1.14 |
| 313.9966 | 1101 | 0.016 | 1.41 | 1.13 | 0.58 | 1.07 | 2.43 |
| 599.5004 | 1203 | 0.016 | 5.06 | 0.52 | 4.62 | 0.65 | 1.09 |
| 810.5970 | 1101 | 0.0162 | 2.51 | 0.42 | 2.14 | 0.55 | 1.17 |
| 801.5297 | 1201 | 0.0166 | 2.58 | 0.97 | 1.96 | 0.59 | 1.31 |
| 830.5650 | 1201 | 0.0166 | 3.31 | 0.46 | 2.99 | 0.41 | 1.11 |
| 629.5452 | 1203 | 0.0169 | 1.95 | 0.66 | 1.41 | 0.77 | 1.38 |
| 716.4981 | 1204 | 0.0169 | 2.35 | 0.34 | 1.82 | 1.00 | 1.29 |
| 858.6210 | 1202 | 0.0175 | 2.95 | 0.61 | 2.42 | 0.86 | 1.22 |
| 524.4725 | 1203 | 0.0177 | 1.08 | 0.92 | 0.47 | 0.70 | 2.31 |
| 534.4558 | 1203 | 0.0177 | 2.57 | 1.08 | 1.70 | 1.28 | 1.51 |
| 861.5265 | 1102 | 0.0177 | 2.36 | 0.43 | 1.97 | 0.65 | 1.20 |
| 670.5708 | 1203 | 0.0178 | 1.69 | 0.89 | 1.02 | 0.91 | 1.65 |
| 748.5280 | 1204 | 0.018 | 2.78 | 0.53 | 2.31 | 0.76 | 1.21 |
| 520.4502 | 1203 | 0.0181 | 3.69 | 0.97 | 2.97 | 0.99 | 1.24 |
| 686.5125 | 1204 | 0.0184 | 2.47 | 0.85 | 1.67 | 1.33 | 1.48 |
| 690.5471 | 1204 | 0.0185 | 2.33 | 0.38 | 1.79 | 1.01 | 1.30 |
| 625.5163 | 1203 | 0.0187 | 2.86 | 0.40 | 2.47 | 0.68 | 1.16 |
| 859.6889 | 1202 | 0.019 | 1.98 | 0.46 | 2.31 | 0.47 | 0.85 |
| 1251.1152 | 1203 | 0.0191 | 1.62 | 1.24 | 0.78 | 1.02 | 2.07 |
| 763.5150 | 1204 | 0.0196 | 3.00 | 0.92 | 3.67 | 0.95 | 0.82 |
| 269.8081 | 1102 | 0.0199 | 2.29 | 0.36 | 2.53 | 0.27 | 0.91 |
| 829.5620 | 1201 | 0.02 | 4.27 | 0.47 | 3.96 | 0.39 | 1.08 |
| 745.4973 | 1204 | 0.0201 | 3.51 | 0.29 | 3.25 | 0.44 | 1.08 |
| 541.3138 | 1201 | 0.0204 | 2.13 | 0.93 | 1.53 | 0.69 | 1.39 |
| 1019.3837 | 1102 | 0.0205 | 2.30 | 0.23 | 2.46 | 0.19 | 0.94 |
| 627.5306 | 1203 | 0.0209 | 2.52 | 0.41 | 2.16 | 0.61 | 1.17 |
| 354.1668 | 1202 | 0.0216 | 0.00 | 0.00 | 0.41 | 0.86 | 0.00 |
| 695.6469 | 1204 | 0.0219 | 2.52 | 1.08 | 1.65 | 1.38 | 1.53 |
| 707.6257 | 1204 | 0.0224 | 4.24 | 0.43 | 3.89 | 0.58 | 1.09 |
| 641.4915 | 1204 | 0.0226 | 2.16 | 1.02 | 1.42 | 1.09 | 1.53 |
| 772.5269 | 1204 | 0.0229 | 3.69 | 0.35 | 3.38 | 0.52 | 1.09 |
| 444.3598 | 1203 | 0.0242 | 2.08 | 0.43 | 1.60 | 0.90 | 1.30 |
| 720.2576 | 1204 | 0.0253 | 0.00 | 0.00 | 0.40 | 0.86 | 0.00 |
| 709.2595 | 1202 | 0.0254 | 2.70 | 0.43 | 2.38 | 0.49 | 1.13 |
| 738.5448 | 1102 | 0.0258 | 2.74 | 0.35 | 2.43 | 0.56 | 1.13 |
| 761.5839 | 1201 | 0.0262 | 2.97 | 0.43 | 3.25 | 0.37 | 0.91 |
| 831.5750 | 1101 | 0.0265 | 2.84 | 0.49 | 2.48 | 0.58 | 1.15 |
| 672.5865 | 1203 | 0.0268 | 4.47 | 0.61 | 3.94 | 0.93 | 1.13 |
| 895.5590 | 1202 | 0.0268 | 2.22 | 0.41 | 1.87 | 0.64 | 1.19 |
| 247.9579 | 1102 | 0.0271 | 0.00 | 0.00 | 0.48 | 1.04 | 0.00 |
| 589.3404 | 1202 | 0.0272 | 6.13 | 0.37 | 5.84 | 0.49 | 1.05 |
| 572.4818 | 1203 | 0.0273 | 5.79 | 0.38 | 5.50 | 0.45 | 1.05 |
| 673.5892 | 1203 | 0.0277 | 3.66 | 0.57 | 3.08 | 1.10 | 1.19 |
| 880.7526 | 1203 | 0.0278 | 7.31 | 0.66 | 6.87 | 0.61 | 1.06 |
| 772.5857 | 1204 | 0.0279 | 3.31 | 0.31 | 3.04 | 0.48 | 1.09 |
| 881.7568 | 1203 | 0.0279 | 6.55 | 0.65 | 6.13 | 0.60 | 1.07 |
| 747.5233 | 1204 | 0.0284 | 3.88 | 0.52 | 3.37 | 0.96 | 1.15 |
| 215.9155 | 1101 | 0.0285 | 4.99 | 0.42 | 5.24 | 0.30 | 0.95 |
| 521.4524 | 1203 | 0.0285 | 1.97 | 1.04 | 1.28 | 1.01 | 1.55 |
| 341.8614 | 1101 | 0.0287 | 3.31 | 0.39 | 3.59 | 0.42 | 0.92 |
| 768.4945 | 1204 | 0.0299 | 3.79 | 0.41 | 3.47 | 0.54 | 1.09 |
| 598.4961 | 1203 | 0.0307 | 6.34 | 0.56 | 5.94 | 0.65 | 1.07 |
| 430.3083 | 1204 | 0.0312 | 2.07 | 0.28 | 1.88 | 0.27 | 1.10 |
| 494.4343 | 1203 | 0.0313 | 1.92 | 1.56 | 0.94 | 1.35 | 2.04 |
| 912.8233 | 1102 | 0.0314 | 0.05 | 0.19 | 0.26 | 0.41 | 0.21 |
| 343.8589 | 1101 | 0.0319 | 2.37 | 0.57 | 2.68 | 0.33 | 0.88 |
| 416.3670 | 1204 | 0.0319 | 0.81 | 0.95 | 0.26 | 0.64 | 3.16 |
| 802.5328 | 1201 | 0.0325 | 1.64 | 0.87 | 1.16 | 0.49 | 1.42 |
| 278.2256 | 1204 | 0.0333 | 4.92 | 0.42 | 4.61 | 0.54 | 1.07 |
| 775.5534 | 1202 | 0.0334 | 2.47 | 0.44 | 2.05 | 0.80 | 1.20 |
| 767.5455 | 1201 | 0.0335 | 2.36 | 0.42 | 2.67 | 0.52 | 0.88 |
| 217.9125 | 1101 | 0.034 | 3.60 | 0.38 | 3.82 | 0.31 | 0.94 |
| 838.7228 | 1204 | 0.0341 | 2.61 | 1.02 | 1.91 | 1.12 | 1.37 |
| 363.3499 | 1201 | 0.0344 | 0.06 | 0.32 | 0.55 | 1.05 | 0.12 |
| 263.8452 | 1101 | 0.0349 | 2.74 | 0.30 | 2.95 | 0.36 | 0.93 |
| 371.3538 | 1203 | 0.0353 | 3.05 | 0.27 | 2.81 | 0.45 | 1.08 |
| 828.7205 | 1203 | 0.0354 | 5.58 | 0.56 | 5.21 | 0.60 | 1.07 |
| 872.5557 | 1102 | 0.0357 | 2.39 | 0.44 | 2.02 | 0.71 | 1.19 |
| 871.5528 | 1102 | 0.0361 | 3.46 | 0.46 | 3.09 | 0.68 | 1.12 |
| 872.7844 | 1102 | 0.0373 | 0.17 | 0.35 | 0.00 | 0.00 | NA |
| 922.8228 | 1204 | 0.0373 | 2.11 | 1.56 | 1.11 | 1.57 | 1.91 |
| 796.5293 | 1204 | 0.0375 | 3.33 | 0.34 | 3.07 | 0.48 | 1.09 |
| 871.5940 | 1202 | 0.0381 | 2.12 | 0.44 | 1.80 | 0.55 | 1.18 |
| 767.5821 | 1201 | 0.0382 | 3.42 | 0.58 | 3.07 | 0.47 | 1.11 |
| 950.7386 | 1203 | 0.0383 | 0.54 | 0.93 | 0.07 | 0.31 | 7.77 |
| 561.4871 | 1204 | 0.0385 | 2.52 | 0.59 | 2.06 | 0.86 | 1.22 |
| 588.3282 | 1202 | 0.0388 | 0.74 | 0.80 | 0.31 | 0.45 | 2.36 |
| 174.1408 | 1203 | 0.0392 | 1.85 | 0.25 | 1.57 | 0.59 | 1.18 |
| 760.5816 | 1101 | 0.0393 | 3.01 | 0.45 | 2.71 | 0.48 | 1.11 |
| 825.5547 | 1102 | 0.0402 | 1.05 | 0.77 | 0.63 | 0.51 | 1.67 |
| 837.7180 | 1204 | 0.0408 | 3.29 | 0.96 | 2.62 | 1.17 | 1.26 |
| 492.4185 | 1203 | 0.0413 | 0.69 | 0.94 | 0.19 | 0.57 | 3.72 |
| 671.5722 | 1204 | 0.0415 | 2.89 | 0.40 | 2.42 | 1.02 | 1.19 |
| 541.3433 | 1202 | 0.0417 | 5.99 | 0.34 | 5.80 | 0.26 | 1.03 |
| 760.5223 | 1204 | 0.0418 | 4.54 | 0.30 | 4.32 | 0.43 | 1.05 |
| 452.2536 | 1204 | 0.0421 | 1.68 | 0.34 | 1.32 | 0.77 | 1.27 |
| 663.5212 | 1204 | 0.0422 | 2.69 | 0.76 | 2.09 | 1.15 | 1.29 |
| 744.4942 | 1204 | 0.0422 | 4.33 | 0.37 | 4.06 | 0.47 | 1.06 |
| 302.2256 | 1204 | 0.0424 | 3.66 | 0.40 | 3.37 | 0.54 | 1.09 |
| 751.5514 | 1203 | 0.043 | 1.39 | 1.00 | 0.76 | 1.02 | 1.84 |
| 775.5531 | 1204 | 0.043 | 3.60 | 0.52 | 3.10 | 1.05 | 1.16 |
| 798.6773 | 1203 | 0.043 | 1.05 | 1.09 | 0.40 | 0.95 | 2.60 |
| 432.3256 | 1204 | 0.0434 | 1.87 | 0.46 | 1.51 | 0.69 | 1.24 |
| 633.3235 | 1202 | 0.0439 | 1.69 | 0.62 | 1.28 | 0.70 | 1.32 |
| 808.5798 | 1201 | 0.044 | 5.31 | 0.32 | 5.12 | 0.27 | 1.04 |
| 615.3540 | 1202 | 0.0443 | 2.52 | 0.41 | 2.25 | 0.49 | 1.12 |
| 857.8044 | 1101 | 0.0444 | 0.12 | 0.29 | 0.36 | 0.47 | 0.34 |
| 858.7341 | 1202 | 0.0449 | 0.16 | 0.38 | 0.67 | 1.17 | 0.24 |
| 804.7208 | 1204 | 0.0452 | 1.64 | 1.06 | 1.01 | 0.97 | 1.63 |
| 874.5514 | 1201 | 0.0453 | 1.32 | 0.75 | 0.85 | 0.78 | 1.56 |
| 300.2676 | 1204 | 0.0462 | 1.24 | 0.63 | 0.84 | 0.66 | 1.47 |
| 756.5512 | 1201 | 0.0465 | 1.64 | 0.55 | 1.29 | 0.60 | 1.27 |
| 369.3474 | 1203 | 0.0466 | 9.26 | 0.25 | 9.07 | 0.39 | 1.02 |
| 305.2439 | 1204 | 0.0472 | 2.75 | 0.32 | 2.48 | 0.53 | 1.11 |
| 660.5006 | 1204 | 0.0473 | 1.36 | 0.96 | 0.76 | 0.98 | 1.78 |
| 748.5721 | 1102 | 0.0489 | 4.55 | 0.34 | 4.24 | 0.67 | 1.07 |
| 309.3035 | 1201 | 0.049 | 0.00 | 0.00 | 0.28 | 0.70 | 0.00 |
| 910.7247 | 1204 | 0.0491 | 3.75 | 0.73 | 3.22 | 1.02 | 1.16 |
| 252.2096 | 1204 | 0.0496 | 1.81 | 0.33 | 1.57 | 0.47 | 1.15 |
| 829.7242 | 1203 | 0.0496 | 4.83 | 0.55 | 4.49 | 0.57 | 1.08 |
| 255.0896 | 1203 | 0.0497 | 0.00 | 0.00 | 0.21 | 0.53 | 0.00 |
| 807.5768 | 1201 | 0.0498 | 6.22 | 0.32 | 6.05 | 0.26 | 1.03 |

**Table 2: List of 37 metabolite subset selected based upon p<0.0001, ¹³C exclusion and inclusion of only mode 1204 molecules.**

| | | | | **Ovarian** | | **Controls** | |
|---|---|---|---|---|---|---|---|
| | **Detected Mass** | **Analysis Mode** | **P_Value** | **AVG** | **SD** | **AVG** | **SD** |
| 1 | 440.3532 | 1204 | 7.56E-06 | 2.03 | 1.15 | 5.22 | 1.77 |
| 2 | 446.3413 | 1204 | 0.0001 | 2.48 | 1.57 | 6.02 | 2.00 |
| 3 | 448.3565 | 1204 | 1.44E-06 | 2.28 | 1.36 | 5.10 | 1.52 |
| 4 | 450.3735 | 1204 | 8.06E-08 | 1.94 | 1.11 | 4.64 | 1.67 |
| 5 | 464.3531 | 1204 | 8.16E-07 | 2.36 | 1.43 | 5.98 | 2.29 |
| 6 | 466.3659 | 1204 | 3.89E-07 | 2.45 | 1.22 | 5.29 | 1.74 |
| 7 | 468.3848 | 1204 | 2.42E-05 | 2.41 | 1.35 | 5.42 | 1.85 |
| 8 | 474.3736 | 1204 | 1.59E-05 | 1.54 | 0.89 | 3.76 | 1.47 |
| 9 | 478.405 | 1204 | 1.91E-05 | 2.52 | 1.25 | 6.16 | 2.56 |
| 10 | 484.3793 | 1204 | 1.12E-07 | 2.72 | 1.65 | 7.04 | 3.00 |
| 11 | 490.3678 | 1204 | 1.37E-07 | 1.58 | 0.89 | 3.64 | 1.40 |
| 12 | 492.3841 | 1204 | 2.80E-08 | 1.82 | 0.97 | 4.00 | 1.50 |
| 13 | 494.3973 | 1204 | 4.55E-07 | 1.45 | 0.72 | 3.52 | 1.52 |
| 14 | 502.4055 | 1204 | 9.88E-08 | 3.34 | 1.70 | 7.21 | 2.71 |
| 15 | 504.4195 | 1204 | 2.43E-06 | 4.56 | 2.57 | 9.74 | 3.48 |
| 16 | 510.3943 | 1204 | 1.50E-05 | 1.53 | 0.70 | 2.92 | 0.93 |
| 17 | 512.4083 | 1204 | 1.75E-05 | 2.68 | 1.59 | 6.36 | 2.61 |
| 18 | 518.3974 | 1204 | 2.02E-06 | 3.73 | 1.77 | 7.93 | 3.00 |
| 19 | 520.4131 | 1204 | 8.77E-06 | 4.43 | 2.09 | 9.42 | 3.64 |
| 20 | 522.4323 | 1204 | 1.88E-05 | 1.04 | 0.20 | 2.19 | 0.93 |
| 21 | 530.437 | 1204 | 1.38E-05 | 5.17 | 3.03 | 12.38 | 5.45 |
| 22 | 532.4507 | 1204 | 4.65E-06 | 7.60 | 3.69 | 18.25 | 8.62 |
| 23 | 534.3913 | 1204 | 2.58E-06 | 1.11 | 0.36 | 2.31 | 1.00 |
| 24 | 538.427 | 1204 | 6.41 E-06 | 1.32 | 0.68 | 3.16 | 1.48 |
| 25 | 540.4393 | 1204 | 4.81 E-05 | 1.65 | 0.98 | 3.53 | 1.39 |
| 26 | 548.4442 | 1204 | 2.35E-07 | 2.21 | 1.32 | 6.21 | 3.37 |
| 27 | 550.4609 | 1204 | 3.37E-05 | 1.05 | 0.24 | 2.11 | 0.92 |
| 28 | 558.4653 | 1204 | 2.75E-05 | 1.23 | 0.49 | 2.42 | 1.01 |
| 29 | 566.4554 | 1204 | 7.38E-06 | 5.57 | 2.97 | 14.93 | 8.32 |
| 30 | 574.4597 | 1204 | 1.60E-06 | 5.38 | 3.71 | 16.16 | 9.51 |
| 31 | 576.4762 | 1204 | 7.44E-07 | 1.61 | 0.83 | 3.17 | 1.27 |
| 32 | 578.493 | 1204 | 1.66E-05 | 5.09 | 3.96 | 14.56 | 8.24 |
| 33 | 590.4597 | 1204 | 4.26E-08 | 5.84 | 3.62 | 13.99 | 7.19 |
| 34 | 592.4728 | 1204 | 7.85E-07 | 1.11 | 0.37 | 2.02 | 0.82 |
| 35 | 594.4857 | 1204 | 1.68E-06 | 7.18 | 4.76 | 16.02 | 7.57 |
| 36 | 596.5015 | 1204 | 1.12E-05 | 2.31 | 1.32 | 5.96 | 3.40 |
| 37 | 598.5121 | 1204 | 2.50E-05 | 12.95 | 9.28 | 36.87 | 22.12 |

**Table 3: List of 29-metabolite subset detected by TOF MS, based upon the previous subset of 37 metabolites.**

| | |
|---|---|
| | **Detected Mass** |
| 1 | 484.3907 |
| 2 | 490.3800 |
| 3 | 512.4196 |
| 4 | 540.4529 |
| 5 | 446.3544 |
| 6 | 538.4361 |
| 7 | 518.4161 |
| 8 | 468.3986 |
| 9 | 492.3930 |
| 10 | 448.3715 |
| 11 | 494.4120 |
| 12 | 474.3872 |
| 13 | 450.3804 |
| 14 | 594.5027 |
| 15 | 520.4193 |
| 16 | 596.5191 |
| 17 | 598.5174 |
| 18 | 522.4410 |
| 19 | 574.4707 |
| 20 | 502.4181 |
| 21 | 592.4198 |
| 22 | 478.4209 |
| 23 | 550.4667 |
| 24 | 504.4333 |
| 25 | 476.4885 |
| 26 | 530.4435 |
| 27 | 578.5034 |
| 28 | 532.4690 |
| 29 | 558.4816 |

### MSMS Fragments for Selected Ovarian Cancer Diagnostic Masses

Each table shows the collision energy in voltage, the HPLC retention time in minutes and the percent intensity of the fragment ion. Masses in the title of the table are neutral, while the masses listed under m/z (amu) are [M-H].

**Table 4**

| **446.4** | | |
|---|---|---|
| **CE: -35 V** | **16.4 min** | |
| ***m***/***z (amu)*** | ***intensity (counts)*** | ***% intensity*** |
| 401.3402 | 10.3333 | 100 |
| 445.3398 | 8.1667 | 79.0323 |
| 427.3226 | 4.5 | 43.5484 |
| 83.0509 | 2.8333 | 27.4194 |
| 223.1752 | 2.5 | 24.1935 |
| 222.1558 | 2.1667 | 20.9677 |
| 205.1506 | 1.8333 | 17.7419 |
| 383.3338 | 1.8333 | 17.7419 |
| 59.0097 | 1.6667 | 16.129 |
| 97.0644 | 1 | 9.6774 |
| 81.0348 | 0.6667 | 6.4516 |
| 109.0709 | 0.6667 | 6.4516 |
| 203.1555 | 0.6667 | 6.4516 |
| 221.1443 | 0.6667 | 6.4516 |
| 409.2901 | 0.6667 | 6.4516 |
| 123.0814 | 0.5 | 4.8387 |
| 177.1904 | 0.5 | 4.8387 |
| 233.2224 | 0.5 | 4.8387 |
| 259.2236 | 0.5 | 4.8387 |
| 428.3086 | 0.5 | 4.8387 |

**Table 5**

| **448.4** | | |
|---|---|---|
| **CE: -35 V** | **16.6 min** | |
| ***m*/*z (amu)*** | ***intensity (counts)*** | ***% intensity*** |
| 403.3581 | 3.75 | 100 |
| 429.3269 | 1.75 | 46.6667 |
| 447.362 | 1.5 | 40 |
| 385.3944 | 1 | 26.6667 |
| 83.0543 | 0.75 | 20 |
| 447.1556 | 0.75 | 20 |
| 111.0912 | 0.5 | 13.3333 |
| 151.1253 | 0.5 | 13.3333 |
| 402.4012 | 0.5 | 13.3333 |
| 411.3049 | 0.5 | 13.3333 |
| 429.4669 | 0.5 | 13.3333 |
| 59.0299 | 0.25 | 6.6667 |
| 69.0397 | 0.25 | 6.6667 |
| 74.0264 | 0.25 | 6.6667 |
| 81.0348 | 0.25 | 6.6667 |
| 187.1241 | 0.25 | 6.6667 |
| 223.192 | 0.25 | 6.6667 |
| 279.2183 | 0.25 | 6.6667 |
| 385.5049 | 0.25 | 6.6667 |
| 404.3538 | 0.25 | 6.6667 |

**Table 6**

| **450.4** | | |
|---|---|---|
| **CE: -35 V** | **16.7 min** | |
| ***m*/*z (amu)*** | ***intensity (counts)*** | ***% intensity*** |
| 431.3514 | 19 | 100 |
| 449.3649 | 15.25 | 80.2632 |
| 405.3885 | 10 | 52.6316 |
| 387.3718 | 4.5 | 23.6842 |
| 405.4792 | 1.5 | 7.8947 |
| 111.0833 | 1.25 | 6.5789 |
| 413.34 | 1.25 | 6.5789 |
| 432.4279 | 1 | 5.2632 |
| 59.0213 | 0.75 | 3.9474 |
| 71.0502 | 0.75 | 3.9474 |
| 97.0681 | 0.75 | 3.9474 |
| 281.2668 | 0.75 | 3.9474 |
| 406.4473 | 0.75 | 3.9474 |
| 450.3442 | 0.75 | 3.9474 |
| 57.0312 | 0.5 | 2.6316 |
| 83.0646 | 0.5 | 2.6316 |
| 123.0772 | 0.5 | 2.6316 |
| 125.0926 | 0.5 | 2.6316 |
| 181.1546 | 0.5 | 2.6316 |
| 233.2167 | 0.5 | 2.6316 |

**Table 7**

| **468.4** | | |
|---|---|---|
| **CE: -35 V** | **16.4 min** | |
| ***m*/*z (amu)*** | ***intensity (counts)*** | ***% intensity*** |
| 449.3774 | 10.5 | 100 |
| 467.3807 | 7.5 | 71.4286 |
| 187.139 | 4 | 38.0952 |
| 449.4809 | 2 | 19.0476 |
| 263.2327 | 1.5 | 14.2857 |
| 423.3984 | 1.5 | 14.2857 |
| 141.1375 | 1.25 | 11.9048 |
| 279.2257 | 1.25 | 11.9048 |
| 169.1366 | 1 | 9.5238 |
| 450.4126 | 1 | 9.5238 |
| 215.188 | 0.75 | 7.1429 |
| 297.2482 | 0.75 | 7.1429 |
| 405.3868 | 0.75 | 7.1429 |
| 468.4527 | 0.75 | 7.1429 |
| 185.1619 | 0.5 | 4.7619 |
| 188.1521 | 0.5 | 4.7619 |
| 213.1552 | 0.5 | 4.7619 |
| 251.2335 | 0.5 | 4.7619 |
| 281.2619 | 0.5 | 4.7619 |
| 113.0926 | 0.25 | 2.381 |

**Table 8**

| **474.4** | | |
|---|---|---|
| **CE: -35 V** | **16.6 min** | |
| ***m*/*z (amu)*** | ***intensity (counts)*** | ***% intensity*** |
| 473.3896 | 1.8 | 100 |
| 455.3659 | 1.05 | 58.3333 |
| 85.0314 | 0.45 | 25 |
| 113.0367 | 0.45 | 25 |
| 455.4621 | 0.35 | 19.4444 |
| 57.0519 | 0.15 | 8.3333 |
| 71.0216 | 0.15 | 8.3333 |
| 97.0682 | 0.15 | 8.3333 |
| 117.0187 | 0.15 | 8.3333 |
| 222.1549 | 0.15 | 8.3333 |
| 456.416 | 0.15 | 8.3333 |
| 473.5285 | 0.15 | 8.3333 |
| 411.3954 | 0.7 | 38.8889 |
| 429.3674 | 0.6 | 33.3333 |
| 75.0151 | 0.5 | 27.7778 |
| 474.3539 | 0.3 | 16.6667 |
| 474.4194 | 0.3 | 16.6667 |
| 223.1912 | 0.2 | 11.1111 |
| 429.4608 | 0.2 | 11.1111 |
| 59.0166 | 0.1 | 5.5556 |

**Table 9**

| **476.5** | | |
|---|---|---|
| **CE: -35 V** | **16.8 min** | |
| ***m*/*z (amu)*** | ***intensity (counts)*** | ***% intensity*** |
| 475.3847 | 4.1818 | 100 |
| 457.387 | 2.9091 | 69.5652 |
| 431.4157 | 1.5455 | 36.9565 |
| 413.4004 | 0.8182 | 19.5652 |
| 279.2634 | 0.4545 | 10.8696 |
| 439.3666 | 0.3636 | 8.6957 |
| 458.3751 | 0.3636 | 8.6957 |
| 458.4715 | 0.3636 | 8.6957 |
| 476.474 | 0.2727 | 6.5217 |
| 57.0378 | 0.1818 | 4.3478 |
| 59.0253 | 0.1818 | 4.3478 |
| 83.0594 | 0.1818 | 4.3478 |
| 97.0756 | 0.1818 | 4.3478 |
| 111.0934 | 0.1818 | 4.3478 |
| 123.0937 | 0.1818 | 4.3478 |
| 235.2167 | 0.1818 | 4.3478 |
| 251.2216 | 0.1818 | 4.3478 |
| 414.401 | 0.1818 | 4.3478 |
| 432.43 | 0.1818 | 4.3478 |
| 71.0121 | 0.0909 | 2.1739 |

**Table 10**

| **478.4** | | |
|---|---|---|
| **CE: -35 V** | **17.1 min** | |
| ***m*/*z (amu)*** | ***intensity (counts)*** | ***% intensity*** |
| 477.3923 | 7.4286 | 100 |
| 459.3884 | 5.2857 | 71.1538 |
| 433.3986 | 2 | 26.9231 |
| 415.3951 | 1.6429 | 22.1154 |
| 478.4099 | 0.7857 | 10.5769 |
| 433.508 | 0.5 | 6.7308 |
| 460.4028 | 0.5 | 6.7308 |
| 125.0717 | 0.3571 | 4.8077 |
| 281.2682 | 0.3571 | 4.8077 |
| 97.0682 | 0.2857 | 3.8462 |
| 111.0815 | 0.2857 | 3.8462 |
| 434.5091 | 0.2857 | 3.8462 |
| 59.0224 | 0.2143 | 2.8846 |
| 123.0979 | 0.2143 | 2.8846 |
| 223.2193 | 0.2143 | 2.8846 |
| 416.4057 | 0.2143 | 2.8846 |
| 434.3839 | 0.2143 | 2.8846 |
| 435.3703 | 0.2143 | 2.8846 |
| 441.4307 | 0.2143 | 2.8846 |
| 477.22 | 0.2143 | 2.8846 |

**Table 11**

| **484.4** | | |
|---|---|---|
| **CE: -40 V** | **15.6 min** | |
| ***m*/*z (amu)*** | ***intensity (counts)*** | ***% intensity*** |
| 315.254 | 1.8333 | 100 |
| 123.1312 | 0.8333 | 45.4545 |
| 297.2741 | 0.8333 | 45.4545 |
| 185.1313 | 0.6667 | 36.3636 |
| 465.4187 | 0.6667 | 36.3636 |
| 279.2508 | 0.5 | 27.2727 |
| 439.4138 | 0.5 | 27.2727 |
| 483.3989 | 0.5 | 27.2727 |
| 171.1296 | 0.3333 | 18.1818 |
| 187.1442 | 0.3333 | 18.1818 |
| 201.161 | 0.3333 | 18.1818 |
| 223.1744 | 0.3333 | 18.1818 |
| 241.2311 | 0.3333 | 18.1818 |
| 295.2515 | 0.3333 | 18.1818 |
| 313.2575 | 0.3333 | 18.1818 |
| 315.3674 | 0.3333 | 18.1818 |
| 421.3846 | 0.3333 | 18.1818 |
| 447.3345 | 0.3333 | 18.1818 |
| 100.8663 | 0.1667 | 9.0909 |
| 111.1092 | 0.1667 | 9.0909 |

**Table 12**

| **490.4** | | |
|---|---|---|
| **CE: -35 V** | **16.1 min** | |
| ***m*/*z (amu)*** | ***intensity (counts)*** | ***% intensity*** |
| 489.3601 | 1.1739 | 100 |
| 319.2795 | 0.413 | 35.1852 |
| 445.3516 | 0.3696 | 31.4815 |
| 241.1903 | 0.3478 | 29.6296 |
| 471.3416 | 0.3478 | 29.6296 |
| 427.3472 | 0.1957 | 16.6667 |
| 113.1006 | 0.1739 | 14.8148 |
| 195.121 | 0.1739 | 14.8148 |
| 223.18 | 0.1739 | 14.8148 |
| 249.1847 | 0.1739 | 14.8148 |
| 490.3405 | 0.1739 | 14.8148 |
| 97.0682 | 0.1522 | 12.963 |
| 267.2006 | 0.1522 | 12.963 |
| 345.279 | 0.1304 | 11.1111 |
| 57.0349 | 0.1087 | 9.2593 |
| 101.0209 | 0.1087 | 9.2593 |
| 143.0888 | 0.1087 | 9.2593 |
| 265.1915 | 0.1087 | 9.2593 |
| 373.2819 | 0.1087 | 9.2593 |
| 472.3936 | 0.1087 | 9.2593 |

**Table 13**

| **492.4** | | |
|---|---|---|
| **CE: -40 V** | **16.7 min** | |
| ***m*/*z (amu)*** | ***intensity (counts)*** | ***% intensity*** |
| 241.1845 | 4.3077 | 100 |
| 249.1966 | 2.6923 | 62.5 |
| 267.2006 | 2.4615 | 57.1429 |
| 97.0682 | 1.8462 | 42.8571 |
| 473.3569 | 1.3846 | 32.1429 |
| 223.1632 | 1.1538 | 26.7857 |
| 195.1839 | 1 | 23.2143 |
| 143.0663 | 0.9231 | 21.4286 |
| 447.3901 | 0.9231 | 21.4286 |
| 101.0285 | 0.8462 | 19.6429 |
| 491.3636 | 0.8462 | 19.6429 |
| 113.1046 | 0.7692 | 17.8571 |
| 319.2661 | 0.6923 | 16.0714 |
| 57.0434 | 0.5385 | 12.5 |
| 59.0224 | 0.4615 | 10.7143 |
| 213.1826 | 0.4615 | 10.7143 |
| 167.1505 | 0.3846 | 8.9286 |
| 171.1149 | 0.3846 | 8.9286 |
| 179.188 | 0.3846 | 8.9286 |
| 193.1595 | 0.3846 | 8.9286 |

**Table 14**

| **494.4** | | |
|---|---|---|
| **CE: -35 V** | **16.7 min** | |
| ***m*/*z (amu)*** | ***intensity (counts)*** | ***% intensity*** |
| 493.3767 | 3 | 100 |
| 475.3845 | 2.6667 | 88.8889 |
| 215.1568 | 1.6667 | 55.5556 |
| 195.1308 | 1.3333 | 44.4444 |
| 213.1519 | 1.3333 | 44.4444 |
| 449.4047 | 1 | 33.3333 |
| 167.144 | 0.6667 | 22.2222 |
| 171.1421 | 0.6667 | 22.2222 |
| 241.2352 | 0.6667 | 22.2222 |
| 267.2011 | 0.6667 | 22.2222 |
| 279.2433 | 0.6667 | 22.2222 |
| 297.2703 | 0.6667 | 22.2222 |
| 307.2744 | 0.6667 | 22.2222 |
| 431.3748 | 0.6667 | 22.2222 |
| 493.5185 | 0.6667 | 22.2222 |
| 494.4362 | 0.6667 | 22.2222 |
| 113.0902 | 0.3333 | 11.1111 |
| 141.1351 | 0.3333 | 11.1111 |
| 151.1484 | 0.3333 | 11.1111 |
| 197.1653 | 0.3333 | 11.1111 |

**Table 15**

| **496.2** | | |
|---|---|---|
| **CE: -35 V** | **16.9 min** | |
| ***m*/*z (amu)*** | ***intensity (counts)*** | ***% intensity*** |
| 495.4216 | 12.6667 | 100 |
| 215.1623 | 8.6667 | 68.4211 |
| 477.4 | 5.6667 | 44.7368 |
| 197.1548 | 4.3333 | 34.2105 |
| 279.2559 | 2.3333 | 18.4211 |
| 297.2573 | 2 | 15.7895 |
| 169.1737 | 1.3333 | 10.5263 |
| 213.1683 | 1.3333 | 10.5263 |
| 433.4433 | 1.3333 | 10.5263 |
| 171.1077 | 1 | 7.8947 |
| 451.476 | 1 | 7.8947 |
| 179.1444 | 0.6667 | 5.2632 |
| 195.1466 | 0.6667 | 5.2632 |
| 241.2119 | 0.6667 | 5.2632 |
| 496.3828 | 0.6667 | 5.2632 |
| 83.0475 | 0.3333 | 2.6316 |
| 84.0218 | 0.3333 | 2.6316 |
| 111.0833 | 0.3333 | 2.6316 |
| 223.1472 | 0.3333 | 2.6316 |
| 225.1985 | 0.3333 | 2.6316 |

**Table 16**

| **502.4** | | |
|---|---|---|
| **CE: -35 V** | **17 min** | |
| ***m*/*z (amu)*** | ***intensity (counts)*** | ***% intensity*** |
| 483.3824 | 1.0435 | 100 |
| 501.4088 | 0.913 | 87.5 |
| 439.3981 | 0.7391 | 70.8333 |
| 457.4191 | 0.5217 | 50 |
| 501.5013 | 0.2609 | 25 |
| 279.2634 | 0.1739 | 16.6667 |
| 458.4876 | 0.1739 | 16.6667 |
| 484.423 | 0.1739 | 16.6667 |
| 502.4433 | 0.1739 | 16.6667 |
| 59.0195 | 0.1304 | 12.5 |
| 109.108 | 0.1304 | 12.5 |
| 111.0894 | 0.1304 | 12.5 |
| 123.1229 | 0.1304 | 12.5 |
| 196.0608 | 0.1304 | 12.5 |
| 221.1879 | 0.1304 | 12.5 |
| 222.1716 | 0.1304 | 12.5 |
| 277.2469 | 0.1304 | 12.5 |
| 317.3037 | 0.1304 | 12.5 |
| 440.3981 | 0.1304 | 12.5 |
| 465.3782 | 0.1304 | 12.5 |

**Table 17**

| **504.4** | | |
|---|---|---|
| **CE: -40 V** | **17.2 min** | |
| ***m*/*z (amu)*** | ***intensity (counts)*** | ***% intensity*** |
| 485.415 | 5.8947 | 100 |
| 503.4284 | 4.0526 | 68.75 |
| 441.415 | 2.5789 | 43.75 |
| 459.4366 | 1.2105 | 20.5357 |
| 486.4246 | 0.6842 | 11.6071 |
| 97.0719 | 0.4211 | 7.1429 |
| 111.0855 | 0.3684 | 6.25 |
| 467.397 | 0.3158 | 5.3571 |
| 504.4312 | 0.3158 | 5.3571 |
| 57.0434 | 0.2632 | 4.4643 |
| 223.1632 | 0.2632 | 4.4643 |
| 263.2388 | 0.2632 | 4.4643 |
| 377.3256 | 0.2632 | 4.4643 |
| 442.4567 | 0.2632 | 4.4643 |
| 169.1464 | 0.2105 | 3.5714 |
| 279.2383 | 0.2105 | 3.5714 |
| 329.3051 | 0.2105 | 3.5714 |
| 59.0166 | 0.1579 | 2.6786 |
| 71.0216 | 0.1579 | 2.6786 |
| 83.0662 | 0.1579 | 2.6786 |

**Table 18**

| **512.4** | | |
|---|---|---|
| **CE: -35 V** | **16.0 min** | |
| ***m*/*z (amu)*** | ***intensity (counts)*** | ***% intensity*** |
| 315.2675 | 12 | 100 |
| 511.3975 | 8.5 | 70.8333 |
| 151.1622 | 2.3333 | 19.4444 |
| 213.1464 | 1.8333 | 15.2778 |
| 297.2767 | 1.5 | 12.5 |
| 493.4184 | 1.3333 | 11.1111 |
| 195.1361 | 1 | 8.3333 |
| 279.2433 | 1 | 8.3333 |
| 511.5163 | 0.8333 | 6.9444 |
| 512.4081 | 0.6667 | 5.5556 |
| 141.1351 | 0.5 | 4.1667 |
| 171.0979 | 0.5 | 4.1667 |
| 313.2579 | 0.5 | 4.1667 |
| 467.3898 | 0.5 | 4.1667 |
| 169.1591 | 0.3333 | 2.7778 |
| 177.1304 | 0.3333 | 2.7778 |
| 231.1633 | 0.3333 | 2.7778 |
| 251.1945 | 0.3333 | 2.7778 |
| 259.2115 | 0.3333 | 2.7778 |
| 314.242 | 0.3333 | 2.7778 |

**Table 19**

| **518.4** | | |
|---|---|---|
| **CE: -40 V** | **16.9 min** | |
| ***m*/*z (amu)*** | ***intensity (counts)*** | ***% intensity*** |
| 517.3886 | 0.8182 | 100 |
| 499.3933 | 0.5909 | 72.2222 |
| 115.0412 | 0.4091 | 50 |
| 455.39 | 0.3636 | 44.4444 |
| 171.1001 | 0.3182 | 38.8889 |
| 171.1296 | 0.3182 | 38.8889 |
| 473.4223 | 0.2727 | 33.3333 |
| 59.0166 | 0.2273 | 27.7778 |
| 401.3229 | 0.2273 | 27.7778 |
| 499.494 | 0.2273 | 27.7778 |
| 113.1046 | 0.1818 | 22.2222 |
| 389.3725 | 0.1818 | 22.2222 |
| 437.4015 | 0.1818 | 22.2222 |
| 481.3541 | 0.1818 | 22.2222 |
| 71.0152 | 0.1364 | 16.6667 |
| 111.0855 | 0.1364 | 16.6667 |
| 125.1095 | 0.1364 | 16.6667 |
| 203.1412 | 0.1364 | 16.6667 |
| 223.152 | 0.1364 | 16.6667 |
| 445.3833 | 0.1364 | 16.6667 |

**Table 20**

| **520.4** | | |
|---|---|---|
| **CE: -42 V** | **16.8 min** | |
| ***m*/*z (amu)*** | ***intensity (counts)*** | ***% intensity*** |
| 501.392 | 2.2353 | 100 |
| 519.4144 | 1.3824 | 61.8421 |
| 457.403 | 0.8235 | 36.8421 |
| 475.4257 | 0.6176 | 27.6316 |
| 115.0412 | 0.4118 | 18.4211 |
| 59.0195 | 0.3529 | 15.7895 |
| 83.0662 | 0.3529 | 15.7895 |
| 459.3964 | 0.3529 | 15.7895 |
| 502.4013 | 0.3529 | 15.7895 |
| 241.1903 | 0.3235 | 14.4737 |
| 297.2482 | 0.2647 | 11.8421 |
| 71.0152 | 0.2353 | 10.5263 |
| 195.1735 | 0.2353 | 10.5263 |
| 223.1688 | 0.2353 | 10.5263 |
| 279.232 | 0.2353 | 10.5263 |
| 447.398 | 0.2353 | 10.5263 |
| 483.4154 | 0.2353 | 10.5263 |
| 97.0719 | 0.2059 | 9.2105 |
| 111.0894 | 0.2059 | 9.2105 |
| 221.1655 | 0.2059 | 9.2105 |

**Table 21**

| **522.4** | | |
|---|---|---|
| **CE: -40 V** | **16.9 min** | |
| ***m*/*z (amu)*** | ***intensity (counts)*** | ***% intensity*** |
| 521.427 | 1.375 | 100 |
| 503.4115 | 1.2917 | 93.9394 |
| 459.4125 | 0.375 | 27.2727 |
| 241.1903 | 0.3333 | 24.2424 |
| 477.4415 | 0.3333 | 24.2424 |
| 503.5295 | 0.25 | 18.1818 |
| 111.0934 | 0.2083 | 15.1515 |
| 115.0453 | 0.2083 | 15.1515 |
| 171.1149 | 0.2083 | 15.1515 |
| 267.219 | 0.2083 | 15.1515 |
| 297.2611 | 0.2083 | 15.1515 |
| 441.4228 | 0.2083 | 15.1515 |
| 223.1688 | 0.1667 | 12.1212 |
| 269.248 | 0.1667 | 12.1212 |
| 271.2537 | 0.1667 | 12.1212 |
| 279.2383 | 0.1667 | 12.1212 |
| 485.415 | 0.1667 | 12.1212 |
| 522.3961 | 0.1667 | 12.1212 |
| 57.0378 | 0.125 | 9.0909 |
| 59.0138 | 0.125 | 9.0909 |

**Table 22**

| **530.4** | | |
|---|---|---|
| **CE: -40 V** | **17.5 min** | |
| ***m*/*z (amu)*** | ***intensity (counts)*** | ***% intensity*** |
| 529.4472 | 1.1563 | 100 |
| 467.4457 | 0.8125 | 70.2703 |
| 511.4368 | 0.8125 | 70.2703 |
| 529.5422 | 0.2188 | 18.9189 |
| 85.0314 | 0.1563 | 13.5135 |
| 485.4564 | 0.1563 | 13.5135 |
| 511.5557 | 0.1563 | 13.5135 |
| 512.4137 | 0.1563 | 13.5135 |
| 75.0216 | 0.125 | 10.8108 |
| 468.4608 | 0.125 | 10.8108 |
| 177.1785 | 0.0938 | 8.1081 |
| 250.1932 | 0.0938 | 8.1081 |
| 251.1978 | 0.0938 | 8.1081 |
| 530.4237 | 0.0938 | 8.1081 |
| 59.0195 | 0.0625 | 5.4054 |
| 97.0645 | 0.0625 | 5.4054 |
| 109.112 | 0.0625 | 5.4054 |
| 113.0567 | 0.0625 | 5.4054 |
| 195.1839 | 0.0625 | 5.4054 |
| 205.2065 | 0.0625 | 5.4054 |

**Table 23**

| **532.5** | | |
|---|---|---|
| **CE: -42 V** | **17.5 min** | |
| ***m*/*z (amu)*** | ***intensity (counts)*** | ***% intensity*** |
| 513.4424 | 1.375 | 100 |
| 469.4526 | 1.25 | 90.9091 |
| 531.4531 | 0.9375 | 68.1818 |
| 195.1315 | 0.25 | 18.1818 |
| 469.5828 | 0.25 | 18.1818 |
| 470.4455 | 0.25 | 18.1818 |
| 111.0855 | 0.1875 | 13.6364 |
| 181.1331 | 0.1875 | 13.6364 |
| 251.1978 | 0.1875 | 13.6364 |
| 487.4436 | 0.1875 | 13.6364 |
| 514.4552 | 0.1875 | 13.6364 |
| 532.4142 | 0.1875 | 13.6364 |
| 59.0138 | 0.125 | 9.0909 |
| 71.0121 | 0.125 | 9.0909 |
| 97.0682 | 0.125 | 9.0909 |
| 113.0647 | 0.125 | 9.0909 |
| 127.0909 | 0.125 | 9.0909 |
| 495.4413 | 0.125 | 9.0909 |
| 513.6126 | 0.125 | 9.0909 |
| 531.6003 | 0.125 | 9.0909 |

**Table 24**

| **538.4** | | |
|---|---|---|
| **CE: -40 V** | **16.4 min** | |
| ***m*/*z (amu)*** | ***intensity (counts)*** | ***% intensity*** |
| 537.4416 | 1.6667 | 100 |
| 519.3973 | 1 | 60 |
| 475.4175 | 0.6667 | 40 |
| 493.4212 | 0.4444 | 26.6667 |
| 59.0224 | 0.3333 | 20 |
| 115.0493 | 0.3333 | 20 |
| 333.3025 | 0.3333 | 20 |
| 501.4088 | 0.3333 | 20 |
| 519.5598 | 0.3333 | 20 |
| 537.5721 | 0.3333 | 20 |
| 101.0285 | 0.2222 | 13.3333 |
| 315.274 | 0.2222 | 13.3333 |
| 457.395 | 0.2222 | 13.3333 |
| 538.3471 | 0.2222 | 13.3333 |
| 538.4516 | 0.2222 | 13.3333 |
| 71.0216 | 0.1111 | 6.6667 |
| 143.1157 | 0.1111 | 6.6667 |
| 171.1493 | 0.1111 | 6.6667 |
| 179.183 | 0.1111 | 6.6667 |
| 221.1655 | 0.1111 | 6.6667 |

**Table 25**

| **540.5** | | |
|---|---|---|
| **CE: -35 V** | **16.3 min** | |
| ***m*/*z (amu)*** | ***intensity (counts)*** | ***% intensity*** |
| 315.2675 | 24.6 | 100 |
| 539.4356 | 15.6 | 63.4146 |
| 223.1696 | 2.4 | 9.7561 |
| 179.1896 | 2.2 | 8.9431 |
| 521.4115 | 1.8 | 7.3171 |
| 297.2703 | 1.2 | 4.878 |
| 495.455 | 1.2 | 4.878 |
| 477.4492 | 0.8 | 3.252 |
| 539.5664 | 0.8 | 3.252 |
| 241.1886 | 0.6 | 2.439 |
| 259.2055 | 0.6 | 2.439 |
| 316.2614 | 0.6 | 2.439 |
| 540.395 | 0.6 | 2.439 |
| 125.1052 | 0.4 | 1.626 |
| 171.1519 | 0.4 | 1.626 |
| 225.176 | 0.4 | 1.626 |
| 257.1789 | 0.4 | 1.626 |
| 279.2496 | 0.4 | 1.626 |
| 313.2314 | 0.4 | 1.626 |
| 314.1621 | 0.4 | 1.626 |

**Table 26**

| **550.5** | | |
|---|---|---|
| **CE: -42 V** | **17.2 min** | |
| ***m*/*z (amu)*** | ***intensity (counts)*** | ***% intensity*** |
| 487.4684 | 1 | 100 |
| 549.4751 | 0.9286 | 92.8571 |
| 531.4531 | 0.7857 | 78.5714 |
| 251.2156 | 0.5714 | 57.1429 |
| 253.2248 | 0.5714 | 57.1429 |
| 111.0934 | 0.4286 | 42.8571 |
| 125.0969 | 0.4286 | 42.8571 |
| 269.2233 | 0.4286 | 42.8571 |
| 271.2475 | 0.4286 | 42.8571 |
| 277.2282 | 0.4286 | 42.8571 |
| 513.468 | 0.4286 | 42.8571 |
| 71.0184 | 0.3571 | 35.7143 |
| 171.1198 | 0.3571 | 35.7143 |
| 297.2417 | 0.3571 | 35.7143 |
| 469.477 | 0.3571 | 35.7143 |
| 115.0815 | 0.2857 | 28.5714 |
| 279.2759 | 0.2857 | 28.5714 |
| 295.2709 | 0.2857 | 28.5714 |
| 433.3751 | 0.2857 | 28.5714 |
| 505.5026 | 0.2857 | 28.5714 |

**Table 27**

| **558.5** | | |
|---|---|---|
| **CE: -35 V** | **17.8 min** | |
| ***m***/***z (amu)*** | ***intensity (counts)*** | ***% intensity*** |
| 557.4735 | 34 | 100 |
| 557.5798 | 3.3333 | 9.8039 |
| 539.4879 | 2 | 5.8824 |
| 495.48 | 1.6667 | 4.902 |
| 278.2406 | 1.3333 | 3.9216 |
| 558.431 | 1.3333 | 3.9216 |
| 279.2371 | 1 | 2.9412 |
| 123.1189 | 0.6667 | 1.9608 |
| 277.2335 | 0.6667 | 1.9608 |
| 496.433 | 0.6667 | 1.9608 |
| 513.4368 | 0.6667 | 1.9608 |
| 127.1074 | 0.3333 | 0.9804 |
| 155.1198 | 0.3333 | 0.9804 |
| 221.1331 | 0.3333 | 0.9804 |
| 279.3563 | 0.3333 | 0.9804 |
| 373.3606 | 0.3333 | 0.9804 |
| 522.4406 | 0.3333 | 0.9804 |
| 555.3219 | 0.3333 | 0.9804 |
| 557.9876 | 0.3333 | 0.9804 |
| 558.3246 | 0.3333 | 0.9804 |

**Table 28**

| **574.5** | | |
|---|---|---|
| **CE: -42 V** | **17.0 min** | |
| ***m*/*z (amu)*** | ***intensity (counts)*** | ***% intensity*** |
| 573.4742 | 1.0571 | 100 |
| 295.2386 | 0.7143 | 67.5676 |
| 555.4666 | 0.5714 | 54.0541 |
| 125.1053 | 0.4857 | 45.9459 |
| 279.2508 | 0.4857 | 45.9459 |
| 171.1051 | 0.4571 | 43.2432 |
| 223.1408 | 0.4286 | 40.5405 |
| 511.4199 | 0.4 | 37.8378 |
| 157.085 | 0.3429 | 32.4324 |
| 493.4546 | 0.3429 | 32.4324 |
| 183.1039 | 0.2857 | 27.027 |
| 277.2282 | 0.2571 | 24.3243 |
| 293.2359 | 0.2571 | 24.3243 |
| 401.3605 | 0.2286 | 21.6216 |
| 113.0966 | 0.2 | 18.9189 |
| 293.2102 | 0.2 | 18.9189 |
| 429.3752 | 0.2 | 18.9189 |
| 249.2203 | 0.1714 | 16.2162 |
| 385.3457 | 0.1714 | 16.2162 |
| 389.3651 | 0.1714 | 16.2162 |

**Table 29**

| **576.5** | | |
|---|---|---|
| **CE: -42 V** | **17.3 min** | |
| ***m*/*z (amu)*** | ***intensity (counts)*** | ***% intensity*** |
| 575.4808 | 2.9048 | 100 |
| 277.2219 | 1.4286 | 49.1803 |
| 297.2676 | 1.4286 | 49.1803 |
| 557.4591 | 1.2381 | 42.623 |
| 513.4765 | 0.9524 | 32.7869 |
| 279.2445 | 0.8095 | 27.8689 |
| 171.11 | 0.7619 | 26.2295 |
| 183.114 | 0.5238 | 18.0328 |
| 295.2322 | 0.5238 | 18.0328 |
| 125.0969 | 0.4762 | 16.3934 |
| 403.3711 | 0.4286 | 14.7541 |
| 111.0775 | 0.381 | 13.1148 |
| 495.458 | 0.381 | 13.1148 |
| 251.2394 | 0.3333 | 11.4754 |
| 293.2102 | 0.3333 | 11.4754 |
| 97.0682 | 0.2857 | 9.8361 |
| 113.0926 | 0.2857 | 9.8361 |
| 205.2011 | 0.2857 | 9.8361 |
| 223.1351 | 0.2857 | 9.8361 |
| 296.2329 | 0.2857 | 9.8361 |

**Table 30**

| **578.5** | | |
|---|---|---|
| **CE: -35 V** | **16.8 min** | |
| ***m*/*z (amu)*** | ***intensity (counts)*** | ***% intensity*** |
| 113.0287 | 4.25 | 100 |
| 103.0116 | 1 | 23.5294 |
| 175.0313 | 1 | 23.5294 |
| 85.0349 | 0.75 | 17.6471 |
| 99.0123 | 0.75 | 17.6471 |
| 75.0119 | 0.5 | 11.7647 |
| 95.0153 | 0.5 | 11.7647 |
| 129.0153 | 0.5 | 11.7647 |
| 497.4489 | 0.5 | 11.7647 |
| 577.4728 | 0.5 | 11.7647 |
| 71.0089 | 0.25 | 5.8824 |
| 87.0021 | 0.25 | 5.8824 |
| 114.0248 | 0.25 | 5.8824 |
| 115.0171 | 0.25 | 5.8824 |
| 117.0105 | 0.25 | 5.8824 |
| 576.0393 | 0.25 | 5.8824 |

**Table 31**

| **592.5** | | |
|---|---|---|
| **CE: -35 V** | **17.0 min** | |
| ***m*/*z (amu)*** | ***intensity (counts)*** | ***% intensity*** |
| 113.0248 | 16.1667 | 100 |
| 85.0418 | 3.3333 | 20.6186 |
| 103.0116 | 2 | 12.3711 |
| 175.0214 | 2 | 12.3711 |
| 117.0227 | 1.6667 | 10.3093 |
| 59.0224 | 1.3333 | 8.2474 |
| 75.0151 | 1.3333 | 8.2474 |
| 95.0226 | 1.3333 | 8.2474 |
| 99.0123 | 1.3333 | 8.2474 |
| 115.009 | 1 | 6.1856 |
| 149.0733 | 1 | 6.1856 |
| 87.0126 | 0.8333 | 5.1546 |
| 129.0153 | 0.8333 | 5.1546 |
| 591.4221 | 0.8333 | 5.1546 |
| 157.0097 | 0.6667 | 4.1237 |
| 415.3721 | 0.6667 | 4.1237 |
| 73.0352 | 0.5 | 3.0928 |
| 415.4945 | 0.5 | 3.0928 |
| 71.0152 | 0.3333 | 2.0619 |
| 89.0307 | 0.3333 | 2.0619 |

**Table 32**

| **594.5** | | |
|---|---|---|
| **CE: -50 V** | **16.7 min** | |
| ***m*/*z (amu)*** | ***intensity (counts)*** | ***% intensity*** |
| 371.3397 | 4.2 | 100 |
| 171.1077 | 3.6 | 85.7143 |
| 315.2609 | 3.6 | 85.7143 |
| 575.4927 | 3.6 | 85.7143 |
| 277.2335 | 3.4 | 80.9524 |
| 201.1328 | 3 | 71.4286 |
| 295.2351 | 2.8 | 66.6667 |
| 297.2832 | 2.8 | 66.6667 |
| 593.4968 | 2.8 | 66.6667 |
| 279.2496 | 2.4 | 57.1429 |
| 557.4646 | 2.2 | 52.381 |
| 141.1351 | 1.8 | 42.8571 |
| 313.2513 | 1.6 | 38.0952 |
| 513.4793 | 1.6 | 38.0952 |
| 557.438 | 1.6 | 38.0952 |
| 125.0968 | 1.4 | 33.3333 |
| 593.57 | 1.4 | 33.3333 |
| 575.6008 | 1.2 | 28.5714 |
| 113.0941 | 1 | 23.8095 |
| 139.1134 | 1 | 23.8095 |

**Table 33**

| **596.5** | | |
|---|---|---|
| **CE: -50 V** | **16.9 min** | |
| ***m*/*z (amu)*** | ***intensity (counts)*** | ***% intensity*** |
| 279.2433 | 53.6 | 100 |
| 315.2609 | 35.8 | 66.791 |
| 297.2638 | 21.6 | 40.2985 |
| 313.2447 | 9.6 | 17.9104 |
| 577.5116 | 7.4 | 13.806 |
| 281.2542 | 6.8 | 12.6866 |
| 595.5011 | 6.2 | 11.5672 |
| 295.2416 | 3.6 | 6.7164 |
| 171.1028 | 3.4 | 6.3433 |
| 515.5056 | 3.2 | 5.9701 |
| 559.4693 | 2.6 | 4.8507 |
| 125.101 | 2.4 | 4.4776 |
| 141.1261 | 2 | 3.7313 |
| 127.1201 | 1.8 | 3.3582 |
| 155.1431 | 1.6 | 2.9851 |
| 169.1249 | 1.4 | 2.6119 |
| 185.1116 | 1.4 | 2.6119 |
| 207.2041 | 1.4 | 2.6119 |
| 280.2479 | 1.2 | 2.2388 |
| 373.3606 | 1.2 | 2.2388 |

**Table 34**

| **598.5** | | |
|---|---|---|
| **CE: -40 V** | **16.9 min** | |
| ***m*/*z (amu)*** | ***intensity (counts)*** | ***% intensity*** |
| 597.5182 | 2.6667 | 100 |
| 579.5044 | 0.6667 | 25 |
| 279.2383 | 0.5833 | 21.875 |
| 298.2523 | 0.5833 | 21.875 |
| 316.2614 | 0.5833 | 21.875 |
| 280.2303 | 0.4167 | 15.625 |
| 281.2431 | 0.4167 | 15.625 |
| 314.255 | 0.4167 | 15.625 |
| 317.2837 | 0.4167 | 15.625 |
| 315.2474 | 0.3333 | 12.5 |
| 282.2576 | 0.25 | 9.375 |
| 297.2417 | 0.25 | 9.375 |
| 517.4654 | 0.25 | 9.375 |
| 171.0952 | 0.1667 | 6.25 |
| 295.2386 | 0.1667 | 6.25 |
| 296.291 | 0.1667 | 6.25 |
| 299.2386 | 0.1667 | 6.25 |
| 313.2243 | 0.1667 | 6.25 |
| 515.5116 | 0.1667 | 6.25 |
| 561.5262 | 0.1667 | 6.25 |

**Table 35: Accurate masses, putative molecular formulae and proposed structures for the thirty ovarian biomarkers detected in organic extracts of human serum.**

| | **Detected Mass** | **Exact Mass** | **Formula** | **Proposed Structure** |
|---|---|---|---|---|
| **1** | 446.3413 | 446.3396 | C₂₈H₄₆O₄ | 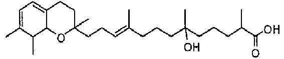 |
| **2** | 448.3565 | 448.3553 | C₂₈H₄₈O₄ | 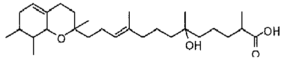 |
| **3** | 450.3735 | 450.3709 | C₂₈H₅₀O₄ | 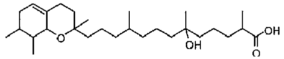 |
| **4** | 468.3848 | 468.3814 | C₂₈H₅₂O₅ | 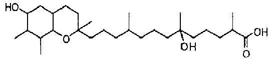 |
| **5** | 474.3872 | 474.3736 | C₃₀H₅₀O₄ | 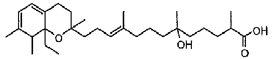 |
| **6** | 478.405 | 478.4022 | C₃₀H₅₄O₄ | 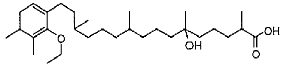 |
| **7** | 484.3793 | 484.3764 | C₂₈H₅₂O₆ | 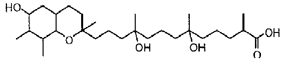 |
| **8** | 490.3678 | 490.3658 | C₃₀H₅₀O₅ | 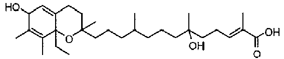 |
| **9** | 492.3841 | 492.3815 | C₃₀H₅₂O₅ | 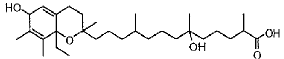 |
| **10** | 494.3973 | 494.3971 | C₃₀H₅₄O₅ | 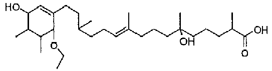 |
| **11** | 496.4157 | 496.4128 | C₃₀H₅₆O₅ | 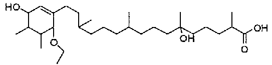 |
| **12** | 502.4055 | 502.4022 | C₃₂H₅₄O₄ | 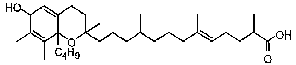 |
| **13** | 504.4195 | 504.4179 | C₃₂H₅₆O₄ | 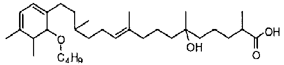 |
| **14** | 512.4083 | 512.4077 | C₃₀H₅₆O₆ | 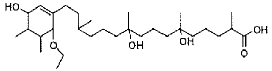 |
| **15** | 518.3974 | 518.3971 | C₃₂H₅₄O₅ | 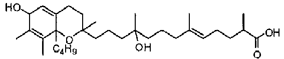 |
| **16** | 520.4131 | 520.4128 | C₃₂H₅₆O₅ | 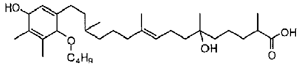 |
| **17** | 522.4323 | 522.8284 | C₃₂H₆₀O₅ | 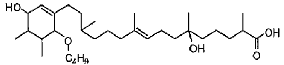 |
| **18** | 530.437 | 530.43351 | C₃₄H₅₈O₄ | 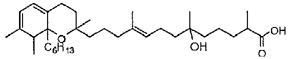 |
| **19** | 532.4507 | 532.44916 | C₃₄H₆₀O₄ | 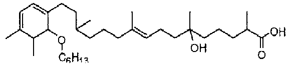 |
| **20** | 538.427 | 538.42334 | C₃₂H₅₈O₆ | 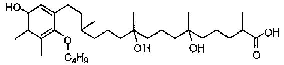 |
| **21** | 540.4393 | 540.4389 | C₃₂H₆₀O₆ | 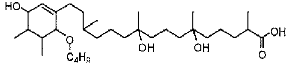 |
| **22** | 550.4609 | 550.4597 | C₃₄H₆₂O₅ | 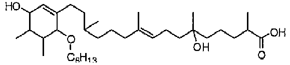 |
| **23** | 558.4653 | 558.4648 | C₃₆H₆₂O₄ | 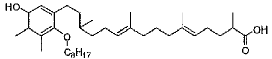 |
| **24** | 574.4597 | 574.4597 | C₃₆H₆₂O₅ | 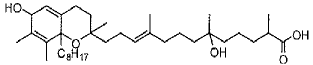 |
| **25** | 576.4757 | 576.4754 | C₃₆H₆₄O₅ | 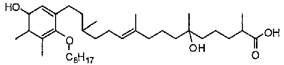 |
| **26** | 578.4848 | 578.4910 | C₃₆H₆₆O₅ | 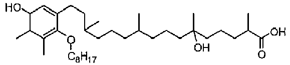 |
| **27** | 592.357 | 592.47029 | C₃₆H₆₄O₆ | 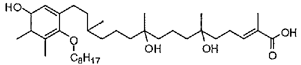 |
| **28** | 594.4848 | 594.4859 | C₃₆H₆₆O₆ | 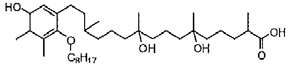 |
| **29** | 596.5012 | 596.5016 | C₃₆H₆₈O₆ | 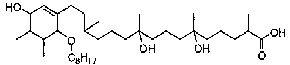 |
| **30** | 598.5121 | 598.5172 | C₃₆H₇₀O₆ | 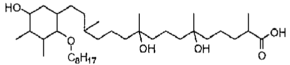 |

### Assignment of MS/MS fragments for Ovarian Cancer Biomarkers

**Table 36: MS/MS fragmentation of ovarian cancer biomarker 446.3544**

| **m/z** | **Formula** | **Molecular fragment** | **Fragment loss** |
|---|---|---|---|
| 445 | C₂₈H₄₅O₄ | 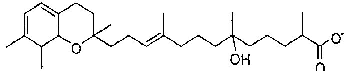 | -H⁺ |
| 427 | C₂₈H₄₃O₃ | 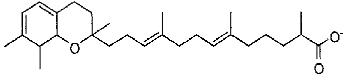 | -H₂O |
| 401 | C₂₇H₄₅O₂ | 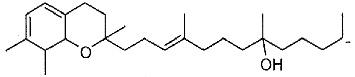 | -CO₂ |
| 383 | C₂₇H₄₃O | 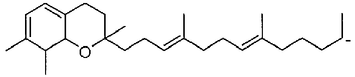 | -(CO₂ + H₂O) |
| 223 | C₁₄H₂₃O₂ | 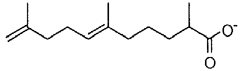 | 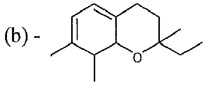 |
| 205 | C₁₄H₂₁O | 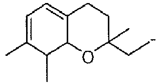 | 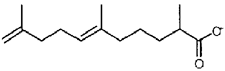 |
| 177 | C₁₂H₁₇O | 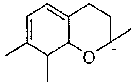 | (g) - C₂H₄ |
| 162 | C₁₁H₁₁₄O | 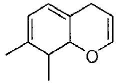 | |

**Table 37: MS/MS fragmentation of ovarian cancer biomarker 448.3715**

| **m/z** | **Formula** | **Molecular fragment** | **Fragment loss** |
|---|---|---|---|
| 447 | C₂₈H₄₇O₄ | 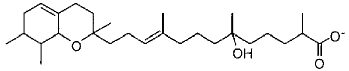 | -H⁺ |
| 429 | C₂₈H₄₅O₃ | 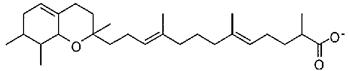 | -H₂O |
| 403 | C₂₇H₄₇O₂ | 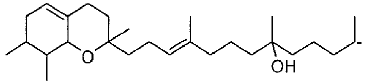 | -CO₂ |
| 385 | C₂₇H₄₅O | 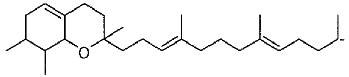 | - (CO₂₊ H₂O) |
| 279 | C₁₉H₃₅O | 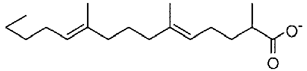 | Ring opening of 429 at O1-C2 and loss of 151 |
| 187 | C₁₀H₁₉O₃ | 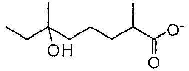 | |
| 151 | C₁₀H₁₅O | 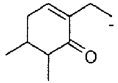 | Ring opening of 429 at O1-C2 and loss of 279 |
| 111 | C₈H₁₅ | 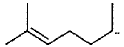 | |

**Table 38: MS/MS fragmentation of ovarian cancer biomarker 450.3804**

| **m/z** | **Formula** | **Molecular fragment** | **Fragment loss** | |
|---|---|---|---|---|
| 449 | C₂₈H₄₉O₄ | 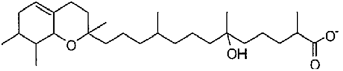 | -H⁺ | |
| 431 | C₂₈H₄₉O₄ | 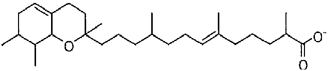 | -H₂O | |
| 413 | C₂₈H₄₅O₂ | 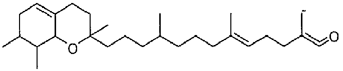 | -2 x H₂O | |
| 405 | C₂₇H₄₉O₂ | 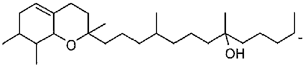 | -CO₂ | |
| 387 | C₂₇H₄₇O | 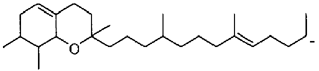 | -(CO₂₊H₂O) | |
| 309 | C₂₀H₃₇O₂ | 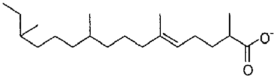 | Ring opening at O1 - C2 and, 431- 125 | |
| 281 | C₁₈H₃₃O₂ | 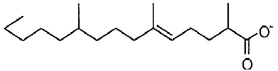 | - | 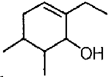 |
| 181 | C₁₁H₁₇O₂ | 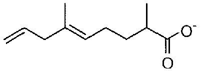 | 281- | 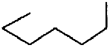 |
| 125 | C₈H₁₃O | 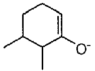 | 431 - 309 | |
| 111 | C₇H₁₁O | 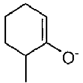 | 125 - CH₂ | |
| 97 | C₆H₉O | 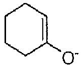 | 111 - CH₂ | |

**Table 39: MS/MS fragmentation of ovarian cancer biomarker 468.3986**

| **m/z** | **Formula** | **Molecular fragment** | **Fragment loss** | |
|---|---|---|---|---|
| 467 | C₂₈H₅₁O₅ | 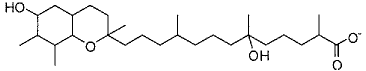 | -H⁺ | |
| 449 | C₂₈H₄₉O₄ | 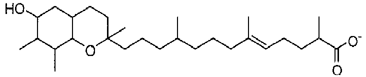 | -H₂O | |
| 431 | C₂₈H₄₇O₃ | 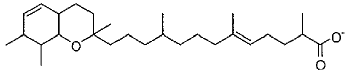 | -2 x H₂O | |
| 423 | C₂₇H₅₁O₂ | 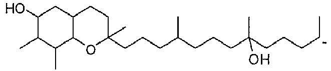 | -CO₂ | |
| 405 | C₂₇H₄₉O₂ | 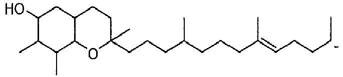 | -(CO₂ + H₂O) | |
| 297 | C₁₈H₃₃O₃ | 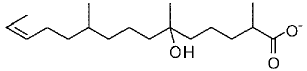 | Ring opening at O1 - C2 and, | |
| | | | - | 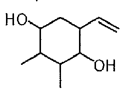 |
| 281 | C₁₈H₃₃O₂ | | Ring opening at O1 - C2 and, | |
| | | 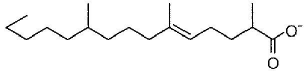 | - | 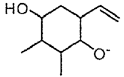 |
| 279 | C₁₈H₃₁O₂ | 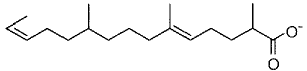 | 297 - H₂O | |
| 263 | C₁₈H₂₉O | 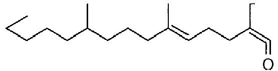 | 281 - H₂O | |
| 251 | C₁₆H₂₇O₂ | 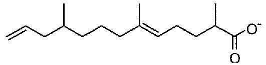 | 281 - C₂H₆ | |
| 169 | C₁₀H₁₇O₂ | 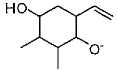 | Ring opening at O1 - C2 and, - 281 | |
| 141 | C₈H₁₃O₂ | 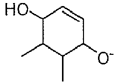 | 169 - C₂H₄ | |

**Table 40: MS/MS fragmentation of ovarian cancer biomarker 474.3736**

| **m**/**z** | **Formula** | **Molecular fragment** | **Fragment loss** |
|---|---|---|---|
| 473 | C₃₀H₄₉O₄ | 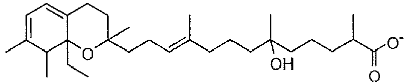 | -H⁺ |
| 455 | C₃₀H₄₇O₃ | 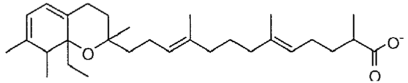 | -H₂O |
| 429 | C₂₉H₄₉O₂ | 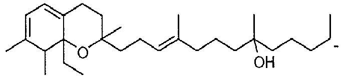 | -CO₂ |
| 411 | C₂₉H₄₇O | 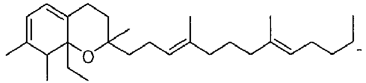 | -(CO₂₊ H₂O) |
| 223 | C₁₅H₂₇O | 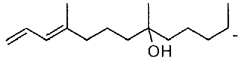 | 429 - |
| | | | 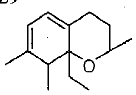 |
| 113 | C₆H₉O₂ | 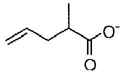 | |

**Table 41: MS/MS fragmentation of ovarian cancer biomarker 478.405**

| **m/z** | **Formula** | **Molecular fragment** | **Fragment loss** |
|---|---|---|---|
| 477 | C₃₀H₅₃O₄ | 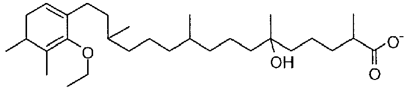 | -H⁺ |
| 460 | C₃₀H₅₁O₃ | 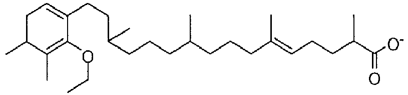 | -H₂O |
| 433 | C₂₉H₅₃O₂ | 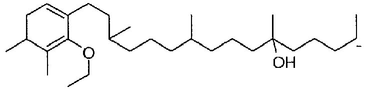 | -CO₂ |
| 415 | C₂₉H₅₁O | 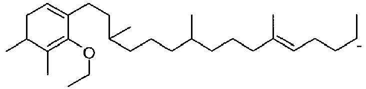 | -(CO₂₊ H₂O) |
| 281 | C₁₈H₃₃O₂ | 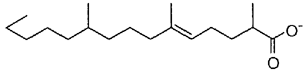 | 460- |
| | | | 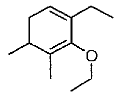 |

**Table 42: MS/MS fragmentation of ovarian cancer biomarker 484.3739**

| **m/z** | **Formula** | **Molecular fragment** | **Fragment loss** | |
|---|---|---|---|---|
| 483 | C₂₈H₅₁O₆ | 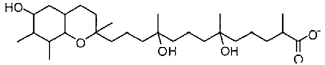 | -H⁺ | |
| 465 | C₂₈H₄₉O₅ | 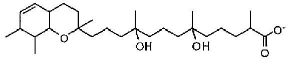 | -H₂O | |
| 447 | C₂₈H₄₇O₄ | 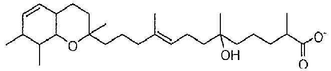 | -2H₂O | |
| 439 | C₂₇H₅₁O₄ | 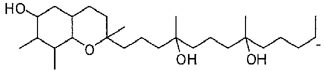 | -CO₂ | |
| 421 | C₂₇H₄₉O₃ | 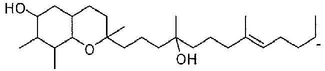 | -(CO₂ + 2H₂O) | |
| 315 | C₁₈H₃₅O₄ | 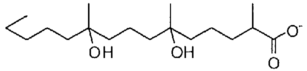 | Ring opening at O1 - C2 | |
| | | | and, - | 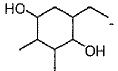 |
| 313 | C₁₈H₃₃O₄ | 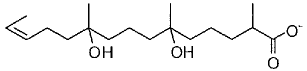 | Ring opening at O1 - C2 | |
| | | | and, - | 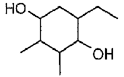 |
| 297 | C₁₈H₃₃O₃ | 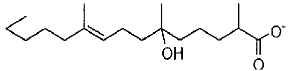 | 315 - H₂O | |
| 279 | C₁₈H₃₁O₂ | 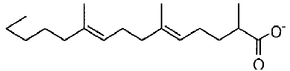 | 297 - H₂O | |
| 241 | C₁₄H₂₅O₃ | 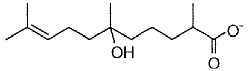 | 447 - | 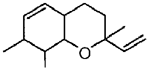 |
| 201 | C₁₁H₂₁O₃ | 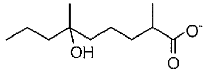 | 465 - | 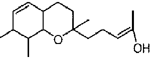 |
| 171 | C₁₀H₁₉O₂ | 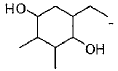 | Ring opening at O1 - C2 and, - 315 | |
| 101 | C₅H₉O₂ | 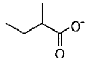 | | |

**Table 43: MS/MS fragmentation of ovarian cancer biomarker 490.3678**

| **m/z** | **Formula** | **Molecular fragment** | **Fragment loss** | |
|---|---|---|---|---|
| 489 | C₃₀H₄₉O₅ | 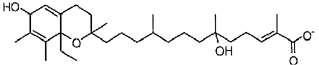 | -H⁺ | |
| 471 | C₃₀H₄₇O₄ | 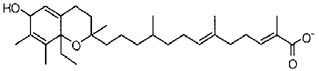 | -H₂O | |
| 445 | C₂₉H₄₉O₃ | 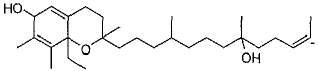 | -CO₂ | |
| 427 | C₂₉H₄₇O₂ | 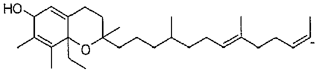 | -(CO₂ + 2H₂O) | |
| 373 | C₂₅H₄₁O₂ | 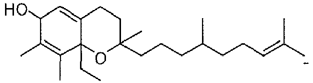 | 471 - | 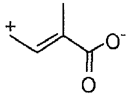 |
| 345 | C₂₃H₃₇O₂ | 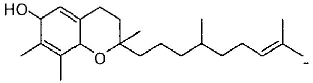 | 373 - C₂H₄ | |
| 319 | C₂₁H₃₅O₂ | 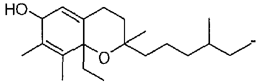 | 373 - C₄H₆ | |
| 267 | C₁₆H₂₇O₃ | 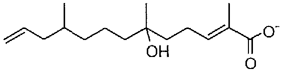 | - | 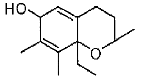 |
| 249 | C₁₆H₂₅O₂ | 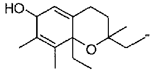 | | |
| 223 | C₁₄H₂₃O₂ | 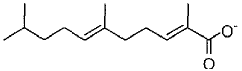 | | |

**Table 44: MS/MS fragmentation of ovarian cancer biomarker 492.3841**

| **m/z** | **Formula** | **Molecular fragment** | **Fragment loss** | |
|---|---|---|---|---|
| 491 | C₃₀H₅₁O₅ | 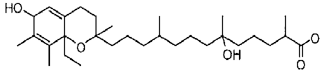 | -H⁺ | |
| 473 | C₃₀H₄₉O₄ | 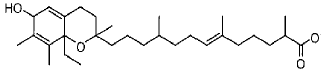 | -H₂O | |
| 445 | C₂₉H₅₁O₃ | 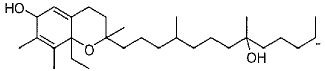 | -CO₂ | |
| 427 | C₂₉H₄₉O₂ | 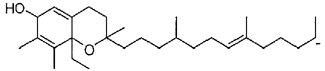 | -(CO₂ + 2H₂O) | |
| 319 | C₂₁H₃₅O₂ | 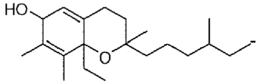 | - | 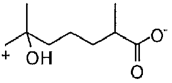 |
| 249 | C₁₆H₂₅O₂ | 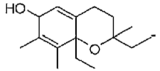 | | |
| 241 | C₁₄H₂₅O₃ | 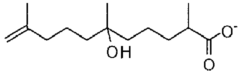 | | |
| 223 | C₁₄H₂₃O₂ | 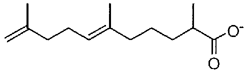 | 241 - H₂O | |
| 213 | C₁₅H₂₄O₂ | 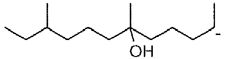 | 445 - | 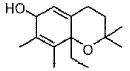 |

**Table 45: MS/MS fragmentation of ovarian cancer biomarker 494.3973**

| **m/z** | **Formula** | **Molecular fragment** | **Fragment loss** | |
|---|---|---|---|---|
| 493 | C₃₀H₅₃O₅ | 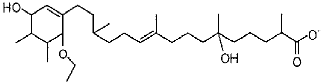 | -H⁺ | |
| 475 | C₃₀H₅₁O₃ | 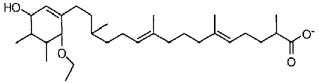 | -H₂O | |
| 449 | C₂₉H₅₃O₃ | 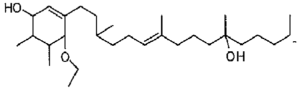 | -CO₂ | |
| 431 | C₂₉H₅₁O₂ | 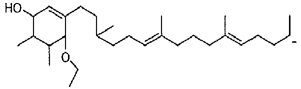 | -(CO₂ + H₂O) | |
| 415 | C₂₉H₅₁O | 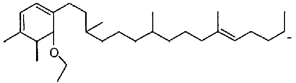 | -(CO₂ + 2H₂O) | |
| 307 | C₂₀H₃₅O₂ | 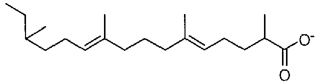 | 415 - | 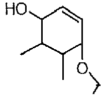 |
| 297 | C₁₈H₃₃O₃ | 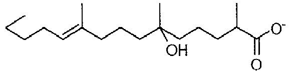 | - | 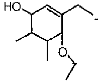 |
| 279 | C₁₈H₃₁O₂ | 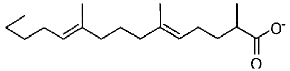 | 297 - H₂O | |
| 267 | C₁₆H₂₇O₃ | 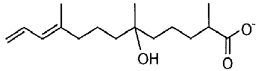 | - | 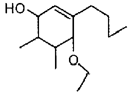 |
| 241 | C₁₄H₂₅O₃ | 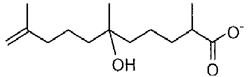 | 267 - C₂H₂ | |
| 235 | C₁₆H₂₇O | 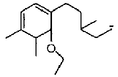 | 279- | 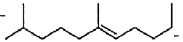 |
| 223 | C₁₄H₂₃O₂ | 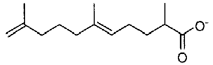 | 477 - | 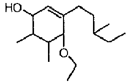 |
| 215 | C₁₂H₂₃O₂ | 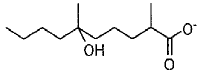 | Fragmentation at C13-C14 and loss of CH₃ | |
| 197 | C₁₂H₂₁O₂ | 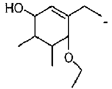 | - phytol chain | |
| 167 | C₁₀H₁₅O₂ | 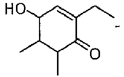 | 197 - C₂H₆ | |
| 151 | C₁₀H₁₅O | 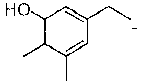 | 197 - C₂H₅OH | |
| 141 | C₉H₁₇O | 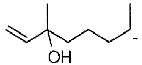 | 451 - | 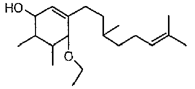 |
| 113 | C₆H₉O₂ | 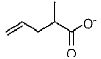 | | |

**Table 46: MS/MS fragmentation of ovarian cancer biomarker 496.4165**

| **m/z** | **Formula** | **Molecular fragment** | **Fragment loss** | |
|---|---|---|---|---|
| 495 | C₃₀H₅₅O₅ | 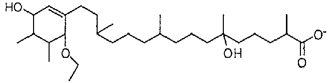 | -H⁺ | |
| 477 | C₃₀H₅₃O₃ | 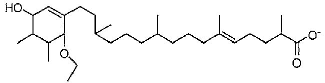 | -H₂O | |
| 451 | C₂₉H₅₅O₃ | 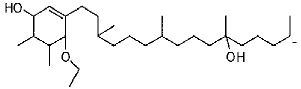 | -CO₂ | |
| 433 | C₂₉H₅₃O₂ | 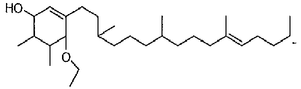 | -(CO₂ + H₂O) | |
| 297 | C₁₈H₃₃O₃ | 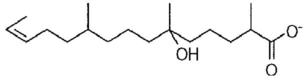 | - | 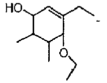 |
| 279 | C₁₈H₃₁O₂ | 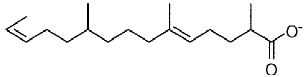 | 297 - H₂O | |
| 241 | C₁₄H₂₅O₃ | 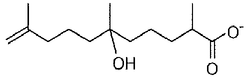 | - | 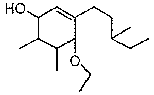 |
| 223 | C₁₄H₂₃O₂ | 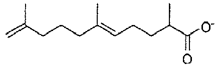 | 241 - H₂O | |
| 215 | C₁₂H₂₃O₂ | 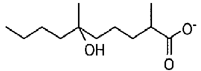 | Fragmentation at C13-C14 and loss of CH₃ | |
| 197 | C₁₂H₂₁O₂ | 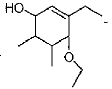 | - phytol chain | |
| 179 | C₁₂H₁₉O | 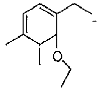 | 197 - H₂O | |
| 169 | C₁₀H₁₇O₂ | 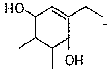 | 179 - C₂H₄ | |

**Table 47: MS/MS fragmentation of ovarian cancer biomarker 502.4055**

| **m/z** | **Formula** | **Molecular fragment** | **Fragment loss** |
|---|---|---|---|
| 501 | C₃₂H₅₃O₄ | 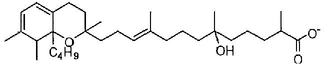 | -H⁺ |
| 483 | C₃₂H₅₁O₃ | 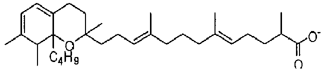 | - H₂O |
| 465 | C₃₂H₄₉O₂ | 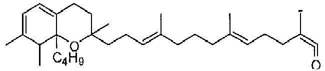 | -2xH₂O |
| 457 | C₃₁H₅₃O₂ | 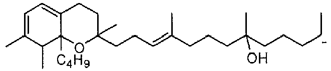 | - CO₂ |
| 439 | C₃₁H₅₁O | 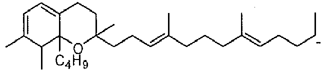 | -(CO₂ + H₂O) |
| 279 | C₁₈H₃₁O₂ | 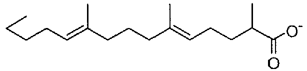 | Ring opening at O1-C2 of 483 and detachment of phytol chain |

**Table 48: MS/MS fragmentation of ovarian cancer biomarker 504.4195**

| **m/z** | **Formula** | **Molecular fragment** | **Fragment loss** | |
|---|---|---|---|---|
| 503 | C₃₂H₅₅O₄ | 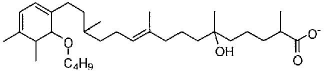 | -H⁺ | |
| 485 | C₃₂H₅₃O₃ | 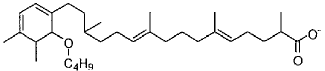 | -H₂O | |
| 467 | C₃₂H₅₁O₂ | 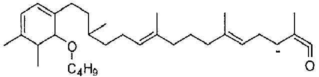 | -2xH₂O | |
| 459 | C₃₁H₅₅O₂ | 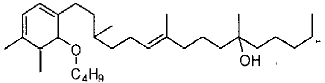 | -CO₂ | |
| 441 | C₃₁H₅₃O | 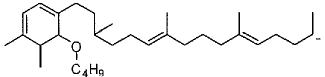 | -(CO₂+H₂O) | |
| 279 | C₁₈H₃₁O₂ | 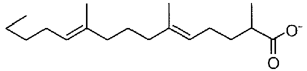 | - | 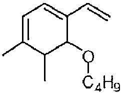 |
| 263 | C₁₇H₂₇O₂ | 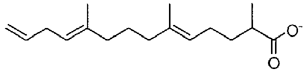 | 279 - CH₄ | |
| 223 | C₁₄H₂₃O₂ | 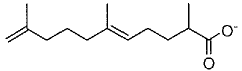 | 263 - C₃H₄ | |
| 169 | C₁₀H₁₇O₂ | 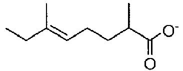 | 223 - C₄H₆ | |

**Table 49: MS/MS fragmentation of ovarian cancer biomarker 512.4083**

| **m/z** | **Formula** | **Molecular fragment** | **Fragment loss** | |
|---|---|---|---|---|
| 511 | C₃₀H₅₅O₆ | 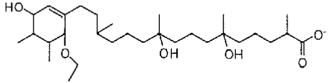 | -H⁺ | |
| 493 | C₃₀H₅₃O₅ | 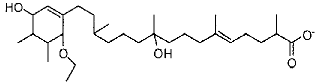 | -H₂O | |
| 467 | C₂₉H₅₅O₄ | 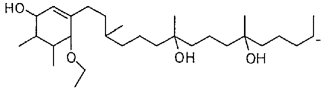 | -CO₂ | |
| 315 | C₁₈H₃₅O₄ | 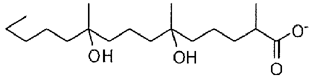 | - | 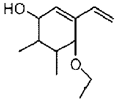 |
| 297 | C₁₈H₃₃O₃ | 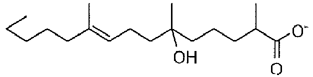 | 315 - H₂O | |
| 279 | C₁₈H₃₁O₂ | 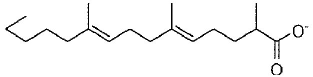 | 297 - H₂O | |
| 259 | C₁₄H₂₇O₄ | 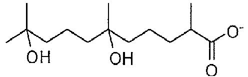 | 315 - C₄H₈ | |
| 251 | C₁₆H₂₇O₂ | 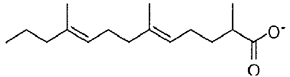 | 279 - C₂H₄ | |
| 151 | C₁₀H₁₅O | 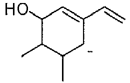 | 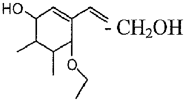 | |

**Table 50: MS/MS fragmentation of ovarian cancer biomarker 518.3974**

| **m/z** | **Formula** | **Molecular fragment** | **Fragment loss** | |
|---|---|---|---|---|
| 517 | C₃₂H₅₃O₅ | 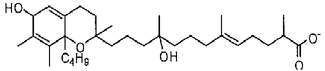 | -H⁺ | |
| 499 | C₃₂H₅₁O₄ | 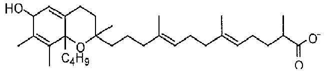 | -H₂O | |
| 481 | C₃₂H₄₉O₃ | 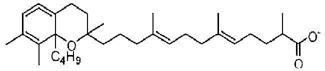 | -2xH₂O | |
| 473 | C₃₁H₅₃O₃ | 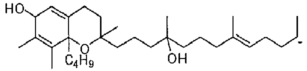 | -CO₂ | |
| 455 | C₃₁H₅₁O₂ | 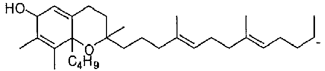 | -(CO₂ + H₂O) | |
| 445 | C₂₉H₄₉O₃ | 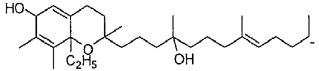 | 473 - C₂H₄ | |
| 437 | C₃₁H₄₉O | 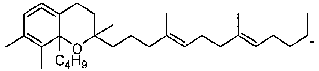 | 455 - H₂O | |
| 389 | C₂₅H₄₁O₃ | 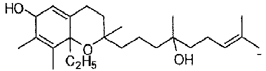 | 445 - C₄H₈ | |
| 279 | C₁₈H₃₁O₂ | 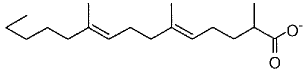 | Ring opening at O1-C2 and, | |
| | | | 499 - | 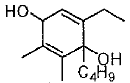 |
| 223 | C₁₄H₃₂O₂ | 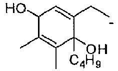 | Ring opening at O1-C2 and detachment of the phytol chain | |

**Table 51: MS/MS fragmentation of ovarian cancer biomarker 520.4131**

| **m/z** | **Formula** | **Molecular fragment** | **Fragment loss** | |
|---|---|---|---|---|
| 519 | C₃₂H₅₅O₅ | 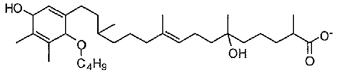 | -H⁺ | |
| 501 | C₃₂H₅₃O₄ | 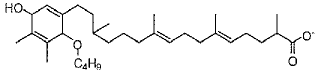 | -H₂O | |
| 483 | C₃₂H₅₁O₃ | 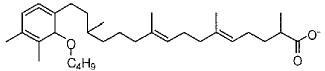 | -2xH₂O | |
| 475 | C₃₁H₅₅O₃ | 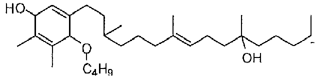 | -CO₂ | |
| 459 | C₃₀H₅₁O₃ | 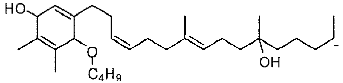 | 475 - CH₄ | |
| 457 | C₃₁H₅₃O₂ | 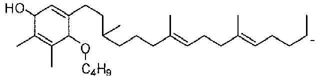 | -(CO₂ + H₂O) | |
| 447 | C₂₈H₄₇O₄ | 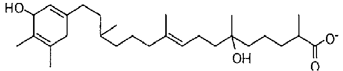 | -C₄H₈O | |
| 297 | C₁₈H₃₃O₃ | 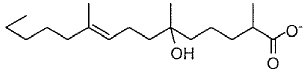 | - | 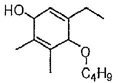 |
| 279 | C₁₈H₃₁O₂ | 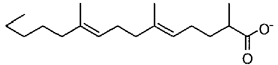 | 297 - H₂O | |
| 241 | C₁₄H₂₅O₃ | 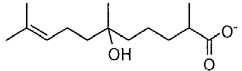 | 297 - C₄H₈ | |
| 223 | C₁₄H₂₃O₂ | 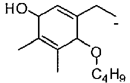 | Ring opening at O1-C2 and detachment of the phytol chain | |
| 195 | C₁₂H₁₉O₄ | 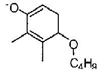 | 223 - C₂H₄ | |
| 115 | C₆H₁₁O₂ | 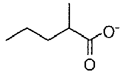 | | |

**Table 52: MS/MS fragmentation of ovarian cancer biomarker 522.4323**

| **m/z** | **Formula** | **Molecular fragment** | **Fragment loss** | |
|---|---|---|---|---|
| 521 | C₃₂H₅₇O₅ | 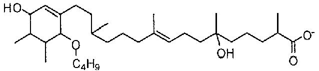 | -H⁺ | |
| 503 | C₃₂H₅₅O₅ | 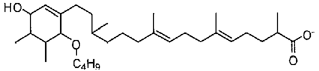 | -H₂O | |
| 485 | C₃₂H₅₅O₅ | 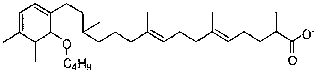 | -2xH₂O | |
| 477 | C₃₁H₅₇O₃ | 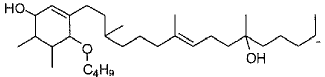 | -CO₂ | |
| 459 | C₃₁H₅₅O₂ | 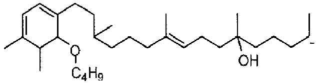 | -(CO₂ + H₂O) | |
| 441 | C₃₁H₅₃O | 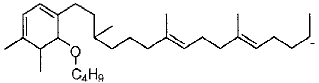 | -(CO₂ + 2H₂O) | |
| 297 | C₁₈H₃₃O₃ | 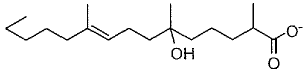 | - | 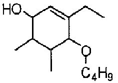 |
| 279 | C₁₈H₃₁O₂ | 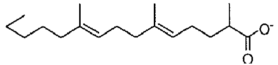 | 297 - H₂O | |
| 269 | C₁₆H₂₉O₃ | 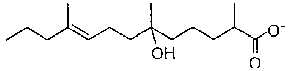 | 297 - C₂H₄ | |
| 241 | C₁₄H₂₅O₃ | 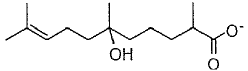 | 269 - C₂H₄ | |
| 115 | C₆H₁₁O₂ | 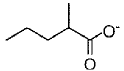 | | |

**Table 53: MS/MS fragmentation of ovarian cancer biomarker 530.437**

| **m/z** | **Formula** | **Molecular fragment** | **Fragment loss** | |
|---|---|---|---|---|
| 529 | C₃₄H₅₇O₄ | 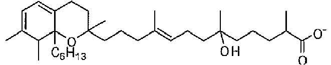 | -H⁺ | |
| 511 | C₃₄H₅₅O₃ | 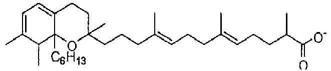 | -H₂O | |
| 485 | C₃₃H₅₇O₂ | 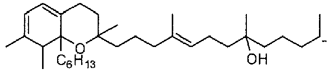 | -CO₂ | |
| 467 | C₃₃H₅₅O | 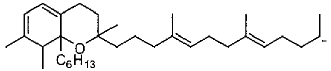 | -(CO₂ + H₂O) | |
| 251 | C₁₆H₂₇O₂ | 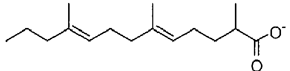 | 511 - | 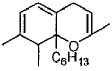 |
| 205 | C₁₅H₂₅ | 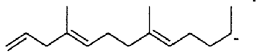 | 467 - | 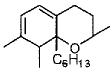 |

**Table 54: MS/MS fragmentation of ovarian cancer biomarker 532.4507**

| **m/z** | **Formula** | **Molecular fragment** | **Fragment loss** | |
|---|---|---|---|---|
| 531 | C₃₄H₅₉O₄ | 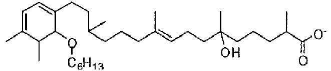 | -H⁺ | |
| 513 | C₃₄H₅₇O₃ | 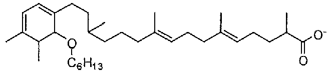 | -H₂O | |
| 495 | C₃₄H₅₅O₂ | 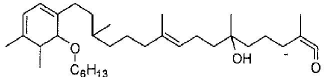 | -2H₂O | |
| 485 | C₃₃H₅₉O₂ | 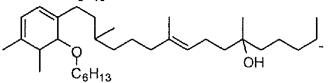 | -CO₂ | |
| 469 | C₃₃H₅₇O | 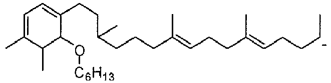 | -(CO₂ + H₂O) | |
| 251 | C₁₆H₂₇O₂ | 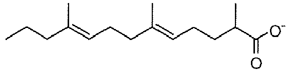 | 513 - | 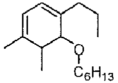 |
| 181 | C₁₂H₂₁O | 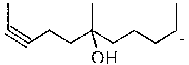 | | |

**Table 55: MS/MS fragmentation of ovarian cancer biomarker 538.427**

| **m/z** | **Formula** | **Molecular fragment** | **Fragment loss** | |
|---|---|---|---|---|
| 538 | C₃₂H₅₇O₆ | 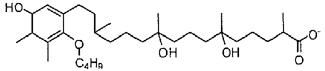 | -H⁺ | |
| 519 | C₃₂H₅₅O₅ | 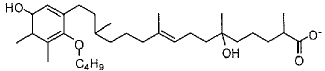 | -H₂O | |
| 501 | C₃₂H₅₃O₄ | 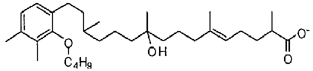 | -2H₂O | |
| 493 | C₃₁H₅₇O₄ | 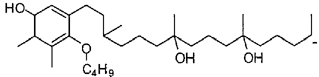 | -CO₂ | |
| 475 | C₃₁H₅₅O₃ | 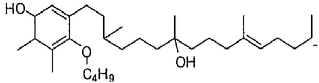 | -(CO₂ + H₂O) | |
| 457 | C₃₁H₅₃O₂ | 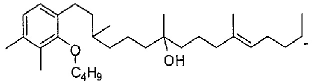 | -(CO₂ + 2H₂O) | |
| 333 | C₂₂H₃₇O₂ | 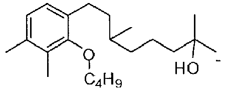 | 457 - C₉H1₆ | |
| 315 | C₁₈H₁₅O₄ | 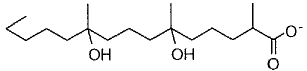 | - | 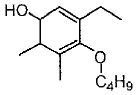 |

**Table 56: MS/MS fragmentation of ovarian cancer biomarker 540.4390**

| **m**/**z** | **Formula** | **Molecular fragment** | **Fragment loss** | |
|---|---|---|---|---|
| 539 | C₃₂H₅₉O₆ | 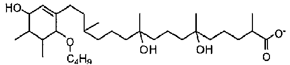 | -H⁺ | |
| 521 | C₃₂H₅₇O₅ | 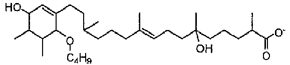 | -H₂O | |
| 495 | C₃₁H₅₉O₄ | 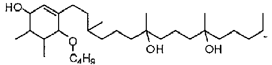 | -CO₂ | |
| 477 | C₃₁H₅₇O₃ | 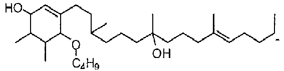 | -(CO₂ + H₂O) | |
| 315 | C₁₈H₃₅O₄ | 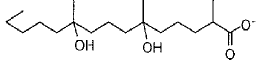 | 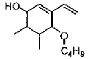 | |
| 313 | C₁₈H₃₃O₄ | 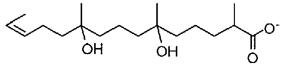 | 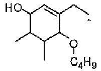 | |
| 297 | C₁₈H₃₃O₃ | 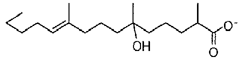 | 315 - H₂O | |
| 279 | C₁₈H₃₁O₂ | 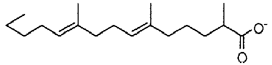 | 297 - H₂O | |
| 259 | C₁₄H₂₇O₄ | 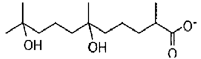 | - | 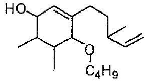 |
| 243 | C₁₄H₂₇O₃ | 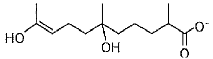 | 259 - CH₄ | |
| 241 | C₁₅H₂₉O₂ | 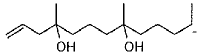 | 495 - 253 | |
| 225 | C₁₄H₂₅O₂ | 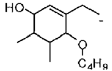 | - phytol chain | |
| 223 | C₁₄H₂₃O₂ | 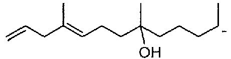 | 241 - H₂O | |
| 179 | C₁₂H₁₉O | 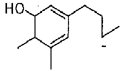 | 253 - C₄H₉OH | |
| 171 | C₁₀H₁₉O₂ | 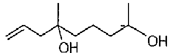 | 213 - C₃H₆ | |

**Table 57: MS/MS fragmentation of ovarian cancer biomarker 550.4609**

| **m/z** | **Formula** | **Molecular fragment** | **Fragment loss** | |
|---|---|---|---|---|
| 549 | C₃₄H₆₁O₅ | 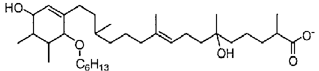 | -H⁺ | |
| 531 | C₃₄H₅₉O₄ | 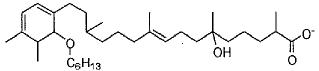 | -H₂O | |
| 513 | C₃₄H₅₇O₃ | 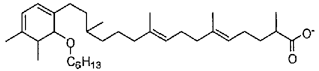 | -2H₂O | |
| 505 | C₃₃H₆₁O₃ | 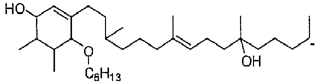 | -CO₂ | |
| 487 | C₃₃H₅₉O₂ | 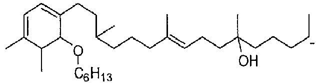 | -(CO₂ + H₂O) | |
| 469 | C₃₃H₅₇O | 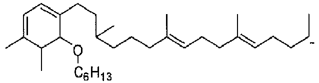 | -(CO₂ + 2H₂O) | |
| 297 | C₁₈H₃₃O₃ | 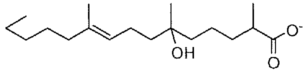 | - | 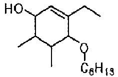 |
| 279 | C₁₈H₃₁O₂ | 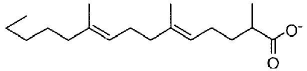 | 297 - H₂O | |
| 269 | C₁₆H₂₉O₃ | 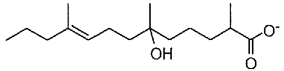 | - | 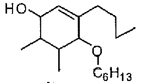 |
| 253 | C₁₆H₂₉O₂ | 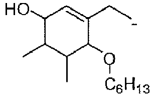 | - phytol chain | |
| 125 | C₉H₁₇ | 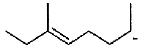 | | |

**Table 58: MS/MS fragmentation of ovarian cancer biomarker 558.4653**

| **m/z** | **Formula** | **Molecular fragment** | **Fragment loss** | |
|---|---|---|---|---|
| 557 | C₃₆H₆₁O₄ | 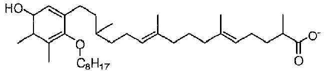 | -H⁺ | |
| 539 | C₃₆H₅₉O₄ | 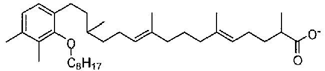 | -H₂O | |
| 513 | C₃₅H₆₁O₂ | 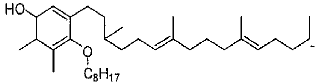 | -CO₂ | |
| 495 | C₃₅H₅₉O | 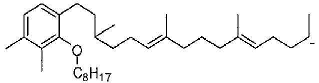 | -(CO₂ + H₂O) | |
| 279 | C₁₈H₃₁O2 | 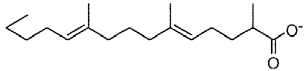 | - | 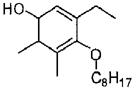 |
| 279 | C₁₈H₃₁O₂ | 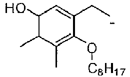 | - phytol chain | |
| 155 | C₉H₁₅O₂ | 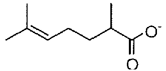 | | |

**Table 59: MS/MS fragmentation of ovarian cancer biomarker 574.4638**

| **m/z** | **Formula** | **Molecular fragment** | **Fragment loss** | |
|---|---|---|---|---|
| 573 | C₃₆H₆₁O₅ | 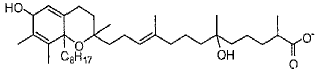 | -H⁺ | |
| 555 | C₃₆H₅₉O₄ | 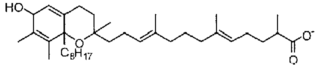 | -H₂O | |
| 537 | C₃₆H₅₇O₃ | 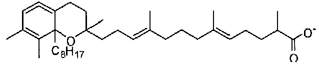 | -2H₂O | |
| 529 | C₃₅H₆₁O₃ | 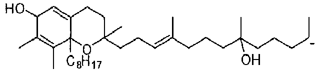 | -CO₂ | |
| 511 | C₃₅H₅₉O₂ | 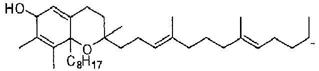 | -(CO₂ + H₂O) | |
| 493 | C₃₅H₅₇O | 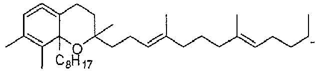 | -(CO₂ + 2H₂O) | |
| 401 | C₂₇H₄₅O₂ | 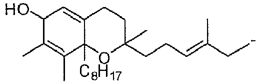 | 511 - C₈H₁₄ | |
| 295 | C₁₈H₃₁O₃ | 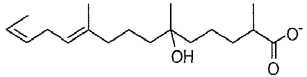 | Ring opening at O1-C2 | |
| | | | and - | 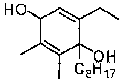 |
| 279 | C₁₈H₃₁O₂ | 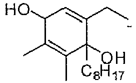 | Ring opening at O1-C2 and loss of phytol chain | |
| 279 | C₁₈H₃₁O₂ | | Ring opening of 555 at O1-C2 and - | |
| | | 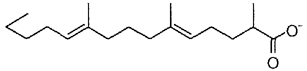 | | 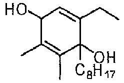 |
| 223 | C₁₄H₂₃O₂ | 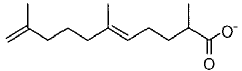 | 279 - C₄H₈ | |

**Table 60: MS/MS fragmentation of ovarian cancer biomarker 576.4762 (C₃₆H₆₄O₅)**

| **m/z** | **Formula** | **Molecular fragment** | **Fragment loss** | |
|---|---|---|---|---|
| 575 | C₃₆H₆₃O₅ | 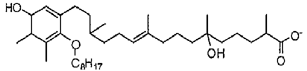 | -H⁺ | |
| 557 | C₃₆H₆₁O₄ | 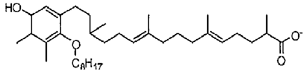 | -H₂O | |
| 539 | C₃₆H₅₉O₃ | 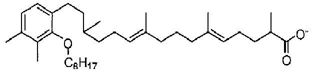 | -2xH₂O | |
| 531 | C₃₅H₆₃O₃ | 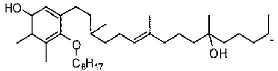 | - CO₂ | |
| 513 | C₃₅H₆₁O₂ | 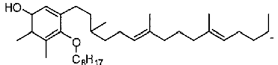 | 557 - CO₂ | |
| 495 | C₃₅H₅₉O | 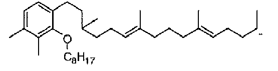 | 531 - CO₂ | |
| 403 | C₂₈H₄₇O₂ | 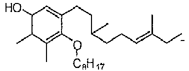 | 495 - C₇H₁₂ | |
| 297 | C₁₈H₃₃O₃ | 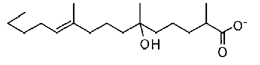 | - | 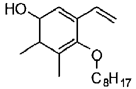 |
| 279 | C₁₈H₃₃O₂ | 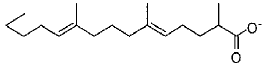 | - | 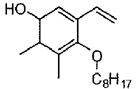 |
| 279 | C₁₈H₃₁O₂ | 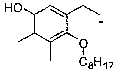 | - phytol chain | |
| 251 | C₁₆H₂₇O₂ | 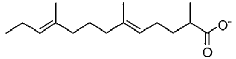 | 557 - | 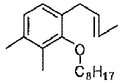 |
| 183 | C₁₁H₁₉O₂ | 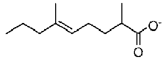 | 557- | 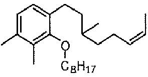 |

**Table 61: MS/MS fragmentation of ovarian cancer biomarker 578.493**

| **m**/**z** | **Formula** | **Molecular fragment** | **Fragment loss** | |
|---|---|---|---|---|
| 577 | C₃₆H₆₅O₅ | 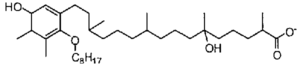 | -H⁺ | |
| 559 | C₃₆H₆₃O₄ | 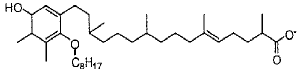 | -H₂O | |
| 541 | C₃₆H₆₁O₃ | 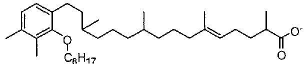 | -2xH₂O | |
| 533 | C₃₅H₆₅O₃ | 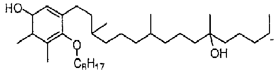 | - CO₂ | |
| 515 | C₃₅H₆₃O₂ | 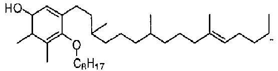 | 559 - CO₂ | |
| 497 | C₃₅H₆₁O | 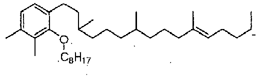 | 533 - CO₂ | |
| 373 | C₂₆H₄₅O | 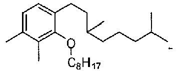 | 541 - C₁₀H₁₆O₂ | |
| 297 | C₁₈H₃₃O₃ | 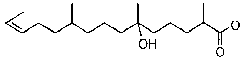 | - | 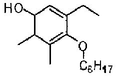 |
| 281 | C₁₈H₃₃O₂ | 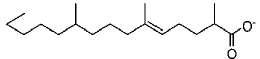 | | |
| 279 | C₁₈H₃₁O₂ | 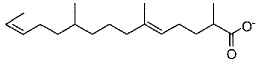 | 297 - H₂O | |
| 279 | C₁₈H₃₁O₂ | 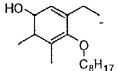 | - phytol chain | |

**Table 62: MS/MS fragmentation of ovarian cancer biomarker 592.4728**

| **m/z** | **Formula** | **Molecular fragment** | **Fragment loss** | |
|---|---|---|---|---|
| 591 | C₃₆H₆₃O₆ | 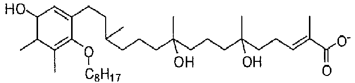 | -H⁺ | |
| 573 | C₃₆H₆₃O₆ | 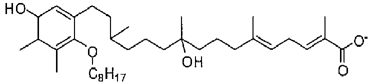 | -H₂O | |
| 529 | C₃₆H₆₃O₆ | 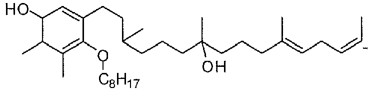 | -(CO₂ + H₂O) | |
| 313 | C₁₈H₃₃O₄ | 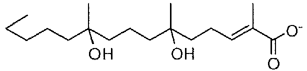 | - | 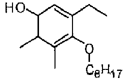 |
| 295 | C₁₈H₃₁O₃ | 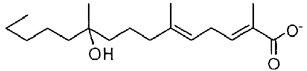 | 313 -H₂O | |

**Table 63: MS/MS fragmentation of ovarian cancer biomarker 594.4857**

| **m/z** | **Formula** | **Molecular fragment** | **Fragment loss** | |
|---|---|---|---|---|
| 593 | C₃₆H₆₅O₆ | 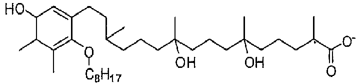 | -H⁺ | |
| 575 | C₃₆H₆₅O₅ | 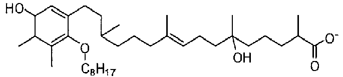 | -H₂O | |
| 557 | C₃₆H₆₃O₄ | 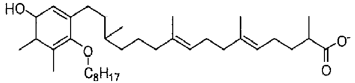 | -2xH₂O | |
| 549 | C₃₅H₆₅O₄ | 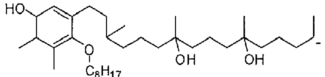 | -CO₂ | |
| 513 | C₃₅H₆₃O₂ | 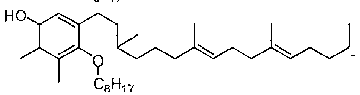 | 549 - CO₂ | |
| 495 | C₃₅H₆₁O | 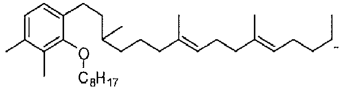 | 513 - H₂O | |
| 315 | C₁₈H₃₅O₄ | 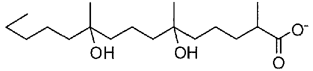 | - | 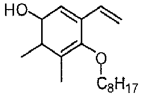 |
| 297 | C₁₈H₃₃O₃ | 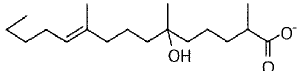 | 315 - H₂O | |
| 279 | C₁₈H₃₁O₂ | 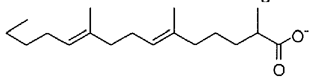 | 421 - H₂O | |
| 279 | C₁₈H₃₁O₂ | 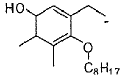 | - phytol chain | |
| 201 | C₁₂H₂₅O₂ | 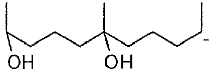 | 421 - | 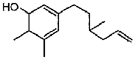 |
| 171 | C₉H₁₅O₃ | 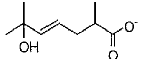 | | |
| 141 | C₈H₁₃O₂ | 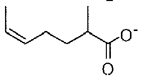 | | |

**Table 64: MS/MS fragmentation of ovarian cancer biomarker 596.5015**

| **m/z** | **Formula** | **Molecular fragment** | **Fragment loss** | |
|---|---|---|---|---|
| 595 | C₃₆H₆₇O₆ | 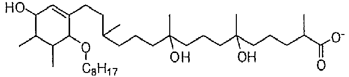 | -H⁺ | |
| 577 | C₃₆H₆₅O₅ | 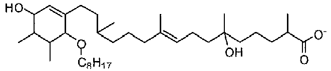 | -H₂O | |
| 559 | C₃₆H₆₃O₄ | 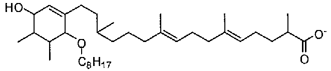 | -2xH₂O | |
| 551 | C₃₅H₆₇O₂ | 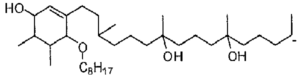 | -CO₂ | |
| 515 | C₃₅H₆₃O₂ | 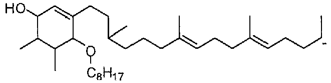 | 559 - CO₂ | |
| 315 | C₁₈H₃₅O₄ | 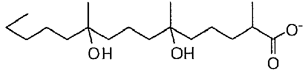 | - | 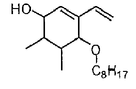 |
| 297 | C₁₈H₃₃O₃ | 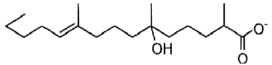 | 315 - H₂O | |
| 281 | C₁₈H₃₂O₂ | 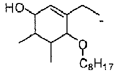 | - phytol chain | |
| 279 | C₁₈H₃₁O₂ | 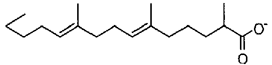 | 297 - H₂O | |
| 171 | C₉H₁₅O₃ | 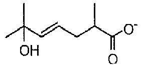 | | |
| 155 | C₉H₁₅O₂ | 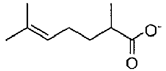 | | |
| 141 | C₉H₁₇O | 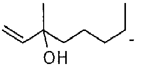 | | |
| 127 | C₈H₁₅O | 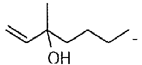 | | |

**Table 65: MS/MS fragmentation of ovarian cancer biomarker 598.5121**

| **m/z** | **Formula** | **Molecular fragment** | **Fragment loss** | |
|---|---|---|---|---|
| 597 | C₃₆H₆₉O₆ | 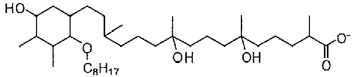 | | |
| 579 | C₃₆H₆₇O₅ | 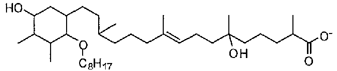 | -H₂O | |
| 561 | C₃₆H₆₅O₄ | 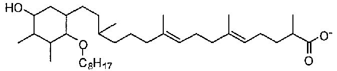 | -2xH₂O | |
| 517 | C₃₅H₆₅O₂ | 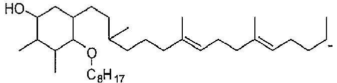 | 561 - CO₂ | |
| 315 | C₁₈H₃₅O₄ | 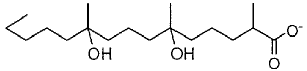 | - | 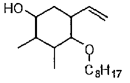 |
| 297 | C₁₈H₃₃O₃ | 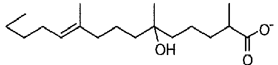 | 315 - H₂O | |
| 279 | C₁₈H₃₁O₂ | 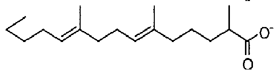 | 297 - H₂O | |

**Table 66. P-values between control and ovarian cancer cohorts for each of the C28 markers.**

| **Mass** | **450** | **446** | **468** | **466** | **448** | **464** |
|---|---|---|---|---|---|---|
| **p-value** | 1.92E-12 | 7.66E-17 | 1.35E-11 | 8.17E-13 | 1.57E-12 | 3.03E-12 |

**Table 67. List of gamma Tocoenoic acids included in expanded triple-quadrupole HTS method..**

| **[M-H]parent/[M-H]daughter** | **formula** | **pvalue (Ovarian vs control)** |
|---|---|---|
| 467.4 / 423.4 | C28H4604 | 1.4E-06 |
| 447.4 / 385.4 | C28H48O4 | 5.7E-13 |
| 501.4 / 457.4 | C28H50O4 | 4.1E-15 |
| 451.4 / 407.4 | C28H48O5 | 2.9E-04 |
| 531.5 / 469.4 | C28H50O5 | 3.7E-10 |
| 529.4 / 467.4 | C28H52O5 | 6.2E-09 |
| 449.4 / 405.4 | C28H52O6 | 5.3E-08 |
| 445.3 / 383.4 | C30H50O4 | 1.2E-09 |
| 477.4 / 433.4 | C30H50O5 | 6.2E-13 |
| 473.4 / 429.4 | C30H52O4 | 3.4E-11 |
| 493.5 / 449.4 | C30H52O5 | 7.3E-10 |
| 535.4 / 473.4 | C30H5404 | 2.8E-03 |
| 465.4 / 403.4 | C30H5405 | 8.4E-11 |
| 463.4 / 419.4 | C30H56O6 | 8.6E-11 |
| 517.4 / 473.4 | C32H54O4 | 4.9E-11 |
| 503.4 / 459.4 | C32H54O5 | 9.6E-15 |
| 523.4 / 461.4 | C32H56O4 | 1.6E-04 |
| 519.4 / 475.4 | C32H56O5 | 7.8E-08 |
| 575.5 / 513.5 | C32H56O6 | 3.4E-09 |
| 521.4 / 477.4 | C32H58O5 | 1.5E-08 |
| 483.4 / 315.3 | C32H58O6 | 4.5E-21 |
| 511.4 / 315.3 | C32H60O5 | 6.9E-16 |
| 549.5 / 487.5 | C32H60O6 | 1.1E-07 |
| 491.4 / 241.2 | C34H58O4 | 3.9E-13 |
| 539.4 / 315.3 | C34H6004 | 8.0E-03 |
| 591.5 / 555.4 | C34H6205 | 2.7E-11 |
| 579.5 / 517.5 | C36H62O5 | 3.2E-02 |
| 589.5 / 545.5 | C36H62O6 | 1.7E-14 |
| 537.4 / 475.4 | C36H64O5 | 1.1E-03 |
| 489.4 / 445.4 | C36H64O6 | 1.7E-15 |
| 573.5 / 223.1 | C36H68O5 | 9.2E-16 |

## Claims

1. A method for diagnosing ovarian cancer or the risk of developing ovarian cancer in a test subject of unknown ovarian cancer status, comprising the steps of:
analyzing a blood sample from a test subject to obtain quantifying data on molecules selected from metabolites identified by the exact masses 474.3736, 478.4022, 484.3764, 490.3658, 492.3815, 494.3971, 496.4128, 502.4022, 504.4179, 512.4077, 518.3971, 520.4128, 522.8284, 530.43351, 532.44916, 540.4389, 550.4597, 558.4648, 574.4597, 576.4754, 578.4910, 596.5016, and 598.5172 where a ± 5 ppm difference would indicate the same metabolite;
wherein the molecular formulas of the metabolites with these masses are C₃₀H₅₀O₄, C₃₀H₅₄O₄, C₂₈H₅₂O₆, C₃₀H₅₀O₅, C₃₀H₅₂O₅, C₃₀H₅₄O₅, C₃₀H₅₆O₅, C₃₂H₅₄O₄, C₃₂H₅₆O₄, C₃₀H₅₆O_{6,} C₃₂H₅₄O₅, C₃₂H₅₆O₅, C₃₂H₆₀O₅, C₃₄H₅₈O₄, C₃₄H₆₀O₄, C₃₂H₆₀O₆, C₃₄H₆₂O₅, C₃₆H₆₂O₄, C₃₆H₆₂O₅, C₃₆H₆₄O₅, C₃₆H₆₆O₅, C₃₆H₆₈O₆, and C₃₆H₇₀O₆, respectively;
wherein the metabolites have LC-MS/MS fragment patterns analyzed under negative ionization as follows:
the metabolite having the molecular formula C₃₀H₅₀O₄ has a LC-MS/MS fragment pattern comprising daughter ions with m/z of: 473, 455, 429, 411, 223, 113;
the metabolite having the molecular formula C₃₀H₅₄O₄ has a LC-MS/MS fragment pattern comprising daughter ions with m/z of: 477, 460, 433, 415, 281;
the metabolite having the molecular formula C₂₈H₅₂O₆ has a LC-MS/MS fragment pattern comprising daughter ions with m/z of: 483, 465, 447, 439, 421, 315, 313, 297, 279, 241, 201, 171, 101;
the metabolite having the molecular formula C₃₀H₅₀O₅ has a LC-MS/MS fragment pattern comprising daughter ions with m/z of: 489, 471, 445, 427, 373, 345, 319, 267, 249, 223;
the metabolite having the molecular formula C₃₀H₅₂O₅ has a LC-MS/MS fragment pattern comprising daughter ions with m/z of: 491, 473, 445, 427, 319, 249, 241, 223, 213;
the metabolite having the molecular formula C₃₀H₅₄O₅ has a LC-MS/MS fragment pattern comprising daughter ions with m/z of: 493, 475, 449, 431, 415, 307, 297, 279, 267, 241, 235, 223, 215, 197, 167, 151, 141, 113;
the metabolite having the molecular formula C₃₀H₅₆O₅ has a LC-MS/MS fragment pattern comprising daughter ions with m/z of: 495, 477, 451, 433, 297, 279, 241, 223, 215, 197, 179, 169;
the metabolite having the molecular formula C₃₂H₅₄O₄ has a LC-MS/MS fragment pattern comprising daughter ions with m/z of: 501, 483, 465, 457, 439, 279;
the metabolite having the molecular formula C₃₂H₅₆O₄ has a LC-MS/MS fragment pattern comprising daughter ions with m/z of: 503, 485, 467, 459, 441, 279, 263, 223, 169;
the metabolite having the molecular formula C₃₀H₅₆O₆ has a LC-MS/MS fragment pattern comprising daughter ions with m/z of: 511, 493, 467, 315, 297, 279, 259, 251, 151;
the metabolite having the molecular formula C₃₂H₅₄O₅ has a LC-MS/MS fragment pattern comprising daughter ions with m/z of: 517, 499, 481, 473, 455, 445, 437, 389, 279, 223;
the metabolite having the molecular formula C₃₂H₅₆O₅ has a LC-MS/MS fragment pattern comprising daughter ions with m/z of: 519, 501, 483, 475, 459, 457, 447, 297, 279, 241, 223, 195, 115;
the metabolite having the molecular formula C₃₂H₆₀O₅ has a LC-MS/MS fragment pattern comprising daughter ions with m/z of: 521, 503, 485, 477, 459, 441, 297, 279, 269, 241, 115;
the metabolite having the molecular formula C₃₄H₅₈O₄ has a LC-MS/MS fragment pattern comprising daughter ions with m/z of: 529, 511, 485, 467, 251, 205;
the metabolite having the molecular formula C₃₄H₆₀O₄ has a LC-MS/MS fragment pattern comprising daughter ions with m/z of: 531, 513, 495, 485, 469, 251, 181;
the metabolite having the molecular formula C₃₂H₆₀O₆ has a LC-MS/MS fragment pattern comprising daughter ions with m/z of: 539, 521, 495, 477, 315, 313, 297, 279, 259, 243, 241, 225, 223, 179, 171;
the metabolite having the molecular formula C₃₄H₆₂O₅ has a LC-MS/MS fragment pattern comprising daughter ions with m/z of: 549, 531, 513, 505, 487, 469, 297, 279, 269, 253, 125;
the metabolite having the molecular formula C₃₆H₆₂O₄ has a LC-MS/MS fragment pattern comprising daughter ions with m/z of: 557, 539, 513, 495, 279, 155;
the metabolite having the molecular formula C₃₆H₆₂O₅ has a LC-MS/MS fragment pattern comprising daughter ions with m/z of: 573, 555, 537, 529, 511, 493, 401, 295, 279, 223;
the metabolite having the molecular formula C₃₆H₆₄O₅ has a LC-MS/MS fragment pattern comprising daughter ions with m/z of: 575, 557, 539, 531, 513, 495, 403, 297, 279, 251, 183;
the metabolite having the molecular formula C₃₆H₆₆O₅ has a LC-MS/MS fragment pattern comprising daughter ions with m/z of: 577, 559, 541, 533, 515, 497, 373, 297, 281, 279;
the metabolite having the molecular formula C₃₆H₆₈O₆ has a LC-MS/MS fragment pattern comprising daughter ions with m/z of: 595, 577, 559, 551, 515, 315, 297, 281, 279, 171, 155, 141, 127;
the metabolite having the molecular formula C₃₆H₇₀O₆ has a LC-MS/MS fragment pattern comprising daughter ions with m/z of: 597, 579, 561, 517, 315, 297, 279; respectively
wherein the molecules are analyzed by a liquid chromatography - mass spectrometry (LC-MS) method or direct injection triple-quadrupole mass spectrometry method;
comparing the quantifying data obtained on molecules in said test subject with quantifying data obtained from said molecules from a plurality of ovarian cancer-negative humans; wherein the absence or significant reduction of one or more than one of said metabolite indicates the presence of ovarian cancer or the risk of developing ovarian cancer.

2. The method according to claim 1 wherein said method is a Fourier transform ion cyclotron mass spectrometry based method.

3. The method according to claim 1 or 2, wherein a liquid/liquid extraction is performed on the sample whereby non-polar metabolites are dissolved in an organic solvent and polar metabolites are dissolved in an aqueous solvent.

4. The method according to any of claims 1 to 3, wherein said method includes using a mass spectrometer comprised of a source which ionizes molecules and a detector for detecting the ionized particles.

5. The method according to claim 4, wherein the detector includes quadrupole-based systems, time-of-flight, magnetic sector, ion cyclotron, and derivations thereof.

6. The method according to claim 4 or 5, wherein the source is selected from electrospray ionization and atomospheric pressure chemical ionization.

7. The method according to any of claims 1 to 6, wherein the mass spectrometer is equipped with a chromatographic separation system.

8. The method of claim 1, wherein the intensity transition of each of said molecules and the intensity transition of an internal standard are measured.

9. The method according to anyone of claims 1 to 8, wherein the blood sample is a whole blood sample, a sub-fraction of whole blood, a blood serum sample or a plasma sample.

## Patentansprüche

1. Verfahren zur Diagnose von Eierstockkrebs oder des Risikos, Eierstockkrebs zu entwickeln, in einem Testindividuum mit unbekanntem Eierstockkrebsstatus, umfassend die Schritte:
Analysieren einer Blutprobe aus einem Testindividuum zur Gewinnung von Quantifizierungsdaten in Bezug auf Moleküle, die aus Metaboliten ausgewählt sind, die durch die genauen Massen 474,3736, 478,4022, 484,3764, 490,3658, 492,3815, 494,3971, 496,4128, 502,4022, 504,4179, 512,4077, 518,3971, 520,4128, 522,8284, 530,43351, 532,44916, 540,4389, 550,4597, 558,4648, 574,4597, 576,4754, 578,4910, 596,5016 und 598,5172 identifiziert wurden, wobei ein Unterschied von ± 5 ppm denselben Metaboliten anzeigen würde;
wobei die Molekülformeln der Metaboliten mit diesen Massen C₃₀H₅₀O₄, C₃₀H₅₉O₄, C₂₈H₅₂O₆, C₃₀H₅₀O₅, C₃₀H₅₂O_{5,} C₃₀H₅₄O₅, C₃₀H₅₆O₅, C₃₂H₅₄O_{4,} C₃₂H₅₆O₄, C₃₀H₅₆O₆, C₃₂H₅₄O₅, C₃₂H₅₆O₅, C₃₂H₆₀O₅, C₃₄H₅₈O₄, C₃₄H₆₀O₄, C₃₂H₆₀O₆ C₃₄H₆₂O₅ C₃₆H₆₂O₄, C₃₆H₆₂O₅, C₃₆H₆₄O_{5,} C₃₆H₆₆O₅, C₃₆H₆₈O₆ bzw. C₃₆H₇₀O₆ sind;
wobei die Metaboliten LC-MS/MS-Fragmentmuster aufweisen, die unter negativer Ionisierung analysiert wurden, wie folgt:
der Metabolit mit der Molekülformel C₃₀H₅₀O₄ hat ein LC-MS/MS-Fragmentmuster, das Tochterionen mit m/z von 473, 455, 429, 411, 223, 113 umfasst;
der Metabolit mit der Molekülformel C₃₀H₅₄O₄ hat ein LC-MS/MS-Fragmentmuster, das Tochterionen mit m/z von 477, 460, 433, 415, 281 umfasst;
der Metabolit mit der Molekülformel C₂₈H₅₂O₆ hat ein LC-MS/MS-Fragmentmuster, das Tochterionen mit m/z von 483, 465, 447, 439, 421, 315, 313, 297, 279, 241, 201, 171, 101 umfasst;
der Metabolit mit der Molekülformel C₃₀H₅₀O₅ hat ein LC-MS/MS-Fragmentmuster, das Tochterionen mit m/z von 489, 471, 445, 427, 373, 345, 319, 267, 249, 223 umfasst;
der Metabolit mit der Molekülformel C₃₀H₅₂O₅ hat ein LC-MS/MS-Fragmentmuster, das Tochterionen mit m/z von 491, 473, 445, 427, 319, 249, 241, 223, 213 umfasst;
der Metabolit mit der Molekülformel C₃₀H₅₄O₅ hat ein LC-MS/MS-Fragmentmuster, das Tochterionen mit m/z von 493, 475, 449, 431, 415, 307, 297, 279, 267, 241, 235, 223, 215, 197, 167, 151, 141, 113 umfasst;
der Metabolit mit der Molekülformel C₃₀H₅₆O₅ hat ein LC-MS/MS-Fragmentmuster, das Tochterionen mit m/z von 495, 477, 451, 433, 297, 279, 241, 223, 215, 197, 179, 169 umfasst;
der Metabolit mit der Molekülformel C₃₂H₅₄O₄ hat ein LC-MS/MS-Fragmentmuster, das Tochterionen mit m/z von 501, 483, 465, 457, 439, 279 umfasst;
der Metabolit mit der Molekülformel C₃₂H₅₆O₄ hat ein LC-MS/MS-Fragmentmuster, das Tochterionen mit m/z von 503, 485, 467, 459, 441, 279, 263, 223, 169 umfasst;
der Metabolit mit der Molekülformel C₃₀H₅₆O₆ hat ein LC-MS/MS-Fragmentmuster, das Tochterionen mit m/z von 511, 493, 467, 315, 297, 279, 259, 251, 151 umfasst;
der Metabolit mit der Molekülformel C₃₂H₅₄O₅ hat ein LC-MS/MS-Fragmentmuster, das Tochterionen mit m/z von 517, 499, 481, 473, 455, 445, 437, 389, 279, 223 umfasst;
der Metabolit mit der Molekülformel C₃₂H₅₆O₅ hat ein LC-MS/MS-Fragmentmuster, das Tochterionen mit m/z von 519, 501, 483, 475, 459, 457, 447, 297, 279, 241, 223, 195, 115 umfasst;
der Metabolit mit der Molekülformel C₃₂H₆₀O₅ hat ein LC-MS/MS-Fragmentmuster, das Tochterionen mit m/z von 521, 503, 485, 477, 459, 441, 297, 279, 269, 241, 115 umfasst;
der Metabolit mit der Molekülformel C₃₄H₅₈O₄ hat ein LC-MS/MS-Fragmentmuster, das Tochterionen mit m/z von 529, 511, 485, 467, 251, 205 umfasst;
der Metabolit mit der Molekülformel C₃₄H₆₀O₄ hat ein LC-MS/MS-Fragmentmuster, das Tochterionen mit m/z von 531, 513, 495, 485, 469, 251, 181 umfasst;
der Metabolit mit der Molekülformel C₃₂H₆₀O₆ hat ein LC-MS/MS-Fragmentmuster, das Tochterionen mit m/z von 539, 521, 495, 477, 315, 313, 297, 279, 259, 243, 241, 225, 223, 179, 171 umfasst;
der Metabolit mit der Molekülformel C₃₄H₆₂O₅ hat ein LC-MS/MS-Fragmentmuster, das Tochterionen mit m/z von 549, 531, 513, 505, 487, 469, 297, 279, 269, 253, 125 umfasst;
der Metabolit mit der Molekülformel C₃₆H₆₂O₄ hat ein LC-MS/MS-Fragmentmuster, das Tochterionen mit m/z von 557, 539, 513, 495, 279, 155 umfasst;
der Metabolit mit der Molekülformel C₃₆H₆₂O₅ hat ein LC-MS/MS-Fragmentmuster, das Tochterionen mit m/z von 573, 555, 537, 529, 511, 493, 401, 295, 279, 223 umfasst;
der Metabolit mit der Molekülformel C₃₆H₆₄O₅ hat ein LC-MS/MS-Fragmentmuster, das Tochterionen mit m/z von 575, 557, 539, 531, 513, 495, 403, 297, 279, 251, 183 umfasst;
der Metabolit mit der Molekülformel C₃₆H₆₆O₅ hat ein LC-MS/MS-Fragmentmuster, das Tochterionen mit m/z von 577, 559, 541, 533, 515, 497, 373, 297, 281, 279 umfasst;
der Metabolit mit der Molekülformel C₃₆H₆₈O₆ hat ein LC-MS/MS-Fragmentmuster, das Tochterionen mit m/z von 595, 577, 559, 551, 515, 315, 297, 281, 279, 171, 155, 141, 127 umfasst;
der Metabolit mit der Molekülformel C₃₆H₇₀O₆ hat ein LC-MS/MS-Fragmentmuster, das Tochterionen mit m/z von 597, 579, 561, 517, 315, 297, 279 umfasst; bzw.
wobei die Moleküle durch ein Flüssigkeitschromatographie-Massenspektrometrie-(LC-MS)-Verfahren oder direktes Injektions-Triple-Quadrupol-Massenspektrometrieverfahren analysiert werden;
Vergleichen der Quantifizierungsdaten, die an Molekülen in dem Testindividuum erhalten werden, mit Quantifizierungsdaten, die aus Molekülen aus einer Anzahl von Eierstockkrebs-negativen Personen erhalten wurden; wobei die Abwesenheit oder die signifikante Reduktion von einem oder mehr als einem Metaboliten das Vorliegen von Eierstockkrebs oder das Risiko zur Entwicklung von Eierstockkrebs anzeigt.

2. Verfahren nach Anspruch 1, wobei das Verfahren ein Verfahren auf Fourier-Transformations-Ionen-Cyclotron-Massenspektrometrie-Basis ist.

3. Verfahren nach Anspruch 1 oder 2, wobei eine Flüssigkeits-/Flüssigkeits-Extraktion an der Probe durchgeführt wird, wobei nicht-polare Metaboliten in einem organischen Lösungsmittel gelöst werden und polare Metaboliten in einem wässrigen Lösungsmittel gelöst werden.

4. Verfahren nach einem der Ansprüche 1 bis 3, wobei das Verfahren die Verwendung eines Massenspektrometers beinhaltet, das eine Quelle, die Moleküle ionisiert, und einen Detektor zum Nachweis der ionisierten Teilchen umfasst.

5. Verfahren nach Anspruch 4, wobei der Detektor Systeme auf Quadrupolbasis, Time-of-flight, Magnetsektor, Ionenzyklotron und Abwandlungen davon umfasst.

6. Verfahren nach Anspruch 4 oder 5, wobei die Quelle aus Elektrospray-Ionisation und chemischer Ionisation unter Atmosphärendruck ausgewählt ist.

7. Verfahren nach einem der Ansprüche 1 bis 6, wobei das Massenspektrometer mit einem chromatographischen Trennsystem ausgestattet ist.

8. Verfahren nach Anspruch 1, wobei der Intensitätsübergang von jedem der Moleküle und der Intensitätsübergang eines internen Standards gemessen werden.

9. Verfahren nach einem der Ansprüche 1 bis 8, wobei die Blutprobe eine Vollblutprobe, eine Unterfraktion von Vollblut, eine Blutserumprobe oder eine Plasmaprobe ist.

## Revendications

1. Méthode de diagnostic d'un cancer de l'ovaire ou du risque de développement d'un cancer de l'ovaire chez un sujet d'essai de statut inconnu vis-à-vis du cancer de l'ovaire, comprenant les étapes suivantes:
analyse d'un échantillon sanguin provenant d'un sujet d'essai pour obtenir des données quantitatives quant aux molécules choisies parmi les métabolites identifiés par les masses exactes 474.3736, 478.4022, 484.3764, 490.3658, 492.3815, 494.3971, 496.4128, 502.4022, 504.4179, 512.4077, 518.3971, 520.4128, 522.8284, 530.43351, 532.44916, 540.4389, 550.4597, 558.4648, 574.4597, 576.4754, 578.4910, 596.5016 et 598.5172 où une différence de ± 5 ppm indique le même métabolite ;
où les formules moléculaires des métabolites correspondant à ces masses sont respectivement C₃₀H₅₀O₄, C₃₀H₅₄O₄, C₂₈H₅₂O₆, C₃₀H₅₀O₅, C₃₀H₅₂O₅, C₃₀H₅₄O₅, C₃₀H₅₆O₅, C₃₂H₅₄O₄, C₃₂H₅₆O₄, C₃₀H₅₆O₆, C₃₂H₅₄O_{5,} C₃₂H₅₆O₅, C₃₂H₆₀O₅, C₃₄H₅₈O₄, C₃₄H₆₀O₄, C₃₂H₆₀O₆, C₃₄H₆₂O₅, C₃₆H₆₂O₄, C₃₆H₆₂O₅, C₃₅H₅₄O₅, C₃₆H₆₆O₅, C₃₆H₆₈O₆ et C₃₆H₇₀O₆;
où les métabolites présentent des diagrammes de fragmentation LC-MS/MS analysés sous ionisation négative comme suit:
le métabolite répondant à la formule moléculaire C₃₀H₅₀O₄ présente un diagramme de fragmentation LC-MS/MS comprenant les ions dérivés de m/z: 473, 455, 429, 411, 223, 113 ;
le métabolite répondant à la formule moléculaire C₃₀H₅₄O₄ présente un diagramme de fragmentation LC-MS/MS comprenant les ions dérivés de m/z: 477, 460, 433, 415, 281 ;
le métabolite répondant à la formule moléculaire C₂₈H₅₂O₆ présente un diagramme de fragmentation LC-MS/MS comprenant les ions dérivés de m/z: 483, 465, 447, 439, 421, 315, 313, 297, 279, 241, 201, 171, 101 ;
le métabolite répondant à la formule moléculaire C₃₀H₅₀O₅ présente un diagramme de fragmentation LC-MS/MS comprenant les ions dérivés de m/z: 489, 471, 445, 427, 373, 345, 319, 267, 249, 223 ;
le métabolite répondant à la formule moléculaire C₃₀H₅₂O₅ présente un diagramme de fragmentation LC-MS/MS comprenant les ions dérivés de m/z 491, 473, 445, 427, 319, 249, 241, 223, 213 ;
le métabolite répondant à la formule moléculaire C₃₀H₅₄O₅ présente un diagramme de fragmentation LC-MS/MS comprenant les ions dérivés de m/z: 493, 475, 449, 431, 415, 307, 297, 279, 267, 241, 235, 223, 215, 197, 167, 151, 141, 113 ;
le métabolite répondant à la formule moléculaire C₃₀H₅₆O₅ présente un diagramme de fragmentation LC-MS/MS comprenant les ions dérivés de m/z: 495, 477, 451, 433, 297, 279, 241, 223, 215, 197, 179, 169 ;
le métabolite répondant à la formule moléculaire C₃₂H₅₄O₄ présente un diagramme de fragmentation LC-MS/MS comprenant les ions dérivés de m/z: 501, 483, 465, 457, 439, 279 ;
le métabolite répondant à la formule moléculaire C₃₂H₅₆O₄ présente un diagramme de fragmentation LC-MS/MS comprenant les ions dérivés de m/z: 503, 485, 467, 459, 441, 279, 263, 223, 169 ;
le métabolite répondant à la formule moléculaire C₃₀H₅₆O₆ présente un diagramme de fragmentation LC-MS/MS comprenant les ions dérivés de m/z: 511, 493, 467, 315, 297, 279, 259, 251, 151 ;
le métabolite répondant à la formule moléculaire C₃₂H₅₄O₅ présente un diagramme de fragmentation LC-MS/MS comprenant les ions dérivés de m/z: 517, 499, 481, 473, 455, 445, 437, 389, 279, 223 ;
le métabolite répondant à la formule moléculaire C₃₂H₅₆O₅ présente un diagramme de fragmentation LC-MS/MS comprenant les ions dérivés de m/z: 519, 501, 483, 475, 459, 457, 447, 297, 279, 241, 223, 195, 115 ;
le métabolite répondant à la formule moléculaire C₃₂H₆₀O₅ présente un diagramme de fragmentation LC-MS/MS comprenant les ions dérivés de m/z: 521, 503, 485, 477, 459, 441, 297, 279, 269, 241, 115;
le métabolite répondant à la formule moléculaire C₃₄H₅₈O₄ présente un diagramme de fragmentation LC-MS/MS comprenant les ions dérivés de m/z: 529, 511, 485, 467, 251, 205 ;
le métabolite répondant à la formule moléculaire C₃₄H₆₀O₄ présente un diagramme de fragmentation LC-MS/MS comprenant les ions dérivés de m/z: 531, 513, 495, 485, 469, 251, 181 ;
le métabolite répondant à la formule moléculaire C₃₂H₆₀O₆ présente un diagramme de fragmentation LC-MS/MS comprenant les ions dérivés de m/z: 539, 521, 495, 477, 315, 313, 297, 279, 259, 243, 241, 225, 223, 179, 171 ;
le métabolite répondant à la formule moléculaire C₃₄H₆₂O₅ présente un diagramme de fragmentation LC-MS/MS comprenant les ions dérivés de m/z: 549, 531, 513, 505, 487, 469, 297, 279, 269, 253, 125;
le métabolite répondant à la formule moléculaire C₃₆H₆₂O₄ présente un diagramme de fragmentation LC-MS/MS comprenant les ions dérivés de m/z: 557, 539, 513, 495, 279, 155 ;
le métabolite répondant à la formule moléculaire C₃₆H₆₂O₅ présente un diagramme de fragmentation LC-MS/MS comprenant les ions dérivés de m/z: 573, 555, 537, 529, 511, 493, 401, 295, 279, 223 ;
le métabolite répondant à la formule moléculaire C₃₆H₆₄O₅ présente un diagramme de fragmentation LC-MS/MS comprenant les ions dérivés de m/z: 575, 557, 539, 531, 513, 495, 403, 297, 279, 251, 183;
le métabolite répondant à la formule moléculaire C₃₆H₆₆O₅ présente un diagramme de fragmentation LC-MS/MS comprenant les ions dérivés de m/z: 577, 559, 541, 533, 515, 497, 373, 297, 281, 279 ;
le métabolite répondant à la formule moléculaire C₃₆H₆₈O₆ présente un diagramme de fragmentation LC-MS/MS comprenant les ions dérivés de m/z: 595, 577, 559, 551, 515, 315, 297, 281, 279, 171, 155, 141, 127 ;
le métabolite répondant à la formule moléculaire C₃₆H₇₀O₆ présente un diagramme de fragmentation LC-MS/MS comprenant les ions dérivés de m/z: 597, 579, 561, 517, 315, 297, 279 ; respectivement
où les molécules sont analysées par une méthode de chromatographie liquide-spectrométrie de masse (LC-MS) ou par une méthode de spectrométrie de masse à triple quadripôle et injection directe ;
en comparant les données quantitatives obtenues sur des molécules issues dudit sujet d'essai à des données quantitatives obtenues à partir des petites molécules issues d'une multitude d'humains négatifs vis-à-vis du cancer de l'ovaire ; où l'absence ou la réduction significative d'un ou de plusieurs desdits métabolites indique la présence d'un cancer de l'ovaire ou le risque de développer un cancer de l'ovaire.

2. Méthode selon la revendication 1, où ladite méthode est une méthode basée sur la spectrométrie de masse par résonance cyclotron des ions à transformée de Fourier.

3. Méthode selon la revendication 1 ou 2, où une extraction liquide/liquide, par laquelle les métabolites non polaires sont dissous dans un solvant organique et les métabolites polaires sont dissous dans un solvant aqueux, est mise en oeuvre sur l'échantillon.

4. Méthode selon l'une quelconque des revendications 1 à 3, où ladite méthode inclut l'utilisation d'un spectromètre de masse constitué d'une source qui ionise les molécules et d'un détecteur pour la détection des particules ionisées.

5. Méthode selon la revendication 4, où le détecteur inclut des systèmes à base de quadripôle, à temps de vol, à secteur magnétique, à résonance cyclotron des ions, et leurs dérivés.

6. Méthode selon la revendication 4 ou 5, où la source est choisie parmi l'ionisation par électrospray et l'ionisation chimique à pression atmosphérique.

7. Méthode selon l'une quelconque des revendications 1 à 6, où le spectromètre de masse est équipé d'un système de séparation chromatographique.

8. Méthode selon la revendication 1, où la transition d'intensité de chacune desdites molécules et la transition d'intensité d'un étalon interne sont mesurées.

9. Méthode selon l'une quelconque des revendications 1 à 8, où l'échantillon sanguin est un échantillon de sang total, une sous-fraction de sang total, un échantillon de sérum sanguin ou un échantillon de plasma.
